(19) Europäisches Patentamt
European Patent Office
Office européen des brevets

(11) **EP 4 220 171 B1**

(12) **EUROPEAN PATENT SPECIFICATION**

(45) Date of publication and mention of the grant of the patent:
**09.09.2026 Bulletin 2026/37**

(21) Application number: **23151625.3**

(22) Date of filing: **20.07.2018**

(51) International Patent Classification (IPC):
***G01N 33/575*** *(2026.01)*

(52) Cooperative Patent Classification (CPC):
**G01N 33/5755; G01N 33/575;** G01N 2333/96494; G01N 2800/52

# (54) MARKERS FOR ENDOMETRIAL CANCER

MARKER FÜR ENDOMETRIUMKREBS

MARQUEURS POUR LE CANCER DE L'ENDOMÈTRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.07.2017 EP 17382483**

(43) Date of publication of application:
**02.08.2023 Bulletin 2023/31**

(62) Document number(s) of the earlier application(s) in accordance with Art. 76 EPC:
**18749741.7 / 3 655 778**

(73) Proprietor: **Fundació Hospital Universitari Vall d'Hebron - Institut de Recerca
08035 Barcelona (ES)**

(72) Inventors:
- **MARTÍNEZ GARCÍA, Elena
09002 Burgos (ES)**
- **COLÁS ORTEGA, Eva
08020 Barcelona (ES)**
- **GIL MORENO, Antonio
08172 Sant Cugat del Vallès (ES)**
- **REVENTÓS PUIGJANER, Jaume
08348 Cabrils (ES)**
- **DOMON, Bruno
CH-2534 Orvin (CH)**
- **LESUR, Antoine
L-8010 Strassen (LU)**
- **CAMPOY MONCAYO, Irene
08028 Barcelona (ES)**

(74) Representative: **Hoffmann Eitle
Hoffmann Eitle S.L.U.
Paseo de la Castellana 140, 3a planta
Edificio LIMA
28046 Madrid (ES)**

(56) References cited:
**EP-A2- 2 251 695**
**WO-A1-2008/049239**
**WO-A1-2017/190896**

- **ELENA MARTINEZ-GARCIA ET AL: "Development of a sequential workflow based on LC-PRM for the verification of endometrial cancer protein biomarkers in uterine aspirate samples & supplementary figures", ONCOTARGET, vol. 7, no. 33, 16 July 2016 (2016-07-16), pages 53102 - 53115, XP055503751**
- **ALBA MOTA ET AL: "Genetic analysis of uterine aspirates improves the diagnostic value and captures the intra-tumor heterogeneity of endometrial cancers", MODERN PATHOLOGY, vol. 30, no. 1, 2 September 2016 (2016-09-02), GB, pages 134 - 145, XP055503647, ISSN: 0893-3952, DOI: 10.1038/modpathol.2016.143**
- **S CABRERA ET AL: "Targeted proteomics identifies proteomic signatures in liquid-biopsies of the endometrium to diagnose endometrial cancer and assist in the prediction of the optimal surgical treatment", INTERNATIONAL JOURNAL OF GYNECOLOGICAL CANCER, vol. 27, no. supplement 4, 1 November 2017 (2017-11-01), US, pages 340 - 340, XP055503787, ISSN: 1525-1438**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **GARCÍA ELENA MARTÍNEZ: "Protein signatures in uterine aspirates to improve diagnosis of endometrial cancer", 12 June 2017 (2017-06-12), XP055872995, Retrieved from the Internet <URL:https://www.tdx.cat/bitstream/handle/10803/458514/emg1de1.pdf?sequence=1&isAllowed=y> [retrieved on 20211214]**
- **IRENE CAMPOY MONCAYO: "Exosomes in uterine aspirates: characterization and identification of diagnostic and prognostic biomarkers in endometrial cancer", 20 June 2017 (2017-06-20), XP055732570, Retrieved from the Internet <URL:https://ddd.uab.cat/pub/tesis/2017/hdl_10803_458138/icm1de1.pdf> [retrieved on 20200921]**

## Description

This application claims the benefit of European Patent Application EP17382483.0 filed July 21, 2017.

## Technical Field

The invention relates to the diagnosis and prognosis of endometrial cancer.

## Background Art

Endometrial cancer (EC) is the most frequently observed invasive tumor of the female genital tract and the fourth most common cancer in women in developed countries, accounting for 61380 diagnosed cases and 10,920 estimated deaths in 2017 in the United States. Nowadays, 70% of the EC cases are diagnosed at early stages of the disease where the tumor is still localized within the endometrium and is associated with an overall 5-year survival rate of 96%. However, 30% of EC patients are diagnosed only at an advanced stage of the disease associated with a drastic decrease in the 5-year survival rate, which is reduced to 67% when myometrial invasion and/or lymph node affectation is already present and to 18% in cases of distant metastasis. Improving early diagnosis is hence a major issue to appropriately manage EC and decrease mortality associated with the disease.

Early detection of EC patients is favored by the presence of symptoms like abnormal vaginal bleeding present in 93% of women diagnosed with EC. However, many other benign disorders generate similar symptoms. Discrimination of patients with benign endometrial pathologies and with EC is only achieved after a tedious diagnostic process consisting of a pelvic examination and transvaginal ultrasonography followed by a confirmatory histopathological examination of an endometrial biopsy. The preferable biopsy used in this procedure is named uterine aspirate and/or pipelle biopsy and is obtained by a minimally invasive aspiration of endometrial fluid from inside the uterine cavity. Because the current diagnostic procedures on uterine aspirates rely on the presence of cellular material, this process has unfortunately a diagnostic failure and an associated inadequate sampling rate of 8% and 15%, respectively. This is increased in postmenopausal women up to 12% and 22%. In those cases, a biopsy guided by hysteroscopy needs to be performed, where this invasive technique presents an increased risk of complications, including uterine perforation, hemorrhage and possible harm to other organs.

To date, many studies have been conducted to identify EC protein biomarkers, mainly in tissue and serum samples (see for example DeSouza LV, et al, "Endometrial cancer biomarker discovery and verification using differentially tagged clinical samples with multidimensional liquid chromatography and tandem mass spectrometry", Mol Cell Proteomics MCP- 2007, vol. no.6, pp.:1170-8, or Kemik P, et al. "Diagnostic and prognostic values of preoperative serum levels of YKL-40, HE-4 and DKK-3 in endometrial cancer", Gynecol Oncol- 2016; vol. no.140, pp.:64-9). None of them have been translated into clinical utility.

Although the biopsies should provide information about tumor histology and tumor grade to help in the risk stratification of the EC patients and guide the surgical staging procedure, unfortunately, the limited number of cells available for examination and the high inter-observer variability in the pathological interpretation results in 40-50% of discordances in EC histotype and grade between biopsies and final hysterectomy specimens. Therefore, the identification of sensitive, specific, and reproducible biomarkers that improve diagnosis, prognosis and preoperative assessment of the histological type and grade of EC tumors is crucial to appropriately manage EC patients and decrease mortality and morbidity associated with this disease.

Among the suptypes or manifestations variety of EC, endometroid endometrial cancer (EEC) is the most common histology in EC and has a good prognosis when compared with non-endometrioid EC cases (NEEC). NEEC represents about 20% of all EC cases but accounts for more than 50% of recurrences and deaths from EC. Among NEEC, the serous EC (SEC) is the most common subtype. There are no availabe tests for discriminating between these two histologies with different outcomes.

Concluding, in spite of the efforts made, there is still the need of biomarkers allowing the diagnosis, and even the prognosis of endometrial cancer with high sensitivity and specificity, in particular the need of biomarkers for clinical practice.

## Summary of Invention

Inventors have determined that certain protein markers detectable in exosomes isolated from uterine fluid, give valuable diagnostic information in endometrial cancer (EC). Many of these proteins were validated in uterine fluid itself without isolation of the exosome fraction. Hence, the proteins are meaningful diagnostic biomarkers allowing discrimination with high sensitivity and specificity between EC and non-cancer controls when analyzed in the uterine fluid.

In addition, inventors have determined that some proteins that can be detected in said uterine fluid, and also in the

exosome fraction of said uterine fluid, are meaningful prognostic biomarkers of endometrial cancer (EC). These proteins allow discrimination between endometrial cancer subtypes with high sensitivity and high specificity, and thus they allow minimizing the risk of false positive and false negative classification among these subtypes.

Moreover, being the proteins detectable in samples that are obtained without invasive practices, such as in the uterine fluid samples, which in turn are routine sample types in the EC diagnostics, supposes a real advantage and improvement in the clinical practice of EC patient classification and managing.

In previous studies, inventors demonstrated that the fluid fraction of uterine aspirates have an important value for the screening of EC protein biomarkers (see for example Martinez-Garcia E, et al. "Development of a sequential workflow based on LC-PRM for the verification of endometrial cancer protein biomarkers in uterine aspirate samples", Oncotarget -2016, vol. no. 7(33), pp.:53102-53114). This document shows a study carried out with a sequential workflow based on LC-PRM, illustrating the importance of a biomarker verification phase to fill the gap between discovery and clinical practice. This study highlighted the benefit of 26 proteins to diagnose EC patients from non-EC patients (controls).

In relation with the new diagnostic biomarkers for EC identified in exosomes of uterine fluid, one aspect of the invention is a method of diagnosis of EC, the method comprising determining the level of expression of BCAM in a uterine fluid sample from the female genital tract.

Another aspect of the invention is the use of BCAM as *in vitro* marker for differentially diagnosing EEC and NEEC in a uterine fluid sample from the female genital tract.

This aspect can also be formulated as an *in vitro* method for detecting one or more endometrial cancer markers in a subject, comprising: (a) obtaining a fluid sample from the female genital tract; and (b) detecting in the sample an amount of the endometrial cancer marker BCAM in an uterine fluid sample from the female genital tract, said marker giving information of the diagnosis of EC. In general terms, it is encompassed the use of protein BCAM as *in vitro* marker for diagnosing endometrial cancer in an isolated uterine fluid sample from the female genital tract.

Diagnosis of EC with the one or more proteins listed above can be carried out by the use of kits comprising means for determining the level of expression of BCAM. The kit may comprise a solid support and means for detecting the level of expression of protein BCAM.

Another aspect of the invention is the use of said kits, comprising means for determining one or more of the proteins defined above, for the diagnosis of EC.

An additional aspect related with the diagnosis of EC, provides a method for identifying a subject suspicious of suffering from EC, the method comprising:

a) determining, *in vitro,* the level of expression of proteins BCAM, or BCAM and KPYM, in a uterine fluid sample from the female;
b) optionally determining, *in vitro,* the level of expression of one or more proteins selected from the group consisting of: AGRIN, MVP, TACD2, FAS, VAMP8, SYIC, SORT, LAT1, TERA, RUVB1, RS16, RSSA, SMD3, ADA10, RPL13A, RL11, IMB1, AGR2, ITA3, RUXE, RL12, PSMD2, MX1, VPS35, ILF2, PDIA1, ANXA4, MMP9, RAB8A, SH3L3, RL29, PLD3, PPIA, ANXA2, SSRA, LAMP2, PODXL, CLD6, IF2B3, CD59, MLEC, PGBM, S10AC, CD14, H10, CD81, AR6P1, VAC14, ITB3 and CD166 in an exosome-containing fraction isolated from uterine fluid; and
c) comparing the level of step (a) and optionally that of step (b) with a reference control level, wherein if the level determined in step (a) and optionally that of step (b) is higher than the reference control level, it is indicative that the subject is suspicious of suffering endometrial carcinoma.

In a further aspect, the present invention provides a method of deciding or recommending whether to initiate a medical regimen of a subject suspicious of suffering endometrial carcinoma, which method comprises the steps of:

a) determining, *in vitro,* the level of expression of protein BCAM in a uterine fluid sample from the female;
b) optionally determining, *in vitro,* the level of expression of one or more proteins selected from the group consisting of: AGRIN, MVP, TACD2, FAS, VAMP8, SYIC, SORT, LAT1, TERA, RUVB1, RS16, RSSA, SMD3, ADA10, RPL13A, RL11, IMB1, AGR2, ITA3, RUXE, RL12, PSMD2, MX1, VPS35, ILF2, PDIA1, ANXA4, MMP9, RAB8A, SH3L3, RL29, PLD3, PPIA, ANXA2, SSRA, LAMP2, PODXL, CLD6, IF2B3, CD59, MLEC, PGBM, S10AC, CD14, H10, CD81, AR6P1, VAC14, ITB3 and CD166 in an exosome-containing fraction isolated from uterine fluids; and
c) comparing the level of step (a) and optionally that of step (b) with a reference control level, wherein if the level determined in step (a) and optionally that of step (b) is higher than the reference control level, it is indicative that the subject is suspicious of suffering endometrial carcinoma.

wherein:

i) if the subject is diagnosed of suffering from endometrial carcinoma, or of being suspicious of suffering from endometrial carcinoma, then the initiation of the medical regimen is recommended, and

ii) if the patient is diagnosed of not suffering from endometrial carcinoma, the follow-up is performed optionally in consideration of the result of an examination of the patient by a physician.

By determining the marker level in a test sample, the skilled person can establish, additionally, which is the most suitable therapy that can be recommended, because the level detected in the sample may reflect the extension (i.e., severity) of the disease.

Another aspect is a method of prognosis of endometrial cancer, the method comprising determining the level of expression of the protein BCAM in a uterine fluid sample from the female genital tract.

The methods give important diagnostic and prognostic information, since accurate prognosis can be done because two different hystological manifestations of EC, namely endometrioid endometrial cancer (EEC) and non-endometroid endometrial cancer (NEEC), can be differentiated, being EEC of better likely outcome (i.e. better prognosis). In other words, the determination of the proteins in the uterine fluid sample allows differentially diagnosing EEC and NEEC.

As will be depicted in the examples below, proteins mentioned above allowed either the diagnose of EC or to accurately prognose EC, in terms that they were differentially expressed in isolated samples from patients suffering from EEC and from patients suffering from NEEC. Proteins with diagnostic value could be differentially detected due to differential expression in uterine fluid samples of EC subjects in relation with non-EC samples (healthy controls). Proteins with prognostic value were significantly increased in EEC subtype in comparison with the levels in NEEC subtype, with the exception of CAPG that was increased in NEEC tumors. So that, they allowed classification of tumors of the most prevalent histological subtypes. The proteins are thus usable tools for improving diagnosis, prognosis and/or preoperative risk assessment, and for assisting in the prediction of the optimal surgical treatment. Also as indicated below, some protein panels (protein combinations) with these and other proteins have been developed that allow for the accurate discrimination of EC vs non-EC samples ,and also for accurate discrimination EC histological types (AUC of 0.99). In combination with the histopathological examination, these panels are expected to preclude the need of more invasive diagnostic samplings and help to predict the optimal surgical treatment for EC patients.

Therefore, in general terms, one aspect of the invention is a method of prognosis of endometrial cancer (EC), the method comprising determining the level of expression of the protein BCAM in a uterine fluid sample from the female genital tract.

A second aspect of the invention is the use of protein BCAM as *in vitro* marker for prognosing EC in a uterine fluid sample from the female genital tract. This aspect can also be formulated as an *in vitro* method for detecting one or more endometrial cancer markers in a subject, comprising: (a) obtaining a fluid sample from the female genital tract; and (b) detecting in the sample an amount of the endometrial cancer marker BCAM said marker giving information of differential diagnosis of EEC and NEEC. In general terms, it is encompassed the use of protein BCAM as *in vitro* marker for prognosing endometrial cancer in an isolated uterine fluid sample from the female genital tract.

In a third aspect, the invention provides the use of protein BCAM for the prognosis of endometrial cancer in a method of the first aspect. That is, the proteins are used for differentially diagnosing EEC and NEEC.

Importantly, the protein biomarkers object of the present invention can be assessed by easy and low-cost methods, such as immunochemistry, chemiluminescent assay, or ELISA, platforms which are widely available in hospitals. Consequently, these protein biomarkers can be easily implemented as routine clinical diagnostic and/or prognostic kits with reduced costs for the health system. In addition, a diagnostic kit test based on the biomarkers provided by the present invention can ameliorate the current process of diagnosis and prognosis, conferring to uterine aspirates the ability of providing valuable diagnostic and prognostic information of the disease.

Therefore, in a fourth aspect the present invention provides the use of a kit for the prognosis of EC, the kit comprising a solid support and means for detecting the level of expression of protein BCAM, and optionally means for detecting the level of expression of one or more of the following proteins XPO2, PRDX1, CLIC1, PDIA1, KPYM, ENOA, GSTP1, GTR1, CH10, MIF, PEBP1, TPIS, NGAL, and LDHA.

In this fourth aspect the kit may comprise a solid support and means for detecting the level of expression of protein BCAM, and optionally means for detecting the level of expression of one or more of the following proteins XPO2, PRDX1, CLIC1, PDIA1, KPYM, ENOA, GSTP1, GTR1, CH10, MIF, PEBP1, TPIS, NGAL, and LDHA for use in the prognosis of EC.

In an additional aspect, the present invention provides the use of means for determining the level of expression of BCAM for the prognosis of endometrial cancer in the method of the first aspect of the invention. Thus, they are means for the differential diagnosis of EEC and NEEC. There are also provided use of means for determining the level of expression of BCAM, and optionally means for determining the level of expression of XPO2, PRDX1, CLIC1, PDIA1, KPYM, ENOA, GSTP1, GTR1, CH10, MIF, PEBP1, TPIS, NGAL, and LDHA, for the prognosis of endometrial cancer in the method of the first aspect of the invention.

In a further aspect, the present invention provides a method for identifying a subject suspicious of suffering from endometrial cancer and for further identifying EC subtype, by differentially diagnosing EEC of NEEC, the method comprising:

a) determining, *in vitro,* the level of expression of protein BCAM; and optionally the level of expression of one or more of proteins XPO2, PRDX1, CLIC1, PDIA1, KPYM, ENOA, GSTP1, GTR1, CH10, MIF, PEBP1, TPIS, NGAL, and LDHA in an uterine fluid sample from the subject's female genital tract; and
b) comparing the level of step (a) with a reference control level, wherein if the level determined in step (a) is higher than the reference control level for BCAM, it is indicative that the subject is suspicious of suffering from NEEC and of suffering EEC.

In this aspect of the invention, the reference control level is indistinctly chosen from non-EC and NEEC samples. As above exposed, the levels of expression in EEC is higher than the levels of expression in NEEC, except for CAPG. In addition, the levels of CADH1, CAPG, CTNB1 and CD44 are also higher than in non-EC (no cancer) control samples.

In a further aspect, the present invention provides a method of deciding or recommending whether to initiate a medical regimen of a subject suffering endometrial cancer in function of the prognosis, which method comprises the steps of:

a) determining, *in vitro,* the level of expression of protein BCAM and optionally the level of expression of one or more of proteins XPO2, PRDX1, CLIC1, PDIA1, KPYM, ENOA, GSTP1, GTR1, CH10, MIF, PEBP1, TPIS, NGAL, and LDHA in an uterine fluid sample from the subject's female genital tract; and
b) establishing the prognosis of said EC by distinguishing EEC and NEEC, if the protein level in the test sample is higher than a reference control level for BCAM

wherein:

i) if the subject is diagnosed of suffering from endometrial cancer, or of being suspicious of suffering from endometrial cancer, and the subject is differentially diagnosed of suffering EEC, then the initiation of the medical regimen for EEC is recommended;

ii) if the subject is diagnosed of suffering from endometrial cancer, or of being suspicious of suffering from endometrial cancer, and the subject is differentially diagnosed of suffering NEEC, then the initiation of the medical regimen for NEEC is recommended; and

by determining the marker level in a test sample, the skilled person can establish, additionally, which is the most suitable therapy that can be recommended, because the level detected in the sample may reflect the aggressiveness of the disease.

## Brief Description of Drawings

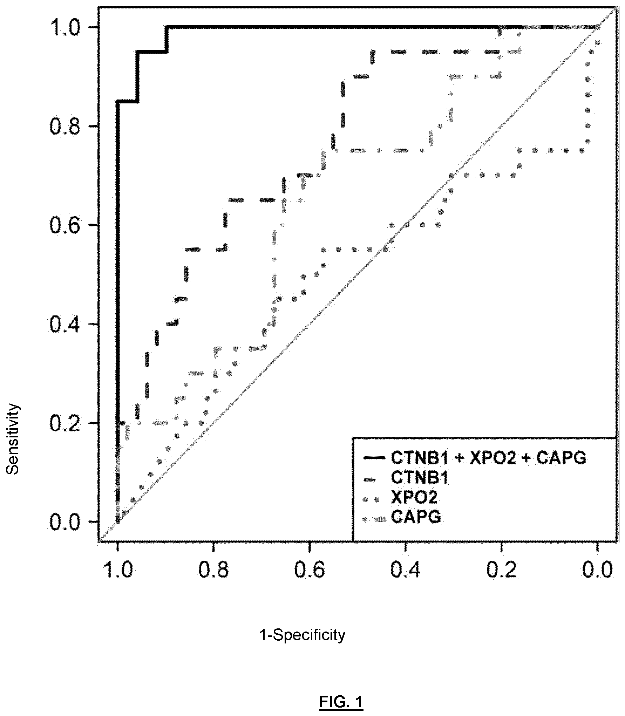

FIG. 1 shows a ROC curve for markers distinguishing EEC from NEEC, specifically NEEC cases are serous endometrial cancer (SEC). The sensitivity and specificity of CTNB1, XPO2 and CAPG individually; and of the combination (or panel of proteins) of the three are showed.

## Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

The present invention provides new biomarkers for the diagnosis and for prognosis of endometrial cancer in the female genital tract fluid.

The term "diagnosis" is known to the person skilled in the art. As used herein "diagnosis" is understood as becoming aware of a particular medical condition complication or risk in a subject; the determination of the nature of the disease or condition; or the distinguishing of one disease or condition from another. It refers both to the process of attempting to determine or identify the possible disease or disorder, and to the opinion reached by this process. A diagnosis, in the sense of diagnostic procedure, can be regarded as an attempt at classification of an individual's condition into separate and distinct categories that allow medical decisions about treatment and prognosis to be made. Subsequently, a diagnostic opinion is often described in terms of a disease or other condition. However, a diagnosis can take many forms. It might be a matter of detecting the presence and naming the disease, lesion, dysfunction or disability. It might be an exercise to attribute a category for management or for prognosis. It may indicate either degree of abnormality on a continuum or kind of abnormality in a classification. In general terms, diagnostic markers listed in this description are those

proteins differentially detected at level expression in isolated samples of controls (non-cancer individuals) versus endometrial cancer samples (including several types of EC).

The *in vitro* method of the first aspect of the invention can be performed with a sample of: (a) an asymptomatic subject, (b) a subject which has already been identified as being suspicious of suffering from endometrial cancer, (c) a subject already diagnosed of endometrial cancer, as complementary confirmation diagnostic assay or (d) a subject with high risk of suffering the disease.

In the present invention, the term "reference control level" referred to in the methods of the any of the aspects of the invention is to be understood as a predefined value of a given molecular marker or a combination of the given molecular markers, in the present case any of the proteins listed in the first or second aspects as well as in particular embodiments, which is derived from the levels of said molecular marker or markers in a sample or group of samples. If the level of expression is determined at the protein level, then the "reference expression level" is a predefined value of protein quantity, whereas if the level of expression is determined at the mRNA level, then the "reference expression level" is a predefined value of mRNA quantity. The samples are taken from a subject or group of subjects wherein the presence, absence, stage, histological subtype or grade, or course of the disease has been properly performed previously. This value is used as a threshold to discriminate subjects wherein the condition to be analyzed is present from those wherein such condition is absent (i.e. subject having endometrial cancer from subjects free of endometrial cancer), to determine the histological subtype of the disease, the risk of developing or of being suffering from endometrial carcinoma, among others. This reference control level is also useful for determining whether the subject has to initiate a medical regimen and how effective the regimen is. The subject or subjects from whom the "reference control level" is derived may include subject/s wherein the condition is absent, subject/s wherein the condition is present, or both. The skilled person in the art, making use of the general knowledge, is able to choose the subject or group of subjects more adequate for obtaining the reference control level for each of the methods of the present invention. Methods for obtaining the reference value from the group of subjects selected are well-known in the state of the art (Burtis C. A. et al., 2008, Chapter 14, section "Statistical Treatment of Reference Values") In a particular case "reference control level" is a cut-off value defined by means of a conventional ROC analysis (Receiver Operating Characteristic analysis). As the skilled person will appreciate, optimal cut-off value will be defined according to the particular applications of the diagnostic or prognostic method: purpose, target population for the diagnosis or prognosis, balance between specificity and sensibility, etc.

"Prognosis" as used herein refers to the prediction of the probable progression and outcome of a disease. It includes: neoplasm grading (attempt to express in replicable terms the level of cell differentiation in neoplasms as increasing anaplasia correlates with the aggressiveness of the neoplasm), neoplasm staging (attempt to express in replicable terms the extent of the neoplasm in the patient), neoplasm histological subtype, and neoplasm molecular subtype. As used herein prognosis means, in particular embodiments, differentiation between endometrioid endometrial cancer and non-endometrioid endometrial cancers.

The term "fluid sample from the female genital tract" refers to a fluid produced by the uterine organ forming part of the female genital tract and which has been taken by aspiration, such as vacuum aspiration (i.e., "uterine aspirate sample"), or by a cornier pipelle, and/or any other method that retrieves fluid from the uterine cavity. Thus, it includes uterine washing. According to the present invention, the aspiration of the fluid is in particular performed without a previous step of saline infusion. That is, the term "aspirate" does not encompass those samples resulting from uterine washings.

In the present invention, "Exosomes" interchangeably referred as "Extracellular vesicles", "microvesicles", "exosome-like vesicles" or, "uterosomes" are cell-derived vesicles that are present in many eukaryotic fluids, including blood, urine, and cultured medium of cell cultures. The reported diameter of exosomes is between 30 and 100 nm, which is larger than low-density lipoproteins (LDL) but much smaller than, for example, red blood cells. Exosomes are either released from the cell when multivesicular bodies fuse with the plasma membrane or released directly from the plasma membrane. Exosomes can potentially be used for diagnosis, for prognosis, for therapy, and as biomarkers for health and disease. For "exosome-containing fraction isolated from UA" is to be understood any purified fraction from the uterine aspirate that comprises mainly exosomes. Non-limiting examples of methods for isolating exosomes from uterine aspirates are detailed below.

In a particular embodiment of the aspect relating to new diagnostic biomarkers for EC identified in exosomes of uterine fluid, optionally in combination with any embodiments above or below, one aspect of the invention is a method of diagnosis of EC the sample is uterine fluid aspirate (UA).

In another embodiment, the method of diagnosis of EC comprises determining the level of expression of one or more proteins selected from AGRIN, MVP, TACD2, FAS, VAMP8, SYIC, SORT, LAT1, TERA, RUVB1, RS16, RSSA, SMD3, ADA10, RPL13A, RL11, IMB1, AGR2, ITA3, RUXE, RL12, PSMD2, MX1, VPS35, ILF2, PDIA1, ANXA4, MMP9, RAB8A, SH3L3, RL29, PLD3, PPIA, ANXA2, SSRA, LAMP2, PODXL, CLD6, IF2B3, CD59, MLEC, PGBM, S10AC, CD14, H10, CD81, AR6P1, VAC14, ITB3 and CD166 in a exosome-containing fraction isolated from UA; and the level of expression of BCAM in an uterine fluid sample from the female genital tract, this later sample without isolation or purification of exosomes.

In a particular embodiment, the method of diagnosis of EC comprises determining the level of expression of two

or more proteins, more in particular three or more proteins, and even more than four proteins.

In another particular embodiment, optionally in combination with any embodiment above or below, the method of diagnosis of EC comprises determining the level of expression of at least one set of proteins (i.e. biomarkers) selected from the group consisting of: AGRIN, CD81, TERA; AGRIN, CD59, MVP; AGR2, AGRIN, CD81; AGRIN, CD166, MVP; AGRIN, CD81; AGRIN, CD166; AGRIN, CD59; and AGRIN, MMP9, in an exosome-containing fraction isolated from UA; and determining the level of expression of BCAM in a uterine aspirate.

In yet another particular embodiment, optionally in combination with any embodiment above or below, the method of diagnosis of EC further comprises determining the level of expression of at least one set of proteins (i.e. biomarkers) selected from the group consisting of the list of Table D (illustrated at the end of this description).

Another aspect of the invention is the use of protein BCAM as *in vitro* marker for diagnosing EC in a uterine fluid sample from the female genital tract. This aspect can also be formulated as an *in vitro* method for detecting one or more endometrial cancer markers in a subject, comprising: (a) obtaining a fluid sample from the female genital tract; and (b) detecting in the sample an amount of the endometrial cancer marker BCAM in an uterine fluid sample from the female genital tract, said marker giving information of the diagnosis of EC. In general terms, it is encompassed the use of protein BCAM as *in vitro* marker for diagnosing endometrial cancer in an isolated uterine fluid sample from the female genital tract.

Also in another more particular embodiment, the use of the kits comprises means for the detection of the level of expression of a protein giving particular prognostic information (i.e. prognostic biomarkers) BCAM.

Another aspect of the invention is the use of said kits, comprising means for determining one or more of the proteins defined above, for the diagnosis of EC.

In another particular embodiment of the use of the kits, the means for detecting the level of expression of the proteins are means for carrying out an assay or technology selected from the group consisting of an immunoassay, a bioluminescence assay, a fluorescence assay, a chemiluminescence assay, electrochemistry assay, mass spectrometry, and combinations thereof.

Uniprot database accession numbers of all the herewith listed proteins correspond to versions accessible on July 07, 2017. In addition they are indicated in Tables from Examples.

AGRIN has the Uniprot database accession number O00468. This protein is a heparin sulfate basal lamina glycoprotein involved in the formation and the maintenance of the neuromuscular junction.

MVP, also known as major vault protein, has the Uniprot database accession number Q14764. It is involved in signal transduction.

VAMP8, also known as vesicle-associated membrane protein 8, has the Uniprot database accession number Q9BV40. It is a soluble N-ethylmaleimide-sensitive factor-attachment protein receptor (SNARE) involved in autophagy through the direct control of autophagosome membrane fusion with the lysososome membrane.

SYIC, also known as isoleucine--tRNA ligase, has the Uniprot database accession number P41252.

RAB8A, also known as Ras-related protein Rab-8A, has the Uniprot database accession number P61006. It is a small GTPases Rab involved in intracellular membrane trafficking.

MX1, also known as interferon-induced GTP-binding protein Mx1, has the Uniprot database accession number P20591. It has antiviral activity against a wide range of RNA viruses and some DNA viruses.

IMB1, also known as importin subunit beta-1, has the Uniprot database accession number Q14974. It is involved in nuclear protein import.

SMD3, also known as Small nuclear ribonucleoprotein Sm D3, has the Uniprot database accession number P62318. It is a component of the spliceosome.

ILF2, also known as Interleukin enhancer-binding factor 2, has the Uniprot database accession number Q12905. It is involved in the regulation of the IL2 gene.

TERA, also known as transitional endoplasmic reticulum ATPase, has the Uniprot database accession number P55072. This protein is involved in the formation of the transitional endoplasmic reticulum.

RL11, also known as 60S ribosomal protein L11, has the Uniprot database accession number P62913. This protein is a component of the ribosome, thus, it is involved in the synthesis of proteins in the cell.

BCAM, also known as basal cell adhesion molecule, has the Uniprot database accession number P50895. This protein is a Laminin alpha-5 receptor.

ANXA2, also known as Annexin A2, has the Uniprot database accession number P07355. This protein is a Calcium-regulated membrane-binding protein which inhibits PSCK9-enhanced LDLR degradation.

LAT1, also known as large neutral amino acids transporter small subunit 1, has the Uniprot database accession number Q01650. This protein is involved in cellular amino acid uptake.

RUVB1, also known as Pontin 52 or INO80 complex subunit H, has the Uniprot database accession number Q9Y265. This protein is a component of the NuA4 histone acetyltransferase complex, which is involved in transcriptional activation of select genes principally by acetylation of nucleosomal histones H4 and H2A.

SH3L3, also known as SH3 domain-binding glutamic acid-rich-like protein 3 or SH3 domain-binding protein 1, has the Uniprot database accession number Q9H299. This protein may be involved in the modulation of glutaredoxin

activity.

RPL13A, also known as 60S ribosomal protein, has the Uniprot database accession number P40429. It is involved in interferon-gamma-induced transcript-selective translation inhibition in inflammation processes.

RS16, also known as 40S ribosomal protein S16, has the Uniprot database accession number P62249.

RSSA, also known as 40S ribosomal protein SA or Laminin receptor 1, has the Uniprot database accession number P08865. It is required for the assembly and/or stability of the 40S ribosomal subunit.

RL12, also known as 60S ribosomal protein L12, has the Uniprot database accession number P30050.

RL29, also known as 60S ribosomal protein L29, has the Uniprot database accession number P47914. This protein is a component of the large ribosomal subunit.

PPIA, also known as peptidyl-prolyl cis-trans isomerase A, cyclophilin A or rotamase A, has the Uniprot database accession number P62937. This protein is involved in protein folding.

AGR2, also known as anterior gradient protein 2 homolog or HPC8, has the Uniprot database accession number O95994. This protein is involved in MUC2 post-transcriptional synthesis and secretion.

PODXL, also known as Podocalyxin or GCTM-2 antigen, has the Uniprot database accession number O00592. It is involved in the regulation of adhesion, cell morphology and cancer progression.

CD59, also known as CD59 glycoprotein or MAC-inhibitory protein, has the Uniprot database accession number P13987. It is involved in the inhibition of the complement membrane attack complex (MAC) action.

TACD2, also known as Tumor-associated calcium signal transducer 2 or cell surface glycoprotein Trop-2, has the Uniprot database accession number P09758. This protein may function as a growth factor receptor.

FAS, also known as Fatty acid synthase, has the Uniprot database accession number P49327. It is involved in the formation of long-chain fatty acids from acetyl-CoA, malonyl-CoA and NADPH.

SORT, also known as Sortilin or Neurotensin receptor 3, has the Uniprot database accession number Q99523. This protein functions as a sorting receptor in the Golgi compartment and as a clearance receptor on the cell surface.

ADA10, also known as Disintegrin and metalloproteinase domain-containing protein 10, has the Uniprot database accession number 014672. It is involved in the proteolytic release of several cell-surface proteins, such as the membrane-bound precursor of TNF-alpha.

PGBM, also known as Basement membrane-specific heparan sulfate proteoglycan core protein or Perlecan, has the Uniprot database accession number P98160. It is involved in fixing the negative electrostatic membrane charge and vascularization.

ITA3, also known as Integrin alpha-3 or VLA-3 subunit alpha, has the Uniprot database accession number P26006. It is a receptor for fibronectin, laminin, collagen, epiligrin, thrombospondin and CSPG4.

RUXE, also known as Small nuclear ribonucleoprotein E, has the Uniprot database accession number P62304. This protein is involved in histone 3'-end processing. May indirectly play a role in hair development.

PSMD2, also known as 26S proteasome non-ATPase regulatory subunit 2, has the Uniprot database accession number Q13200. It is involved in ATP-dependent degradation of ubiquitinated proteins.

VPS35, also known as Vacuolar protein sorting-associated protein 35, has the Uniprot database accession number Q96QK1. It is involved in the prevention of missorting of selected transmembrane cargo proteins into the lysosomal degradation pathway.

ANXA4, also known as Annexin A4 or Lipocortin IV, has the Uniprot database accession number P09525. It is involved in membrane fusion related-processes and exocytosis.

PLD3, also known as Phospholipase D3 or Choline phosphatase 3, has the Uniprot database accession number Q8IV08. This protein may be involved in APP processing. S10AC, also known as Protein S100-A12 or Calgranulin-C, has the Uniprot database accession number P80511. This protein is involved in the regulation of inflammatory processes and immune response.

CD14, also known as Monocyte differentiation antigen CD14, has the Uniprot database accession number P08571. This protein is a coreceptor for bacterial lipopolysaccharide (LPS) involved in the innate immune response to bacterial LPS.

LAMP2, also known as Lysosome-associated membrane glycoprotein 2, has the Uniprot database accession number P13473. It is involved in chaperone-mediated autophagy.

CLD6, also known as Claudin-6 or Skullin, has the Uniprot database accession number P56747. It is involved in tight junction-specific obliteration of the intercellular space.

IF2B3, also known as Insulin-like growth factor 2 mRNA-binding protein 3 or VICKZ family member 3, has the Uniprot database accession number O00425. It is involved in the recruitment of target transcripts to cytoplasmic protein-RNA complexes.

MLEC, also known as Malectin, has the Uniprot database accession number Q14165. This protein is involved in the early steps of protein N-glycosylation.

H10, also known as Histone H1.0 or Histone H1', has the Uniprot database accession number P07305. This protein is involved in condensation of nucleosome chains into higher-order structures.

CD166, also known as CD166 antigen, has the Uniprot database accession number Q13740. It is involved in both heterotypic and homotypic cell-cell contacts.

CD81, also known as CD81 antigen or Tetraspanin-28, has the Uniprot database accession number P60033. This protein may be involved the regulation of lymphoma cell growth.

AR6P1, also known as ADP-ribosylation factor-like protein 6-interacting protein 1, has the Uniprot database accession number Q15041. This protein is involved SLC1A1/EAAC1-mediated glutamate transport.

VAC14, also known as Protein VAC14 homolog or Tax1-binding protein 2, has the Uniprot database accession number Q08AM6. This protein is involved in the biogenesis of endosome carrier vesicles (ECV) / multivesicular bodies (MVB) transport intermediates from early endosomes.

ITB3, also known as Integrin beta-3, has the Uniprot database accession number P05106. This protein is involved in cell signaling transduction since it forms cellular receptors to several ligands, such as fibronectin, laminin or ostoeopontin among others.

PIGR, also known as polymeric immunoglobulin receptor, has the Uniprot database accession number P01833, June 26, 2007 - v4. This receptor binds polymeric IgA and IgM at the basolateral surface of epithelial cells.

VIME, also known as Vimentins, are class-III intermediate filaments found in various non-epithelial cells, especially mesenchymal cells. Vimentin is attached to the nucleus, endoplasmic reticulum, and mitochondria, either laterally or terminally. It has the Uniprot database accession number P08670, January 23, 2007 - v4.

CTNB1, also known as catenin beta-1, has the Uniprot database accession number P35222, February 1, 1994 - v1. It acts as a negative regulator of centrosome cohesion and blocks anoikis of malignant kidney and intestinal epithelial cells.

CAYP1, also known as calcyphosin, has the Uniprot database accession number Q13938, November 1, 1997 - v1. It is a calcium-binding protein that may play a role in cellular signaling events.

WFDC2, WAP four-disulfide core domain protein 2, is a broad range protease inhibitor, also known as Epididymal secretory protein E4. It has the Uniprot database accession number Q14508 January 23, 2002 - v2.

CADH1, also known as cadherin-1 or E-cadherin, has the Uniprot database accession number P12830, July 1, 1993 - v3. This protein is involved in mechanisms regulating cell-cell adhesions, mobility and proliferation of epithelial cells. It has a potent invasive suppressor role.

CD44, also known as CD44 antigen, has the Uniprot database accession number P16070, October 5, 2010 - v3. Mediates cell-cell and cell-matrix interactions through its affinity for HA, and possibly also through its affinity for other ligands such as osteopontin, collagens, and matrix metalloproteinases (MMPs).

XPO2, also known as exportin-2, has the Uniprot database accession number P55060, March 29, 2005 - v3. Among others, this protein has been disclosed as exporting receptor for importin-alpha, mediating importin-alpha re-export from the nucleus to the cytoplasm after import substrates (cargos) and binding cooperatively to importin-alpha and to the GTPase Ran in its active GTP-bound form.

SG2A1, also known as mammaglobin-B, has the Uniprot database accession number O75556, November 1, 1998 - v1. It may bind androgens and other steroids.

ENOA, also known as alpha-enolase, has the Uniprot database accession number P06733, January 23, 2007 - v2. It is a multifunctional enzyme that, as well as its role in glycolysis, plays a part in various processes such as growth control, hypoxia tolerance and allergic responses. May also function in the intravascular and pericellular fibrinolytic system due to its ability to serve as a receptor and activator of plasminogen on the cell surface of several cell-types such as leukocytes and neurons. Stimulates immunoglobulin production.

LEG3, known as galectin-3 and also referred as Galectin-3, is a Galactose-specific lectin which binds IgE. May mediate with the alpha-3, beta-1 integrin the stimulation by CSPG4 of endothelial cells migration. Together with DMBT1, required for terminal differentiation of columnar epithelial cells during early embryogenesis (By similarity). In the nucleus: acts as a pre-mRNA splicing factor. Involved in acute inflammatory responses including neutrophil activation and adhesion, chemoattraction of monocytes macrophages, opsonization of apoptotic neutrophils, and activation of mast cells. It has the Uniprot database accession number P17931, November 25, 2008 - v5.

LEG1 is also known as Protein LEG1 homolog, involved in early liver development. It has the Uniprot database accession number P09382, March 29, 2005 - v2.

CAPG, also known as macrophage-capping protein, has the Uniprot database accession number P40121, November 30, 2010 - v2. It is a calcium-sensitive protein which reversibly blocks the barbed ends of actin filaments but does not sever preformed actin filaments. It may play an important role in macrophage function.

PRDX1, also known as peroxiredoxin-1, has the Uniprot database accession number Q06830, June 1, 1994 - v1. It is involved in redox regulation of the cell.

CLIC1, also known as Chloride intracellular channel protein 1, has the Uniprot database accession number O00299, January 23, 2007 - v4. It inserts into membranes and form chloride ion channels. Channel activity depends on the pH. Membrane insertion seems to be redox-regulated and may occur only under oxydizing conditions. Involved in regulation of the cell cycle.

PDIA1, also known as protein disulfide-isomerase, has the Uniprot database accession number P07237, November 1, 1997 - v3. It catalyzes the formation, breakage and rearrangement of disulfide bonds.

KPYM, also known as pyruvate kinase PKM, has the Uniprot database accession number P14618, January 23, 2007 - v4. It is a glycolytic enzyme that catalyzes the transfer of a phosphoryl group from phosphoenolpyruvate (PEP) to ADP, generating ATP and plays a general role in caspase independent cell death of tumor cells.

GSTP1, also known as glutathione S-transferase P, has the Uniprot database accession number P09211, January 23, 2007 - v2. It regulates negatively CDK5 activity via p25/p35 translocation to prevent neurodegeneration GTR1 has the Uniprot database accession number P11166, October 3, 2006 - v2. It is a facilitative glucose transporter. This isoform may be responsible for constitutive or basal glucose uptake. It has a very broad substrate specificity; can transport a wide range of aldoses including both pentoses and hexoses.

CH10, also known as 10 kDa heat shock protein, mitochondrial, has the Uniprot database accession number P61604, January 23, 2007 - v2. It is essential for mitochondrial protein biogenesis, together with CPN60. Binds to CPN60 in the presence of Mg-ATP and suppresses the ATPase activity of the latter.

MIF, also known as macrophage migration inhibitory factor, has the Uniprot database accession number P14174, January 23, 2007 - v4. It is involved in the innate immune response to bacterial pathogens PEBP1, phosphatidylethanolamine-binding protein 1, it binds ATP, opioids and phosphatidylethanolamine. It is a serine protease inhibitor which inhibits thrombin, neuropsin and chymotrypsin but not trypsin, tissue type plasminogen activator and elastase (By similarity). Inhibits the kinase activity of RAF1 by inhibiting its activation and by dissociating the RAF1/MEK complex and acting as a competitive inhibitor of MEK phosphorylation. It has the Uniprot database accession number P30086, January 23, 2007 - v3.

TPIS, also known as triosephosphate isomerase, has the Uniprot database accession number P60174, October 19, 2011 - v3. This protein is involved in the pathway gluconeogenesis, which is part of carbohydrate biosynthesis.

NGAL, also known as Neutrophil gelatinase-associated lipocalin, has the Uniprot database accession number P80188, November 1, 1995 - v2. It is involved in apoptosis due to interleukin-3 (IL3) deprivation and in innate immunity.

LDHA, also known as L-lactate dehydrogenase A chain, has the Uniprot database accession number P00338, January 23, 2007 - v2. This protein is involved in step 1 of the subpathway that synthesizes (S)-lactate from pyruvate.

Some of these proteins have been related with EC gradation in tissue samples. However, no meaningful data are correlated with values in uterine fluid samples of the female genital tract. As above exposed, detection of markers in uterine fluids (aspirates or washings) imply the advantage of collecting data from routine sampling in clinical practice of the gynecological diseases, avoiding costs and importantly, tissue biopsies.

In a particular embodiment of the method of differential diagnosis of endometrial cancer, the uterine fluid sample is uterine aspirate fluid sample from the female genital tract.

In yet another particular embodiment, it comprises further determining the level of expression of BCAM.

In another particular embodiment, the method further comprises determining the level of expression of BCAM in an exosome-containing fraction isolated from a uterine aspirate.

Yet, in another particular embodiment, the method further comprises determining the level of expression of at least one set of proteins selected from the group consisting of AGR2, BCAM, PODXL; BCAM, PODXL; BCAM, PIGR; BCAM, RL29; BCAM, PODXL; CLD6, PPIA, AGRIN, BCAM; ANXA, BCAM; BCAM, RAB8A; BCAM, SYIC; and the sets listed in Table C.

In another particular embodiment of the method, the differential diagnosis of endometrial cancer is determined by distinguishing endometrioid endometrial cancer of non-endometroid endometrial cancer and non-cancer.

In another particular embodiment of the method, the level of expression is determined at the protein level.

In a more particular embodiment, the protein level is determined by an assay or technology selected from the group consisting of an immunoassay, a bioluminescence assay, a fluorescence assay, a chemiluminescence assay, electrochemistry assay, mass spectrometry, and combinations thereof.

In another particular embodiment, the level of expression of protein is determined by using an antibody or a fragment thereof able to bind to the protein.

More in particular, said antibody or fragment thereof forms part of the use of a kit.

As indicated, another aspect of the invention is the use of protein BCAM as *in vitro* marker for the prognosis of endometrial cancer in a uterine fluid sample from the female genital tract.

The invention also relates to the use of protein BCAM for the prognosis of endometrial cancer, in the method of any one of the aspects and embodiments.

Another aspect is also the use of a kit for the prognosis of endometrial cancer, and for differential diagnosis of endometrial cancer, the kit comprising a solid support and means for detecting the level of expression of BCAM, and optionally means for detecting the level of expression of one or more of the following proteins XPO2, PRDX1, CLIC1, PDIA1, KPYM, ENOA, GSTP1, GTR1, CH10, MIF, PEBP1, TPIS, NGAL, and LDHA.

In a particular embodiment of the use of the kit, the kit comprises a solid support and means for detecting the level of expression of AGR2, BCAM, PODXL; BCAM, PODXL; BCAM, PIGR; BCAM, RL29; BCAM, PODXL; CLD6, PPIA,

AGRIN, BCAM; ANXA, BCAM;BCAM, RAB8A; BCAM, SYIC; and the sets listed in Table C.

In a particular embodiment of the use of the kit, the means for detecting the level of expression of the proteins are means for carrying out an assay or technology selected from the group consisting of an immunoassay, a bioluminescence assay, a fluorescence assay, a chemiluminescence assay, electrochemistry assay, mass spectrometry, and combinations thereof.

More in particular, the means for detecting the level of expression of the proteins are antibodies or fragments thereof.

Another aspect of the invention is the use of a kit comprising a solid support and means for detecting the level of expression of BCAM.

Another aspect is the use of a kit comprising a solid support and means for detecting the level of expression of at least one set of proteins selected from the group consisting of AGR2, BCAM, PODXL; BCAM, PODXL; BCAM, PIGR; BCAM, RL29; BCAM, PODXL; CLD6, PPIA, AGRIN, BCAM; ANXA, BCAM;BCAM, RAB8A; BCAM, SYIC; and the sets listed in Table C.

In a particular embodiment of the use of the kits, they further comprise means for detecting the level of expression of at least the set of proteins BCAM, MMP9.

In a more particular embodiment of the use of the kits, the means for detecting the level of expression of the proteins are means for carrying out an assay or technology selected from the group consisting of an immunoassay, a bioluminescence assay, a fluorescence assay, a chemiluminescence assay, electrochemistry assay, mass spectrometry, and combinations thereof.

More in particular, the use of the kits comprises as the means for detecting the level of expression of the proteins, antibodies or fragments thereof.

In another particular embodiment of the use of the kits, they are kits for carrying out an enzyme-linked immunosorbent assay.

In another particular embodiment, the use of the kits further comprises a pannel diagram, to categorize an individual sample.

In another particular embodiment of this aspect of the invention, optionally in combination with any embodiment above or below, the uterine fluid sample is uterine aspirate fluid sample from the female genital tract.

It is highly advantageous that the proteins could be differentially detected in the uterine aspirate, since minimal manipulation is required and clinically meaningful information can be obtained.

In another particular embodiment of the first and other aspects of the invention, optionally in combination with any embodiment above or below, the method comprises determining the level of expression of two proteins comprising BCAM and a protein selected from the group consisting of PIGR, VIME, CTNB1, CAYP1, SG2A1, WFDC2, CADH1, CD44, LEG3, LEG1, and CAPG.

In another particular embodiment, the method comprises determining the level of expression of two proteins comprising BCAM and a protein selected from the group consisting of PIGR, VIME, CTNB1, CAYP1, SG2A1, WFDC2, CADH1, CD44, LEG3, LEG1, CAPG, AGR2, PODXL, MMP9, CD59, CLD6, IF2B3, PLD3, MX1, XPO2, PRDX1, CLIC1, PDIA1, KPYM, ENOA, GSTP1, GTR1, CH10, **MIF,** PEBP1, TPIS, NGAL, and LDHA.

In another particular embodiment of the first and further method aspects, the method comprises determining the level of expression of two proteins comprising BCAM and a protein selected from the group consisting of AGR2, PODXL, MMP9, CD59, CLD6, IF2B3, PLD3, and MX1.

In another particular embodiment of the first and further method aspects of the invention, optionally in combination with any embodiment above or below, the method comprises determining the level of expression of three proteins comprising BCAM and proteins selected from the group consisting of PIGR, VIME, CTNB1, CAYP1, SG2A1, WFDC2, CADH1, CD44, LEG3, LEG1, CAPG, AGR2, PODXL, MMP9, CD59, CLD6, IF2B3, PLD3, MX1, XPO2, PRDX1, CLIC1, PDIA1, KPYM, ENOA, GSTP1, GTR1, CH10, MIF, PEBP1, TPIS, NGAL, and LDHA, wherein at least one of the proteins is selected from the group consisting of PIGR, VIME, CTNB1, CAYP1, SG2A1, WFDC2, CADH1, CD44, LEG3, LEG1, and CAPG.

In another particular embodiment of the first and further method aspects of the invention, optionally in combination with any embodiment above or below, the method comprises determining the level of expression of three proteins comprising BCAM and proteins selected from the group consisting of PIGR, VIME, CTNB1, CAYP1, SG2A1, WFDC2, CADH1, CD44, LEG3, LEG1, CAPG, AGR2, PODXL, MMP9, CD59, CLD6, IF2B3, PLD3, MX1, XPO2, PRDX1, CLIC1, PDIA1, KPYM, ENOA, GSTP1, GTR1, CH10, MIF, PEBP1, TPIS, NGAL, and LDHA.

In another particular embodiment of the first and further method aspects of the invention, the method comprises determining the level of expression of three proteins comprising BCAM and proteins selected from the group consisting of AGR2, PODXL, MMP9, CD59, CLD6, IF2B3, PLD3, and MX1.

In another particular embodiment of the method for prognosis of EC, the method further comprises determining the level of expression of BCAM in an exosome-containing fraction isolated from uterine aspirate.

Inventors detected in a fraction of the uterine aspirate, said fraction comprising in a more purified form the

exosomes that certain markers (proteins) were differentially expressed in EEC and NEEC. Thus, the invention encompasses the optional step of determining in a processed uterine aspirate (the exosome fraction) particular markers vor verifying the prognostic determined in the uterine fluid, or for increasing the sensitivity of the method in case uterine fluid without isolation of exosomes gives no relevant data.

In a more particular embodiment, the method for the prognosis or of differential diagnosis of EC comprises determining the level of expression of at least one set of proteins selected from the group consisting of AGR2, BCAM, PODXL; BCAM, PODXL; BCAM, PIGR; BCAM, RL29; BCAM, PODXL; AGRIN, BCAM; ANXA, BCAM;BCAM, RAB8A; BCAM, SYIC; and the sets listed in Table C.

Particular combinations selected from AGR2, BCAM, PODXL; BCAM, PODXL; BCAM, PIGR (in Table B below); as well as those listed in Table C, (illustrated at the end of this description) were determined of high prognostic value when the levels of expression of the proteins were detected in a uterine aspirate. Next Table B shows AUC and Confidence interval (CI) of 95 % values. Data derive from the analysis of samples from EEC and NEEC. In Table C, also AUC values are indicated.

On the other hand, Table A shows AUC values and CI of 95 % of the following combinations: BCAM, RL29; BCAM, PODXL; CLD6, PPIA, AGRIN, BCAM; ANXA, BCAM; BCAM, RAB8A; BCAM, SYIC; giving important prognostic information when the levels of expression of the proteins were detected in an exosome-containing fraction isolated from uterine aspirate. Data derive from the analysis of samples from EEC and NEEC.

TABLE A

| PROTEIN 1 | PROTEIN 2 | AUC | CI 95% inf. | CI 95% sup. | Sample |
|---|---|---|---|---|---|
| CLD6 | RAB8A | 0,894 | 0,775 | 1 | Exosomes |
| CLD6 | PODXL | 0,931 | 0,849 | 1 | Exosomes |
| BCAM | RL29 | 0,922 | 0,857 | 0,987 | Exosomes |
| BCAM | PODXL | 0,908 | 0,823 | 0,993 | Exosomes |
| CLD6 | PPIA | 0,926 | 0,839 | 1 | Exosomes |
| AGRIN | BCAM | 0,903 | 0,821 | 0,984 | Exosomes |
| ANXA | BCAM | 0,919 | 0,838 | 0,999 | Exosomes |
| BCAM | RAB8A | 0,923 | 0,853 | 0,993 | Exosomes |
| BCAM | SYIC | 0,913 | 0,825 | 1 | Exosomes |
| CLD6 | IFB3 | 0,894 | 0,775 | 1 | Exosomes |

TABLE B

| PROTEIN 1 | PROTEIN 2 | PROTEIN 3 | AUC | CI 95% inf. | CI 95% sup. | Sample |
|---|---|---|---|---|---|---|
| LAMP2 | MMP9 | PIGR | 0,984 | 0,956 | 1 | Uterine aspirate |
| AGRIN | MMP9 | PIGR | 0,98 | 0,946 | 1 | Uterine aspirate |
| AGR2 | PIGR | PLD3 | 0,977 | 0,94 | 1 | Uterine aspirate |
| AGR2 | BCAM | PODXL | 0,973 | 0,933 | 1 | Uterine aspirate |
| BCAM | PODXL | | 0,943 | 0,862 | 1 | Uterine aspirate |
| PIGR | PLD3 | | 0,949 | 0,871 | 1 | Uterine aspirate |
| BCAM | PIGR | | 0,936 | 0,863 | 1 | Uterine aspirase |

Also described but not part of the invention is a method that comprises determining the level of expression of CTNB1, XPO2 and CAPG. As will be illustrated and depicted below (FIG.1) this combination allows a highly sensitive and specific differential diagnosis of EEC versus NEEC (serous EC; SEC).

In another particular embodiment of the first and further method aspects of the invention, optionally in combination with any embodiment above or below, the method comprises determining the level of expression of four, five, six, seven, eight, nine, ten or eleven proteins comprising BCAM and at least one of the proteins selected from the group consisting of PIGR, VIME, CTNB1, CAYP1, SG2A1, WFDC2, CADH1, CD44, LEG3, LEG1, and CAPG.

In another particular embodiment of the first and further method aspects of the invention, optionally in

combination with any embodiment above or below, the method comprises determining the level of expression of the following proteins PIGR, VIME, CTNB1, CAYP1, SG2A1, WFDC2, CADH1, CD44, LEG3, LEG1, CAPG, AGR2, BCAM, PODXL, MMP9, and CD59.

In any of the embodiments provided above or below, for any of the aspects of the invention, the level of expression is determined at the protein level. In this embodiment, the protein marker(s) include, but do not limit to, native-sequence peptides, isoforms, chimeric polypeptides, all homologs, fragments, and precursors of the markers, including modified forms of the polypeptides and derivatives thereof. In a more particular embodiment the protein level is determined by an assay or technology selected from the group consisting of an immunoassay, a bioluminescence assay, a fluorescence assay, a chemiluminescence assay, electrochemistry assay, mass spectrometry, and combinations thereof.

In particular embodiments provided above or below, the level of expression is determined by immunochemistry.

The term "immunochemistry" as used herein refers to a variety of techniques for detecting antigens (usually proteins and peptides, and in the present case any of the proteins listed above alone or in combination) in a sample by exploiting the principle of antibodies binding specifically to said antigens. Visualizing an antibody-antigen interaction can be accomplished in a number of ways. In the most common instance, an antibody is conjugated to an enzyme, such as peroxidase, that can catalyse a colour-producing reaction. Alternatively, the antibody can also be tagged to a fluorophore, such as fluorescein or rhodamine. The immunochemistry technique can be direct or indirect. The direct method is a one-step staining method and involves a labeled antibody (e.g. FITC-conjugated antiserum) reacting directly with the antigen. While this technique utilizes only one antibody and therefore is simple and rapid, the sensitivity is lower due to little signal amplification, such as with indirect methods, and is less commonly used than indirect methods. The indirect method involves an unlabeled primary antibody (first layer) that binds to the target antigen in the sample and a labeled secondary antibody (second layer) that reacts with the primary antibody. This method is more sensitive than direct detection strategies because of signal amplification due to the binding of several secondary antibodies to each primary antibody if the secondary antibody is conjugated to the fluorescent or enzyme reporter.

Further amplification can be achieved if the secondary antibody is conjugated to several biotin molecules, which can recruit complexes of avidin-, streptavidin or Neutravidin-enzyme. The indirect method, aside from its greater sensitivity, also has the advantage that only a relatively small number of standard conjugated (labeled) secondary antibodies needs to be generated. With the direct method, it would be necessary to label each primary antibody for every antigen of interest. It must be borne in mind that immunochemistry techniques can also be used to detect certain nucleic acid sequences if a tagged nucleic acid probe (designed to specifically bind to a certain target nucleic acid sequence) can later on be detected with a labelled antibody. Thus, the detection of the protein could be performed by using a tagged nucleic acid designed to bind a specific sequence of the target protein RNA, and then detecting said tagged nucleic acid with a labelled antibody which selectively binds to the tag.

Immunoassay procedures suitable include enzyme-linked immunosorbent assays (ELISA, such as multiplex ELISA), enzyme immunodot assay, agglutination assay, antibody-antigen-antibody sandwich assay, antigen-antibody-antigen sandwich assay, immunocromatography, or other immunoassay formats well-known to the ordinarily skilled artisan, such as radioimmunoassay, as well as protein microarray formats.

In one embodiment, in combination with any of the embodiments provided above or below, the level of expression of protein is determined by an immunoassay.

In another embodiment, in combination with any of the embodiments provided above or below, the level of expression of protein is determined by ELISA; more in particular multiplex ELISA.

Alternatively, the level of expression of protein can be determined by bioluminescence, fluorescence, chemiluminescence, electrochemistry, or mass spectrometry.

Alternatively, the level of expression of protein can be determined by measuring the levels of proteotypic peptides of the protein (peptides with an amino acid sequence uniquely associated with the studied protein in a given proteome) by mass spectrometry.

In another embodiment, in combination with any of the embodiments provided above or below, the level of expression of protein is determined using an antibody or a fragment thereof able to bind to the target protein(s).

The term "antibody or a fragment thereof able to bind to the target protein(s)" is to be understood as any immunoglobulin or fragment thereof able to selectively bind the target protein. It includes monoclonal and polyclonal antibodies. The term "fragment thereof encompasses any part of an antibody having the size and conformation suitable to bind an epitope of the target protein. Suitable fragments include F(ab), F(ab') and Fv. An "epitope" is the part of the antigen being recognized by the immune system (B-cells, T-cells or antibodies).

The antibodies used for specific detection can be polyclonal or monoclonal. There are well known means in the state of the art for preparing and characterizing antibodies. Methods for generating polyclonal antibodies are well known in the prior art. Briefly, one prepares polyclonal antibodies by immunizing an animal with the protein; then, serum from the immunized animal is collected and the antibodies isolated. A wide range of animal species can be used for the production of the antiserum. Typically the animal used for production of antisera can be a rabbit, mouse, rat, hamster, guinea pig or goat.

Moreover, monoclonal antibodies (MAbs) can be prepared using well-known techniques. Typically, the procedure involves immunizing a suitable animal with the protein associated with the disease. The immunizing composition can be administered in an amount effective to stimulate antibody producing cells. Methods for preparing monoclonal antibodies are initiated generally following the same lines as the polyclonal antibody preparation. The immunogen is injected into animals as antigen. The antigen may be mixed with adjuvants such as complete or incomplete Freund's adjuvant. At intervals of two weeks, approximately, the immunization is repeated with the same antigen.

In another particular embodiment of the third aspect, the means to carry out the invention form part of a kit. The antibody or fragment thereof for detecting the target protein(s) can be included in a kit. The kit may additionally comprise means (additives, solvents) to visualize the antibody-protein interactions.

These antibodies can be used as "means" for determining the expression of the target proteins in the fifth aspect of the invention.

Thus, in a particular embodiment of the first and further method aspects of the invention, the level of expression of a protein is determined using an antibody or a fragment thereof able to bind to the protein.

In another particular embodiment, said antibody or fragment thereof forms part of a kit.

All embodiments provided above, under the first and further method aspects of the invention, regarding the proteins to be analyzed (from two to eleven of the list), are also particular embodiments of the use of the second, third and fourth aspects of the invention.

Alternatively, the level of expression is determined at the mRNA level.

In one embodiment, the amount of mRNA of each one of the markers are detected via polymerase chain reaction using, for example, oligonucleotide primers that hybridize to one or more polynucleotide endometrial cancer markers or complements of such polynucleotides. Within other embodiments, the amount of mRNA is detected using a hybridization technique, employing oligonucleotide probes that hybridize to one or more polynucleotide endometrial cancer markers or complements of such polynucleotides.

When using mRNA detection, the method may be carried out by combining isolated mRNA with reagents to convert to cDNA according to standard methods well known in the art, treating the converted cDNA with amplification reaction reagents (such as cDNA PCR reaction reagents) in a container along with an appropriate mixture of nucleic acid primers; reacting the contents of the container to produce amplification products; and analyzing the amplification products to detect the presence of one or more of the polynucleotide endometrial cancer markers in the sample. For mRNA, the analyzing step may be accomplished using Northern Blot analysis to detect the presence of polynucleotide endometrial cancer markers in the sample. The analysis step may be further accomplished by quantitatively detecting the presence of polynucleotide endometrial cancer markers in the amplification product, and comparing the quantity of marker detected against a panel of expected values for the known presence or absence of such markers in normal and malignant tissue derived using similar primers.

In another embodiment, the invention provides a method wherein mRNA is detected by: (a) isolating mRNA from a sample and combining the mRNA with reagents to convert it to cDNA; (b) treating the converted cDNA with amplification reaction reagents and nucleic acid primers that hybridize to one or more of the polynucleotide endometrial cancer markers endometrial cancer marker to produce amplification products; (c) analyzing the amplification products for determining the amount of mRNA present encoding the protein endometrial cancer marker; and (d) comparing the determined amount of mRNA to an amount detected against a panel of expected values for normal and diseased tissue (e.g. , malignant tissue) derived using similar methods.

In particular embodiments of the invention, RT-PCR can be used to amplify the mRNA for protein endometrial cancer markers for detection and analysis. Other embodiments of the invention use quantitative RT-PCR to quantitatively determine amount of mRNA for protein endometrial cancer markers. Further embodiments of the invention use real time RT-PCR for quantification and analysis.

Regarding the device or kits of the invention, said kits are, in particular embodiments of the invention provided for the analysis of patient samples. Such devices or kits will include reagents that specifically identify one or more proteins, where at least a subset of proteins is selected from proteins listed above, as well as from Tables A to F, listed below. Devices of interest include arrays, where the reagents are spatially separated on a substrate such as a slide, gel, multi-well plate, etc. Alternatively, the reagents may be provided as a kit comprising reagents in a suspension or suspendable form, e.g. reagents bound to beads. Reagents of interest include reagents specific for autoantibody markers. Such reagents may include antigenic proteins or peptides, and the like. Such devices or kits may further comprise cytokine-specific antibodies or fragments thereof; and the like.

As above indicated, one aspect of the invention is the use of the kits comprising a solid support and means for detecting the level of expression of BCAM, and optionally means for detecting the level of expression of one or more of the following proteins XPO2, PRDX1, CLIC1, PDIA1, KPYM, ENOA, GSTP1, GTR1, CH10, **MIF,** PEBP1, TPIS, NGAL, and LDHA, said kits for the prognosis of endometrial cancer, by distinguishing EEC of NEEC.

In a particular embodiment of the use of the kits, the kits are those comprising a solid support and means for detecting the level of expression of two, three, four, five, six, seven, eight, nine, ten and eleven proteins comprising BCAM

and proteins selected from the group consisting of PIGR, VIME, CTNB1, CAYP1, SG2A1, WFDC2, CADH1, CD44, LEG3, LEG1, CAPG, AGR2, PODXL, MMP9, CD59, CLD6, IF2B3, PLD3 and MX1, XPO2, PRDX1, CLIC1, PDIA1, KPYM, ENOA, GSTP1, GTR1, CH10, MIF, PEBP1, TPIS, NGAL, and LDHA. More particularly, the kits comprising a solid support and means for detecting the level of expression of three of these proteins.

In a particular embodiment of the use of the kits, they are kits comprising a solid support and means for detecting the level of expression of at least one set of proteins selected from the group consisting of AGR2, BCAM, PODXL; BCAM, PODXL; BCAM, PIGR; BCAM, RL29; BCAM, PODXL; CLD6, PPIA, AGRIN, BCAM; ANXA, BCAM; BCAM, RAB8A; BCAM, SYIC; and the sets listed in Table C.

In another particular embodiment of the use of the kits, the means for detecting the level of expression of the proteins are antibodies or fragments thereof that specifically bind to the target protein(s).

According to the fifth aspect, the invention relates to the use of kits comprising a solid support and means for detecting the level of expression of BCAM.

In a particular embodiment of the fifth aspect of the use of the kits, the kits comprise a solid support and means for detecting the level of expression of at least one set of proteins selected from the group consisting of AGR2, BCAM, PODXL; BCAM, PODXL; BCAM, PIGR; BCAM, RL29; BCAM, PODXL; CLD6, PPIA, AGRIN, BCAM; ANXA, BCAM;BCAM, RAB8A; BCAM, SYIC; and the sets listed in Table C.

In a more particular embodiment, the use of the kits comprises means for detecting the level of expression of a combination from 2 to 500 sets, and more particularly from 2 to 400.

In another particular embodiment, optionally in combination with any of the embodiments above or below, the kits further comprise means for detecting the level of expression of at least the set of proteins consisting of
BCAM, MMP9.

Particular combinations selected from next Table E; as well as those listed in Table D, (illustrated at the end of this description) were determined of high diagnostic value when the levels of expression of the proteins were detected in a uterine aspirate. Next Table E shows AUC and Confidence interval (CI) of 95 % values. Data derive from the analysis of samples from non-EC and EC (including EEC and NEEC). In Table D, also AUC are indicated.

TABLE E. Combinations of diagnostic value in uterine aspirate

| PROTEIN 1 | PROTEIN 2 | PROTEIN 3 | AUC | CI 95% inf. | CI 95% sup. | Sample |
|---|---|---|---|---|---|---|
| MMP9 | PODXL | RAB8A | 0,976 | 0,942 | 1 | Uterine aspirate |
| MMP9 | PODXL | RSSA | 0,981 | 0,956 | 1 | Uterine aspirate |
| AGRIN | MMP9 | PODXL | 0,979 | 0,953 | 1 | Uterine aspirate |
| MMP9 | PODXL | VAMP8 | 0,971 | 0,935 | 1 | Uterine aspirate |
| MMP9 | MX1 | | 0,951 | 0,891 | 1 | Uterine aspirate |
| MMP9 | RSSA | | 0,962 | 0,919 | 1 | Uterine aspirate |
| MMP9 | MVP | | 0,956 | 0,906 | 1 | Uterine aspirate |
| MMP9 | RAB8A | | 0,96 | 0,918 | 1 | Uterine aspirate |
| MMP9 | VAMP8 | | 0,952 | 0,905 | 1 | Uterine aspirate |
| BCAM | MMP9 | | 0,954 | 0,898 | 1 | Uterine aspirate |
| MMP9 | AGRIN | | 0,94 | 0,89 | 0,992 | Uterine aspirate |

TABLE F. Combinations of diagnostic value in an exosome-containing fraction isolated from uterine aspirate

| PROTEIN 1 | PROTEIN 2 | PROTEIN 3 | AUC | CI 95% inf. | CI 95% sup. | Sample |
|---|---|---|---|---|---|---|
| AGRIN | CD81 | TERA | 0,944 | 0,902 | 0,986 | Exosomes |
| AGRIN | CD59 | MVP | 0,944 | 0,902 | 0,986 | Exosomes |
| AGR2 | AGRIN | CD81 | 0,943 | 0,903 | 0,982 | Exosomes |
| AGRIN | CD166 | MVP | 0,938 | 0,896 | 0,98 | Exosomes |
| AGRIN | CD81 | | 0,934 | 0,89 | 0,979 | Exosomes |
| AGRIN | CD166 | | 0,926 | 0,878 | 0,975 | Exosomes |
| AGRIN | CD59 | | 0,921 | 0,871 | 0,97 | Exosomes |

(continued)

| PROTEIN 1 | PROTEIN 2 | PROTEIN 3 | AUC | CI 95% inf. | CI 95% sup. | Sample |
|---|---|---|---|---|---|---|
| AGRIN | MMP9 | | 0,9 | 0,848 | 0,961 | Exosomes |

On the other hand, Table F shows AUC values and CI of 95 % of the following combinations: AGRIN, CD81, TERA; AGRIN, CD59, MVP; AGR2, AGRIN, CD81; AGRIN, CD166, MVP; AGRIN, CD81; AGRIN, CD166; AGRIN, CD59; AGRIN, MMP9, giving important diagnostic information when the levels of expression of the proteins were detected in an exosome-containing fraction isolated from uterine aspirate. Data derive from the analysis of samples from non-EC and EC (including EEC and NEEC).

As indicated for the fourth aspect, in a particular embodiment of this fifth aspect, the means for detecting the level of expression of the proteins are antibodies or fragments thereof that specifically bind to the target protein(s).

In another particular embodiment of the fourth and fifth aspects of the invention, the use of the kits are use of ELISA kits. In this embodiment, the kit comprises a solid support and means for determining the level of expression of any of the proteins and combinations of proteins provided above. In another embodiment, the kit comprises a solid support and antibodies or fragments thereof which specifically bind to the target proteins to be detected, these antibodies being conjugated with a reporter molecule capable of producing a signal.

The "solid support" includes a nitrocellulose membrane, glass or a polymer. The most commonly used polymers being cellulose, polyacrylamide, nylon, polystyrene, polyvinyl chloride or polypropylene. The solid supports may be in the form of strips, tubes, beads, discs or microplates, or any other surface suitable for conducting an immunoassay.

The "reporter molecule" as used in the present specification is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (ie., radioisotopes). In the case of an enzyme immunoassay, an enzyme is conjugated to the second antibody, generally by means of glutaraldehyde or periodate. As will be readily recognized, however, a wide variety of different conjugation techniques exist, which are readily available to those skilled in the art. Commonly used enzymes include horseradish peroxidase, glucose oxidase, β-galactosidase and alkaline phosphatase, among others. The substrates to be used with the specific enzymes are generally chosen for the production, upon hydrolysis by the corresponding enzyme, of a detectable colour change. For example, 5-bromo-4-chloro-3-indolyl phosphate/nitroblue tetrazolium is suitable for use with alkaline phosphatase conjugates; for peroxidase conjugates, 1,2-phenylenediamine, 5-aminosalicylic acid, 3,3:5,5:tetra methyl benzidine or tolidine are commonly used. It is also possible to employ fluorogenic substrates, which yield a fluorescent product rather than the chromogenic substrates noted above. Examples of fluorogenic substrates are fluorescein and rhodamine. When activated by illumination with light of a particular wavelength, the fluorochrome-labelled antibody absorbs the light energy, inducing a state of excitability in the molecule, followed by emission of the light at a characteristic colour visually detectable with a light microscope. Immunofluorescence and EIA techniques are both well established in the art and are particularly preferred for the present method. However, other reporter molecules, such as radioisotope, chemiluminescent, and bioluminescent molecules and/or dyes and other chromogenic substances, may also be employed.

The choice of a particular reporter molecule conjugated antibody will be, for the most part, determined by the intended use and user of the test kit of the present invention.

Binding assays for measuring biomarker levels may use solid phase or homogenous formats. Suitable assay methods include sandwich or competitive binding assays. Examples of sandwich immunoassays are described in U.S. Pat. No. 4,168,146 and U.S. Pat. No. 4,366,241. Examples of competitive immunoassays include those disclosed in U.S. Pat. No. 4,235,601, U.S. Pat. No. 4,442,204 and U.S. Pat. No. 5,208,535.

Multiple biomarkers may be measured using a multiplexed assay format, e.g., multiplexing through the use of binding reagent arrays, multiplexing using spectral discrimination of labels, multiplexing of flow cytometric analysis of binding assays carried out on particles, e.g., using the Luminex® system.

The assays of the present invention may be conducted by any suitable method. In one embodiment, biomarker levels are measured in a single sample, and those measurement may be conducted in a single assay chamber or assay device, including but not limited to a single well of an assay plate, a single assay cartridge, a single lateral flow device, a single assay tube, etc. Biomarker levels may be measured using any of a number of techniques available to the person of ordinary skill in the art, e.g., direct physical measurements (e.g., mass spectrometry) or binding assays (e.g., immunoassays, agglutination assays and immunochromatographic assays). The method may also comprise measuring a signal that results from a chemical reactions, e.g., a change in optical absorbance, a change in fluorescence, the generation of chemiluminescence or electrochemiluminescence, a change in reflectivity, refractive index or light scattering, the accumulation or release of detectable labels from the surface, the oxidation or reduction or redox species, an electrical current or potential, changes in magnetic fields, etc. Suitable detection techniques may detect binding events by

measuring the participation of labeled binding reagents through the measurement of the labels via their photoluminescence (e.g., via measurement of fluorescence, time-resolved fluorescence, evanescent wave fluorescence, up-converting phosphors, multi-photon fluorescence, etc.), chemiluminescence, electrochemiluminescence, light scattering, optical absorbance, radioactivity, magnetic fields, enzymatic activity (e.g., by measuring enzyme activity through enzymatic reactions that cause changes in optical absorbance or fluorescence or cause the emission of chemiluminescence). Alternatively, detection techniques may be used that do not require the use of labels, e.g., techniques based on measuring mass (e.g., surface acoustic wave measurements), refractive index (e.g., surface plasmon resonance measurements), or the inherent luminescence of an analyte.

In another embodiment, the use of the kit is a microarray.

In another embodiment, the use of the kit is a microarray including a defined set of genes encoding protein endometrial cancer markers. All the embodiments provided above for particular proteins to be analyzed (from two to eleven of the list), whose expression is significantly altered by endometrial disease, are also particular embodiments of microarrays.

In another particular embodiment, the use of the kits of the invention further comprises a pannel diagram, to categorize an individual sample.

A further aspect of the present invention was a method of deciding or recommending whether to initiate a medical regimen of a subject suffering endometrial cancer in function of the prognosis. In a particular embodiment of this method, it comprises

a) determining, *in vitro,* the level of expression of BCAM and optionally the level of expression of one or more of proteins XPO2, PRDX1, CLIC1, PDIA1, KPYM, ENOA, GSTP1, GTR1, CH10, MIF, PEBP1, TPIS, NGAL, and LDHA in an uterine fluid sample from the subject's female genital tract; and
b) establishing the prognosis of said EC by distinguishing EEC and NEEC, if the protein level in the test sample is higher than a reference control level for BCAM;

wherein:

i) if the subject is diagnosed of suffering from endometrial cancer, or of being suspicious of suffering from endometrial cancer, and the subject is differentially diagnosed of suffering EEC, then the initiation of the medical regimen for EEC is recommended, which consists of total hysterectomy and bilateral salpingo-oophorectomy, optionally complemented with lymphadenectomy;

ii) if the subject is diagnosed of suffering from endometrial cancer, or of being suspicious of suffering from endometrial cancer, and the subject is differentially diagnosed of suffering NEEC, then the initiation of the medical regimen for NEEC is recommended, which consists of total hysterectomy and bilateral salpingo-oophorectomy, pelvic and para-aortic lymphadenectomy, omentectomy, and peritioneal biopsies.

In another particular embodiment method of deciding or recommending whether to initiate a medical regimen of a subject suffering endometrial cancer in function of the established prognosis, an adjuvant treatment is recommended selected from radiotherapy, chemotherapy and combinations thereof if the subject is diagnosed of suffering EEC. In yet another particular embodiment, and if the subject is diagnosed of suffering NEEC, chemotherapy is recommended as adjuvant treatment.

Also described but not part of the invneiton is a method for the prognosis of endometrial cancer by distinguishing endometrioid endometrial cancer (EEC) of non-endometroid endometrial cancer (NEEC), the method comprising determining the level of expression of one or more of the following PIGR, and VIME proteins in an isolated sample from the female genital tract.

Inventors detected for the first time that these two proteins were useful in distinguishing EC subtypes by departing from a sample of the female genital tract. The sample can in particular be selected from a tissue biopsy of the uterine organ and a fluid of the genital tract (i.e. uterine aspirate). This method comprises further detecting the expression level of BCAM. The expression level is in particular determined at protein level, in particular by means of antibodies or fragments of said antibodies that specifically bind the protein(s). Kits of parts and means that are used for carrying out the method are also part of the invention.

With the assay performed by the inventors, there has been identified a protein giving critical information of EC. This is the case of VIME, which was differentially expressed in EEC samples and NEEC (SEC) samples. Thus, this proteins not only allow the classification of the EC subtype but also confirming diagnosis. Invention encompasses a method of diagnosis and/or prognosis of EC, the method comprising determining the level of expression of VIME and in an isolated sample of a female, in particular from the female genital tract.

Besides, assayed samples allow determining the differential expression of some proteins in the fluid sample from

the female genital tract between EC and endometrial hyperplasia. Endometrial hyperplasia is a thickening of the endometrium caused by the excess of strogen stimuli. It is a benign disease. However, it is considered a precursor lesion of EC, and should be distinctively diagnosed. In an assay with EC samples (of any subtype; EEC and NEEC) and with non-EC samples controls includig hyperplasia cases, 16 proteins were found differentially abundant with adjusted p-values <0.05 and fold changes higher than 2 between hyperplasias and the other non-EC controls (women with normal endometrium or benign diseases different from hyperplasias, such as myomas or polyps). Four of them, NAMPT, ENOA, CATD and GSTP1 showed AUC higher than 0.85. After proper validation, they open to a diagnose of hyperplasias from EC. Thus, the invention also relates to a method for diagnosis and/or prognosis of endometrial hyperplasia in a subject, the method comprising determining the level of expression of one or more of the following proteins: NAMPT, ENOA, CATD and GSTP1, in an isolated sample of a female, in particular from the female genital tract. Early detection of the hyperplasia, precursor lesion of EC, allows increasing the follow-on of patients in order to detect as soon as possible any malignancy. Related with this aspect, the invention also provides a method for monitoring a medical regimen for hyperplasia using the one or more of the NAMPT, ENOA, CATD and GSTP1 markers of the invention: a decrease or return to a normal level of the marker (i.e., to the level of a hypetplasia-free control subject) can indicate that the patient has reacted favourably to the medical regimen and, therefore, said regimen is effective; if the level of the marker does not significantly change or it increases, this can indicate that the regimen is not effective and/or there is high risk of an hyperplasia evolving to EC. In those cases, a surgical treatment should be performed.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

## Examples

### Example 1. Biomarkers of diagnosis and prognosis of EC in exosomes derived from uterine fluid, and in the uterine fluid.

1. Samples, reagents and methods

1.1 Patients and sample description

Patients were recruited at three different institutions: HUVH (Hospital Universitari Vall d'Hebron, Barcelona, Spain), HUAV (Hospital Universitari Arnau de Vilanova, Lleida, Spain) and UMCF (University Medical Center of Freiburg, Freiburg, Germany). Each participating institution obtained ethical approval and samples were obtained after the participants signed the informed consent.

Uterine aspirates (UAs) were obtained by aspiration with a Cornier Pipelle (Gynetics Medical Products). Samples were placed in 1.5 mL tubes and kept on ice through all the processing which included addition of phosphate buffered saline (PBS) in a 1:1 ratio (v/v), gently pipetting of the sample, and centrifugation at 2500g (4 °C) in a F45-30-11 rotor (Eppendorf Microcentrifuge 5417R) for 20 min to remove the cellular fraction. The remaining supernatant (SN) fraction, was then aliquoted and frozen at -80 °C until needed.

For exosome (abbreviated ELVs) isolation, ELVs were obtained from the SNs of UAs by differential centrifugation, following a modification of a previously described ELVs isolation protocol by Thery et al. http://www.ncbi.nlm.nih.gov/pubmed/18228490. Briefly, SNs were thawed and diluted in PBS to a final volume of 25 mL. A centrifugation step at 10,000xg (4 °C) for 30 min was performed on a Thermo Scientific Heraeus MultifugeX3R Centrifuge (FiberLite rotor F15-8x-50c) to remove cell debris, macroparticles and apoptotic bodies. The resulting pellet enriched in MVs was resuspended in 50 μL of PBS and frozen at -80 °C. Then, the supernatant was transferred to ultracentrifuge tubes (Beckman Coulter) and filled with PBS to perform a first ultracentrifugation step at 100,000xg (4 °C) for 2 h on a Thermo Scientific Sorvall WX UltraSeries Centrifuge with an AH-629 rotor. The supernatant of this second centrifugation was the soluble fraction and was frozen at -80 °C. This first pellet was resuspended in PBS and again centrifuged at 100,000xg (4 °C) for 1 h. The final pellet enriched in ELVs (possibly along with microvesicles (MVs) and some remaining apoptotic bodies) was resuspended in 50 μL of PBS. Five microliters from MVs and ELVs pellets were reserved at -80 °C for particle size distribution and quantification by NTA while the rest of the sample was frozen at -80 °C for protein extraction.

1.2 Protein extraction and identification by Liquid chromatography-parallel reaction monitoring (LC-PRM) analysis

For the discovery phase, ELVs were resuspended in a lysis buffer composed of 40 mM Tris pH 8, 300 mM NaCl,

10 mM EDTA, 2 %Triton X-100 and 1:100 protease inhibitors (Sigma-Aldrich) in a 1:1 ratio (v/v). Then, samples were frozen at -20°C for at least 8 hours and then thawed on ice and sonicated five cycles of 5 seconds at amplitude 100% (Labsonic M, Sartorius Stedim Biotech) to ensure membrane disruption. The extracted proteins were stored at -20°C until needed.

Validation phase was performed on ELVs and on the uterine aspirate (UA) without need of isolating ELVs. To extract proteins from ELVs for the validation phase, the detergent contained in the lysis buffer was changed for <1% NP-40, to make the protein extraction already suitable for direct in-solution digestion and LC-MS/MS. The rest of the procedure remained the same. To extract proteins from the fluid fractions of UAs; those were sonicated 5 cycles of 5 seconds each at 100% amplitude (Labsonic M, Sartorius Stedim Biotech) to disrupt microvesicles, protein aggregates, and/or mucus present in the sample. Then, the Albumin & IgG depletion spin trap Kit (GE Healthcare) was used following the manufacturer's instructions to remove albumin and immunoglobulin G from the samples. All the extracted proteins were stored at -20°C until needed. Diagnostic and prognostic value data were also obtained with uterine fluid sample (ELVs or UA without isolation of ELVs) in which no depletion of albumin and immunoglobulin G was carried out.

For the discovery phase, peptides dissolved in 0.1% formic acid were first loaded on a 150 μm ID x 20 cm nano-LC in a house packed column (Jupiter C18, 3 μm, 300 Å, Phenomenex, Torrance, CA) and separated with an EASY nanoLC system (Thermo Scientific). For the elution of the peptides, a 1 hour linear gradient of 5-40% ACN (0.2% FA) at a constant flow rate of 600nL/min was used. The LC system was coupled to a QExactive plus Orbitrap tandem mass spectrometer (Thermo Fisher Scientific) and RAW files were acquired with XCalibur software (Thermo Fisher Scientific). Tandem mass spectra were performed with a Top-12 method with precursor isolation window of m/z 2.0. The resolution was 70.000 at m/z 400 for the survey scan (with AGC $1e^6$, maximal injection time 200 ms and a scan range of m/z 300-2000) and 17.500 for MS/MS spectra (AGC at $5e^5$, maximal injection time 50 ms and scan range m/z 200-2000). Normalized collision energy (NCE) was set at 25 and exclusion time was set to 10 s.

Regarding protein identification, for SILAC quantification, RAW files were analyzed using MaxQuant (version 1.3.0.3). Default parameters were taken: MS/MS fragment error tolerance of 20 ppm, Carbamidomethylatin (C) fixed and Oxidation (M) as well as Acetyl (Protein N-term) as variable modification. Arginine (Arg10) and lysine (Lys8) were selected for the heavy label and applied the "match between run" option. The RAW files were searched against the HUMAN SwissProt database. For the Presence/Absence analysis, a label free analysis was done with PEAKS 7.0 (http://www.bioinfor.com/). For the database search, RAW files were searched against the Human Uniprot database containing 37254 entries. For the search parameters, precursor error tolerance was set at 10 ppm and for the MS2 fragments the tolerance was at 0.01Da. A maximum of 2 misscleavages for Trypsin were allowed. Carbamidometylation (C), Deamidation (NQ) and Oxidations (M) were variable modifications. Only peptides with false discovery rates (FDR) lower 1% were considered. For validation of potential biomarkers, PEAKS results were uploaded in Scaffold proteome software (http://www.proteomesoftware.com/).

The statistical analysis of the discovery phase was performed to obtain two different outpouts: (1) A qualitative data consisting of proteins that were present or absent in the different subgroups of patients; and (2) a quantitative data consisting of the expression measures obtained from SILAC ratios representing relative abundance of each protein vs. internal standards.

For the qualitative data, a Fisher exact test was applied to each protein to compare presence/absence between groups (p-value < 0.001). For the quantitative data, a Student t-test was performed between each pair of groups in order to select differentially expressed proteins. The test was performed only for those proteins that were present in more than 4 individuals per group. In order to address the problem of multiple comparisons derived from performing many tests (one per protein), the p-values were adjusted to obtain a strong control over FDR using the Benjamini and Hockberg methods (adj.pvalue < 0.25 and FC > 1.3).

All analyses were performed using the statistical program "R" (R version 3.2, Copyright (C) 2015 The R Foundation for Statistical Computing).

A total of 54 proteins were selected for the targeted experiment by selected reaction monitoring (SRM) based on the results from the discovery phase (49), together with 5 candidates selected from previous results of our group. Two unique peptides per protein were selected to be monitored by SRM based on their detectability in previous mass spectrometric experiments. For each selected peptide, an isotopically-labeled version ($^{15}N_2$,$^{13}C_6$-Lysine, 15N4,13C6-Arginine) was bought and spiked in each sample to be used as internal standard. Internal standards were spiked in a concentration within the linear response range, which was established for each individual peptide based on experimental dilution curves (data not shown). In parallel, isotopically-labeled peptides were mixed and used to generate MS2 spectral library and retention time knowledge database.

A total of 85 endogenous peptides and their corresponding internal standards were measured by SRM in Lys-C and trypsin-digested samples from an independent cohort of 107 patients. The five most intense transitions per peptide were monitored on a triple quadrupole mass spectrometer (5500 Q-Trap, AB Sciex Instruments, Foster, CA, USA) equipped a reversed-phase chromatography 25-cm column with an inner diameter of 75 μM, packed with 1.9 μM C18 particles (NikkyoTechnos Co., Ltd. Japan) and a 2-cm pre-column (Acclaim PepMap 100, C18, 15 μM, 100A). Loading

buffer: H2O with 0.1% formic acid; eluting buffer: ACN, 0.1% formic acid. The flow rate used was 250 nL/min and a chromatographic gradient ranging from 5 to 40% eluting buffer in 40 min was used. Blank runs were performed between the SRM measurements of biological samples to avoid sample carryover. Measurements were done in scheduled SRM mode, using a window of 300 seconds and a target cycle time of 1.5 seconds.

For the data analysis of the verification phase, transition groups corresponding to the targeted peptides were evaluated with Skyline v2.5 based on the co-elution of the transition traces associated with a targeted peptide, both in its light and heavy form; the correlation between the light SRM relative intensities and the heavy counterpart. All peptide peaks were visually inspected. Areas of all transitions were used as input for the linear mixed-effects model implemented in the MSstats Skyline plug-in (v3.3) to calculate protein fold-changes and adjusted p-values among the different sample groups.

For the development of predictors, MSstats was used to estimate the quantity of proteins present in all samples based on log2-transformed transition areas, which were then used as input variables to a logistic regression model between defined groups. The classification ability of each protein was evaluated within 2/3 of the dataset using the area under the curve (AUC) as performance indicator. The most discriminative protein was selected as the first classifier. Most discriminative proteins were repeatedly added while increasing AUC values (deltaAUC > 0.02). The procedure of classification evaluation was repeated 500 times using a different subset of patients in each iteration. Sample subsets were generated by randomly selecting patients from the original cohort without replacement and balanced for each sample subgroup. A final consensus model was comprised of the combination of proteins, which were selected most in 500 repeats. The final model was fitted to the full dataset and the predictive accuracy was quantified using the area under the ROC curve, sensitivity, specificity, and accuracy. The pROC package for the statistical program "R" was used to draw ROCs, to calculate AUCs and other performance values (i.e. sensitivity, specificity, and accuracy); these were obtained considering the "optimal cutoff" as the point were the sum of sensitivities and specificities reached the maximum value.

2. Results

2.1. Discovery phase

For the discovery phase of this project, uterine aspirates from 10 EEC (also abbreviated EC1) patients, 10 from NEEC (also abbreviated EC2, and that were particularly SEC) and 10 Control patients (N=30) were used to isolate ELVs.

Once ensured the purity of the vesicles isolated, the same amount of Super-SILAC mix (internal standard) was spiked in each of the 30 samples (EEC n=10, NEEC n=10 and CTRL n=10) in a 2:1 *Heavy*/*Light* ratio, right before the separation by 1D-SDS-PAGE. Each gel lane was sliced into 10 bands, and each band was subjected to trypsin digestion to extract peptides, resulting in 300 individual LC-MS/MS runs. Protein database searching of MS/MS data resulted in the overall identification of 2138 proteins, considering only those proteins identified with at least 1 unique peptide, and those peptides with a false discovery rate (FDR) lower than 1%. The MS/MS spectra were further processed with the software MaxQuant leading to the quantification of 920 proteins, that is an overall quantification rate of a 43% of the identified proteome.

A total of 152 proteins were differentially expressed with adjusted p-value < 0,25 and fold-change lower than -1,3 or higher than 1,3. From those, 147 proteins were potential diagnostic biomarkers (differential from the comparison CTRL vs. EC), and 28 proteins were potential prognostic biomarkers (differential from the comparison EEC vs. NEEC). In parallel to the quantitative analysis, a *presence*/*absence* study was conducted in order to take into consideration proteins that failed to be quantified because they lacked the heavy counterpart but were still relevant for being present in a certain group. For this, RAW files were reanalyzed with the PEAKS software and a Fisher Test was performed to select those proteins significantly present in a group (p-value < 0.001). A total of 30 candidates were included following this analysis, corresponding to 29 potential diagnostic biomarkers and 9 potential prognostic biomarkers.

Altogether, a final list of 54 candidates was generated combining (i) the relative quantification analysis, (ii) the presence/absence study, and (iii) biological criteria such as the exclusion of proteins whose family was overrepresented in the candidate list, or proteins down-regulated in cancer; always respecting the statistical restrictions stated above. From those 54 candidate biomarkers, 50 corresponded to diagnostic biomarkers and 15 to prognostic biomarkers, from which 37 had only diagnostic potential, 2 had only prognostic potential and 13 had both, diagnostic and prognostic potential.

2.2. Verification of protein biomarkers in UAs ELVs

Once obtained the list of 54 candidates described in the previous section, it was aimed to verify the potential of those candidates by using LC-SRM in a new and bigger cohort of patients. As in the discovery phase study, the ELVs fraction of UAs was the selected sample of analysis. In addition to evaluate the individual potential of each marker to diagnose EC and differentiate between histological subtypes, in here there was also sought to generate diagnostic and prognostic models by combining different candidates. Moreover, it was assessed the classification power of each

candidate in the whole fluid fraction of UAs in an independent cohort.

A total of 107 patients were recruited for the verification phase (cohort B), divided in three groups: EEC or EC1 (n=45), NEEC or EC2 (n=21) and CTRL (n=41). Specifically, NEEC corresponded to patients diagnosed with serous endometrial cancer (SEC). From those, ELVs from UAs were isolated and characterized as seen in previous sections (data not shown). The list of 54 protein candidate biomarkers generated in the discovery phase was verified by LC-SRM. To do so, two unique tryptic peptides were selected per protein, from which a total of 85 endogenous peptides were finally monitored by scheduled-SRM. However, there were only detected 69 of the 85 peptides corresponding to 51 proteins; three of the candidates (TNR6, CLH1 and PSB3) were not detected in any of the samples.

As a result of this SRM experiment, there was observed that 43 out of the 48 (89.6%) potential diagnostic biomarkers (i.e. significant differences in abundance were observed between CTRL and EC) were also significant in this verification phase (adj.pvalue < 0.01), thus confirming their potential as individual diagnostic biomarkers. A total of 29 out of the 45 quantified proteins presented high accuracy to individually discriminate between EC and CTRL cases (AUC values higher than 0.75, highlighted in bold in Table 6). The 5 most significant individual biomarkers were AGRIN (AUC= 0.90, CI95: 0.85-0.96), TACD2 (AUC= 0.87, CI95: 0.81-0.94), SORT (AUC=0.86, CI95: 0.79-0.93), MVP (AUC= 0.86, CI95: 0.78-0.93) and FAS (AUC= 0.85, CI95: 0.78-0.92). Table 2.1 below shows the list of selected biomarkers for EC diagnosis in ELVs.

Regarding the potential prognostic biomarkers (i.e. comparison of EC1 (EEC) vs. EC2 (NEEC)) only 5 proteins out of the 15 candidates were found to be differentially expressed in the verification phase (adj. p-value < 0.01). The verified biomarkers were CLD6, IF2B3, BCAM, PLD3 and MX1. A total of 4 out of the 45 quantified proteins presented high accuracy to individually discriminate between EC1 and EC2 cases with AUC values higher than 0.75: CLD6 (AUC= 0.88, CI95: 0.76-1.00), BCAM (AUC= 0.87, CI95: 0.76-0.97), IF2B3 (AUC=0.80, IC95: 0.68-0.93) and PLD3 (AUC= 0.79, IC95: 0.66-0.93). Table 2.2 below shows the list of selected biomarkers for EC prognosis in ELVs.

2.3. Verification of candidates by targeted proteomics in the whole fluid of UAs

In order to understand whether it would be feasible to transfer the use of these biomarkers to a sample that is easier to access and does not require the isolation of the extracellular vesicles, it was aimed to study the biomarkers identified in exosomes in the whole fluid fraction of UAs. For that, a total of 67 patients (cohort C) were selected and divided into three groups: EEC (n=22), NEEC (n=20) and CTRL (n=25).

For this part of the study, a total of 51 proteins were analyzed. Two unique tryptic peptides were selected per protein, from which a total of 75 endogenous peptides were finally monitored by scheduled-SRM. A total of 37 proteins were only detected and subsequently quantified for AUC estimation.

The results obtained in this verification study were evaluated in comparison to the one previously performed, in order to understand the feasibility to translate ELV's based biomarkers to the whole fluid of UAs. First, it was observed that the detectability of the ELVs biomarkers in the whole fluid of UAs was very limited compared to the detectability when ELVs were analyzed; out of the 51 biomarkers detected in ELVs, only 34 (66,7%) were detected in UAs.

In next Table 1.1 and Table 1.2, the list of significant biomarkers for EC diagnosis in whole fluid fraction of UAs, and for EC prognosis (comparison between EEC and NEEC) in said whole fluid fraction of UAs.

Table 1.1. Biomarkers of diagnosis in uterine aspirates

| Biomarkers of diagnosis in uterine aspirates | | | | | | | | | | |
|---|---|---|---|---|---|---|---|---|---|---|
| Protein | log2FC | FC | SE | Tvalue | DF | pvalue | adj.pvalue | AUC | IC95% | |
| sp\|P14780\|MMP9_HUMAN | -2.76 | 0.15 | 0.37 | -7.44 | 64 | 3.12E-10 | 1.15E-08 | .914 | .843 | .985 |
| sp\|O00468\|AGRIN_HUMAN | -1.43 | 0.37 | 0.21 | -6.83 | 64 | 3.63E-09 | 6.72E-08 | .864 | .772 | .956 |
| sp\|Q14764\|MVP_HUMAN | -1.41 | 0.38 | 0.21 | -6.63 | 64 | 8.23E-09 | 1.01E-07 | .884 | .805 | .962 |
| sp\|Q9BV40\|VAMP8_HUMAN | -1.00 | 0.50 | 0.18 | -5.57 | 64 | 5.51E-07 | 5.10E-06 | .855 | .766 | .944 |
| sp\|P41252\|SYIC_HUMAN | -1.55 | 0.34 | 0.29 | -5.40 | 64 | 1.05E-06 | 7.76E-06 | .854 | .762 | .947 |
| sp\|P61006\|RAB8A_HUMAN | -1.11 | 0.46 | 0.21 | -5.23 | 64 | 1.96E-06 | 1.21E-05 | .836 | .737 | .936 |
| sp\|P20591\|MX1_HUMAN | -1.48 | 0.36 | 0.30 | -4.98 | 64 | 5.09E-06 | 2.69E-05 | .859 | .770 | .948 |
| sp\|Q14974\|IMB1_HUMAN | -1.68 | 0.31 | 0.35 | -4.81 | 64 | 9.39E-06 | 4.34E-05 | .849 | .756 | .941 |
| sp\|O00299\|CLIC1_HUMAN | -1.38 | 0.38 | 0.29 | -4.78 | 64 | 1.07E-05 | 4.40E-05 | .832 | .736 | .929 |
| sp\|P62318\|SMD3_HUMAN | -1.11 | 0.46 | 0.24 | -4.72 | 64 | 1.31E-05 | 4.86E-05 | .792 | .683 | .902 |
| sp\|Q12905\|ILF2_HUMAN | -0.98 | 0.51 | 0.21 | -4.56 | 64 | 2.39E-05 | 8.03E-05 | .838 | .737 | .939 |
| sp\|P55072\|TERA_HUMAN | -0.77 | 0.59 | 0.17 | -4.41 | 64 | 4.09E-05 | 1.26E-04 | .789 | .682 | .895 |
| sp\|P62913\|RL11_HUMAN | -1.15 | 0.45 | 0.28 | -4.16 | 64 | 9.62E-05 | 2.74E-04 | .768 | .653 | .882 |
| sp\|P50895\|BCAM_HUMAN | -0.79 | 0.58 | 0.19 | -4.11 | 64 | 1.15E-04 | 3.04E-04 | .773 | .664 | .882 |
| sp\|P07355\|ANXA2_HUMAN | -1.15 | 0.45 | 0.28 | -4.04 | 64 | 1.48E-04 | 3.41E-04 | .759 | .642 | .876 |
| sp\|Q01650\|LAT1_HUMAN | -0.87 | 0.55 | 0.22 | -4.05 | 64 | 1.43E-04 | 3.41E-04 | .827 | .718 | .936 |
| sp\|Q9Y265\|RUVB1_HUMAN | -1.13 | 0.46 | 0.28 | -4.02 | 64 | 1.58E-04 | 3.45E-04 | .796 | .691 | .901 |
| sp\|Q9H299\|SH3L3_HUMAN | -0.83 | 0.56 | 0.21 | -3.95 | 64 | 2.00E-04 | 4.11E-04 | .774 | .663 | .885 |
| sp\|P40429\|RPL13A_HUMAN | -1.33 | 0.40 | 0.35 | -3.80 | 64 | 3.26E-04 | 6.35E-04 | .790 | .674 | .905 |
| sp\|P62249\|RS16_HUMAN | -1.24 | 0.42 | 0.33 | -3.69 | 64 | 4.59E-04 | 8.50E-04 | .693 | .562 | .825 |
| sp\|P08865\|RSSA_HUMAN | -1.01 | 0.50 | 0.28 | -3.59 | 64 | 6.40E-04 | 1.13E-03 | .782 | .670 | .894 |
| sp\|P30050\|RL12_HUMAN | -1.18 | 0.44 | 0.33 | -3.57 | 64 | 6.76E-04 | 1.14E-03 | .779 | .670 | .889 |
| sp\|P07237\|PDIA1_HUMAN | -0.68 | 0.62 | 0.19 | -3.55 | 64 | 7.25E-04 | 1.17E-03 | .795 | .689 | .901 |
| sp\|P47914\|RL29_HUMAN | -1.07 | 0.48 | 0.30 | -3.53 | 64 | 7.76E-04 | 1.20E-03 | .761 | .643 | .879 |
| sp\|P62937\|PPIA_HUMAN | -0.98 | 0.51 | 0.32 | -3.03 | 64 | 3.56E-03 | 5.26E-03 | .741 | .625 | .857 |
| sp\|O95994\|AGR2_HUMAN | -0.93 | 0.52 | 0.33 | -2.83 | 64 | 6.14E-03 | 8.74E-03 | .703 | .580 | .826 |
| sp\|P14618\|KPYM_HUMAN | -1.04 | 0.49 | 0.37 | -2.82 | 64 | 6.45E-03 | 8.84E-03 | .798 | .692 | .904 |

Table 1.2. Biomarkers of prognosis in uterine aspirates

| Biomarkers of prognosis in uterine aspirates | | | | | | | | | |
|---|---|---|---|---|---|---|---|---|---|
| Protein | log2FC | SE | Tvalue | DF | pvalue | adj.pvalue | AUC | IC95% | |
| sp\|P01833\|PIGR_HUMAN | -3.18 | 0.58 | -5.43 | 64.00 | 0 | 3.38E-05 | 8.80E-01 | .769 | .991 |
| sp\|O95994\|AGR2_HUMAN | -1.72 | 0.35 | -4.95 | 64.00 | 0 | 1.06E-04 | 8.30E-01 | .709 | .950 |
| sp\|P50895\|BCAM_HUMAN | 0.91 | 0.21 | 4.43 | 64.00 | 0 | 4.58E-04 | 1.66E-01 | .042 | .290 |
| sp\|O00592\|PODXL_HUMAN | -1.86 | 0.46 | -4.08 | 64.00 | 0 | 1.18E-03 | 8.43E-01 | .721 | .965 |
| sp\|P14780\|MMP9_HUMAN | -1.59 | 0.43 | -3.69 | 64.00 | 0 | 3.41E-03 | 8.02E-01 | .665 | .940 |
| sp\|P13987\|CD59_HUMAN | -0.94 | 0.28 | -3.32 | 64.00 | 0 | 9.17E-03 | 8.05E-01 | .660 | .949 |

Table 2.1. Biomarkers of diagnosis in Exosome-containing fraction (ELV) isolated from UA:

| Entry Name | UniProt Number | FC | log2FC | adj.pvalue | AUC | IC95% |
|---|---|---|---|---|---|---|
| AGRIN_HUMAN | O00468 | 4,46 | 2,16 | 1,25E-13 | **0,90** | 0,85 - 0,96 |
| MVP_HUMAN | Q14764 | 7,50 | 2,91 | 3,01E-10 | **0,86** | 0,78 - 0,93 |
| TACD2_HUMAN | P09758 | 4,76 | 2,25 | 3,01E-10 | **0,87** | 0,81 - 0,94 |
| FAS_HUMAN | P49327 | 4,90 | 2,29 | 4,30E-10 | **0,85** | 0,78 - 0,92 |
| SYIC_HUMAN | P41252 | 9,74 | 3,28 | 1,20E-09 | **0,84** | 0,77 - 0,91 |
| VAMP8_HUMAN | Q9BV40 | 4,17 | 2,06 | 1,57E-09 | **0,84** | 0,76 - 0,92 |
| SORT_HUMAN | Q99523 | 3,10 | 1,63 | 1,57E-09 | **0,86** | 0,79 - 0,93 |
| LAT1_HUMAN | Q01650 | 5,65 | 2,50 | 4,51E-09 | **0,84** | 0,76 - 0,91 |
| TERA_HUMAN | P55072 | 4,93 | 2,30 | 9,49E-09 | **0,82** | 0,74 - 0,90 |
| RUVB1_HUMAN | Q9Y265 | 4,76 | 2,25 | 9,49E-09 | **0,82** | 0,74 - 0,90 |
| RSSA_HUMAN | P08865 | 3,63 | 1,86 | 9,49E-09 | **0,82** | 0,74 - 0,90 |
| SMD3_HUMAN | P62318 | 4,38 | 2,13 | 1,09E-08 | **0,82** | 0,75 - 0,90 |
| ADA10_HUMAN | O14672 | 2,44 | 1,29 | 2,21E-08 | **0,80** | 0,71 - 0,89 |
| RPL13A_HUMAN | P40429 | 10,98 | 3,46 | 3,01E-08 | **0,80** | 0,72 - 0,88 |
| PGBM_HUMAN | P98160 | 3,95 | 1,98 | 3,05E-08 | n.q | n.q |
| RL11_HUMAN | P62913 | 8,32 | 3,06 | 3,53E-08 | **0,80** | 0,71 - 0,88 |
| IMB1_HUMAN | Q14974 | 3,27 | 1,71 | 3,53E-08 | **0,80** | 0,71 - 0,89 |
| AGR2_HUMAN | O95994 | 5,84 | 2,55 | 5,10E-08 | **0,79** | 0,69 - 0,88 |
| ITA3_HUMAN | P26006 | 2,26 | 1,18 | 5,10E-08 | **0,81** | 0,73 - 0,89 |
| RUXE_HUMAN | P62304 | 3,03 | 1,60 | 7,34E-08 | **0,78** | 0,70 - 0,87 |
| RL12_HUMAN | P30050 | 9,82 | 3,30 | 7,80E-08 | **0,79** | 0,71 - 0,88 |
| RS16_HUMAN | P62249 | 9,04 | 3,18 | 1,36E-07 | **0,79** | 0,71 - 0,87 |
| PSMD2_HUMAN | Q13200 | 5,09 | 2,35 | 3,02E-07 | **0,79** | 0,70 - 0,87 |
| MX1_HUMAN | P20591 | 3,94 | 1,98 | 3,77E-07 | **0,81** | 0,73 - 0,89 |
| VPS35_HUMAN | Q96QK1 | 2,00 | 1,00 | 7,86E-07 | **0,78** | 0,69 - 0,87 |
| ILF2_HUMAN | Q12905 | 3,20 | 1,68 | 8,51E-07 | **0,79** | 0,70 - 0,88 |
| PDIA1_HUMAN | P07237 | 2,65 | 1,41 | 9,50E-07 | **0,77** | 0,68 - 0,86 |
| MMP9_HUMAN | P14780 | 1,97 | 0,98 | 7,67E-06 | **0,79** | 0,70 - 0,88 |
| ANXA4_HUMAN | P09525 | 3,58 | 1,84 | 1,20E-05 | 0,72 | 0,61 - 0,82 |
| RAB8A_HUMAN | P61006 | 3,18 | 1,67 | 1,20E-05 | 0,73 | 0,62 - 0,84 |
| SH3L3_HUMAN | Q9H299 | 2,32 | 1,22 | 1,28E-05 | 0,72 | 0,62 - 0,83 |
| RL29_HUMAN | P47914 | 7,00 | 2,81 | 1,55E-05 | 0,74 | 0,64 - 0,83 |
| PLD3_HUMAN | Q8IV08 | 1,74 | 0,80 | 1,82E-05 | **0,75** | 0,65 - 0,85 |
| PPIA_HUMAN | P62937 | 3,86 | 1,95 | 2,86E-05 | 0,71 | 0,60 - 0,82 |
| ANXA2_HUMAN | P07355 | 3,49 | 1,80 | 3,66E-05 | 0,70 | 0,59 - 0,81 |
| S10AC_HUMAN | P80511 | 7,75 | 2,95 | 5,40E-05 | n.q | n.q |
| CD14_HUMAN | P08571 | 3,89 | 1,96 | 6,48E-05 | n.q | n.q |
| SSRA_HUMAN | P43307 | 3,05 | 1,61 | 6,67E-05 | 0,72 | 0,61 - 0,82 |
| LAMP2_HUMAN | P13473 | 2,16 | 1,11 | 7,78E-05 | 0,72 | 0,62 - 0,83 |
| PODXL_HUMAN | O00592 | 2,86 | 1,52 | 2,61E-04 | 0,71 | 0,61 - 0,81 |
| CLD6_HUMAN | P56747 | 1,83 | 0,87 | 2,61E-04 | 0,67 | 0,57 - 0,77 |
| IF2B3_HUMAN | O00425 | 1,97 | 0,98 | 4,27E-04 | **0,77** | 0,68 - 0,86 |
| CD59_HUMAN | P13987 | 2,80 | 1,49 | 6,17E-04 | 0,64 | 0,52 - 0,76 |
| MLEC_HUMAN | Q14165 | 2,05 | 1,04 | 2,55E-03 | 0,68 | 0,57 - 0,78 |
| H10_HUMAN | P07305 | 1,65 | 0,72 | 6,93E-03 | n.q | n.q |
| CD166_HUMAN | Q13740 | 1,59 | 0,67 | 9,42E-03 | 0,66 | 0,55 - 0,77 |
| CD81_HUMAN | P60033 | 2,01 | 1,00 | 1,15E-02 | 0,63 | 0,51 - 0,75 |
| AR6P1_HUMAN | Q15041 | 2,47 | 1,31 | 3,43E-02 | n.q | n.q |
| BCAM_HUMAN | P50895 | 1,24 | 0,31 | 3,78E-02 | 0,63 | 0,52 - 0,74 |
| VAC14_HUMAN | Q08AM6 | 1,61 | 0,68 | 5,29E-02 | n.q | n.q |
| ITB3_HUMAN | P05106 | 1,33 | 0,41 | 5,61E-02 | 0,61 | 0,50 - 0,73 |

Table 2.2. Biomarkers for EC prognosis in ELVs.

| Entry Name | UniProt Number | FC | log2FC | adj.pvalue | AUC | IC95% |
|---|---|---|---|---|---|---|
| CLD6_HUMAN | P56747 | 4,06 | 2,02 | 9,63E-13 | 0,88 | 0,76 - 1,00 |
| BCAM_HUMAN (*) | P50895 | 2,12 | 1,08 | 4,60E-08 | 0,87 | 0,76 - 0,97 |
| IF2B3_HUMAN | O00425 | 3,27 | 1,71 | 5,28E-06 | 0,80 | 0,68 - 0,93 |
| PLD3_HUMAN | Q8IV08 | 2,01 | 1,01 | 6,72E-05 | 0,79 | 0,66 - 0,93 |
| MX1_HUMAN | P20591 | 3,49 | 1,80 | 7,91E-04 | 0,74 | 0,61 - 0,87 |

**Example 2**. **Prognosis of EC by distinguishing EEC (EC1) from NEEC (EC2) cases in a cohort of 116 patients, in uterine aspirates.**

1. Samples, reagents and methods

1.1 Patients and sample description

A total of 116 women were recruited in the Vall Hebron University Hospital (Barcelona, Spain), the Hospital Universitari Arnau de Vilanova (Lleida, Spain) and the University Medical Center Freiburg (Freiburg, Germany) from 2012 to 2015. Informed consent forms, approved by the Ethical Committees of each Hospital, were signed by all patients. All women entered the EC diagnostic process due to EC suspicion, *i.e.,* presentation of aa abnormal uterine blooding (AUB) and/or a thickness of the endometrium higher than 4mm for postmenopausal women and 8mm for premenopausal women based on the results of a transvaginal ultrasonography. From the 116 women, 69 were diagnosed with EC, including 49 endometrioid EC (EEC) and 20 non-endometrioid serous ECs (SEC or NEEC). The remaining 47 women were non-EC women with normal endometrium or diagnosed with benign disorders (including endometrial hyperplasias). Clinico-pathological features of the patients are described in nexta Table of patients.

Table 3. Clinical characteristics of women enrolled in the study.

| * One case with undetermined grade | | | |
|---|---|---|---|
| | **EEC (n=49)** | **SEC (n=20)** | **Non-EC control (n=47)** |
| **Age (years)** | | | |
| **Median** | 67 | 73 | 53 |
| **Minimum** | 37 | 51 | 30 |
| **Maximum** | 87 | 93 | 80 |
| **Collection center** | | | |
| **VHIR** | 41 | 12 | 37 |
| **Lleida** | 5 | 8 | - |
| **Freiburg** | 3 | - | 10 |
| **Uterine condition** | | | |
| **Premenopausal** | 7 | 1 | 16 |
| | **EEC (n=49)** | **SEC (n=20)** | **Non-EC control (n=47)** |
| **Postmenopausal** | 42 | 19 | 31 |
| **Histologic grade** | | | |
| **Grade 1** | 5* | - | |
| **Grade 2** | 33 | - | |
| **Grade 3** | 10 | 20 | |
| **FIGO stage** | | | |
| **IA** | 25 | 5 | |
| **IB** | 13 | - | |
| **II** | 9 | 3 | |
| **IIIA** | - | 2 | |
| **IIIB** | - | 1 | |

(continued)

| | | |
|---|---|---|
| **FIGO stage** | | |
| **IIIC1** | - | - |
| **IIIC2** | 1 | 6 |
| **IVA** | 1 | 2 |
| **IVB** | - | 1 |
| **Myometrial invasion** | | |
| **<50%** | 30 | 12 |
| **>50%** | 19 | 8 |
| **Lymphovascular invasion** | | |
| **Yes** | 9 | 11 |
| **No** | 40 | 9 |

Uterine aspirates were collected by aspiration with a Cornier Pipelle (Eurogine Ref. 03040200) and transferred to 1.5 ml microtubes. Phosphate buffer saline was added in a 1:1 (v/v) ratio and centrifuged at 2,500 x g for 20 min in order to separate the fluid fraction from the cellular fraction. The fluids of uterine aspirates, ranging volumes from 100μl to 1ml, were kept at -80°C until used.

1.2. Sample preparation for Liquid chromatography-parallel reaction monitoring (LC-PRM) analysis

The sample preparation for the LC-PRM analysis was performed as described previously by Martinez-Garcia E, et al. "Development of a sequential workflow based on LC-PRM for the verification of endometrial cancer protein biomarkers in uterine aspirate samples", Oncotarget -2016, vol. no. 7(33), pp.:53102-53114 (*supra*). Briefly, fluid fractions from uterine aspirates were sonicated and 50 μl of each sample were depleted of albumin and immunoglobulin G proteins using the Albumin & IgG depletion spin trap kit (GE Healthcare). Total protein concentration was estimated by Bradford assay. Then, all 116 samples were divided in two equal aliquots of 12.5 μg to perform the subsequent steps and analyses with technical duplicates. Samples were denaturated, reduced and alkylated prior to a two-step sequential proteolysis using first Lys-C endoproteinase MS grade (Thermo Scientific) at a protease/total protein amount ratio of 1/150 (w/w) for 4 h at 37°C, and second, trypsin (Promega) at a protease/total protein amount ratio of 1/50 (w/w) at 37°C overnight. A mixture of the stable isotope-labeled synthetic peptides (Thermo Fisher, crude quality) was spiked in each sample (C terminal arginine $^{13}C_6$, $^{15}N_4$, $\Delta m$ = 10 Da, C terminal lysine $^{13}C_6$, $^{15}N_2$, $\Delta m$ = 8 Da or when it was not applicable with a heavy leucine $^{13}C_6$, $^{15}N_1$, $\Delta m$ = 7 Da or phenylalanine $^{13}C_9$, $^{15}N_1$, $\Delta m$ = 10 Da). Finally, samples were purified by solid phase extraction (Sep Pak tC18, 50 mg, Waters), vacuum dried and resuspended in 0.1% formic acid prior LC-PRM analysis. As in example 1, prognostic value data were also obtained with uterine fluid sample (UA) in which no depletion of albumin and immunoglobulin G was carried out.

1.3. LC-PRM setup

The separation of the peptides was performed on a Dionex Ultimate 3000 RSLC chromatography system operated in column switching mode. The equivalent of 250 ng of digested sample was injected and loaded onto a trap column (75 μm × 2 cm, C18 pepmap 100, 3μm) using a mobile phase of 0.05% trifluoroacetic acid and 1% acetonitrile in water at a flow rate of 5 μl/min. Peptides were further eluted onto the analytical column (75 μm × 15 cm, C18 pepmap 100, 2μm) at 300 nl/min by a linear gradient starting from 2 % solvent A to 35 % solvent B in 48 min. The solvent A was 0.1% formic acid in water and the solvent B was 0.1% formic acid in acetonitrile. The PRM analysis was performed on a Q Exactive plus mass spectrometer (Thermo Scientific). The MS cycle consisted of a full MS1 scan performed at a resolving power of 70,000 (at 200 m/z) followed by time scheduled targeted MS2 scans, with a normalized collision energy of 20, acquired at a resolving power of 35,000 (at 200 m/z). The quadrupole isolation window of precursor ions was set to 1 m/z unit for the MS2 events and the duration of the time scheduled windows for each pair of endogenous and isotopically labeled peptides was set to 2 min.

1.4. PRM data processing

The PRM data were processed as described in Martinez *et al* (supra). Briefly, the areas of extracted ion chromatograms (XIC) of the five most intense fragment ions of each precursor (*i.e.,* PRM transition) were extracted using the Skyline program (v3.1) (McCoss Lab, University of Washington, USA). The identity of the peptides was confirmed

using a spectral matching approach based on the cosine of the spectral contrast angle (cos θ) calculated between the peak areas of the five transitions of the reference (a PRM acquisition of the synthetic peptides mix without biological matrix) and the areas of the corresponding transitions for the endogenous and heavy peptides in the clinical samples. Peptide detection and identification were accepted if both the cos θ of the endogenous and the isotope labeled version of a peptide were higher than 0.98. MS measurements below the limits of quantification generated scores below 0.98 and in such cases the area values were replaced by an estimation of the background.

1.5. Statistical analysis

The light/heavy area ratio of each peptide was extracted from Skyline and the average between duplicates was calculated. The statistical analysis was performed in SPSS (v20.0) (IBM, Armonk, NY, USA) and Graph Pad Prism (v.6.0) (GraphPad Software, La Jolla, CA, USA). Comparison of the levels of the monitored peptides between groups of patients was performed using the nonparametric Mann-Whitney U test, since the data did not follow a normal distribution. P-values were adjusted for multiple comparisons using the Benjamini-Hochberg FDR method. Adjusted p-values lower than 0.05 were considered statistically significant. Receiver operating characteristic (ROC) analysis was used to assess the specificity and sensitivity of the biomarkers and the area under the ROC curve (AUC) was estimated for each individual protein.

1.6. Development of the classifiers

A logistic regression model was adjusted to the data in order to assess the power of the different combinations of proteins to classify samples in two clinical categories. ROC curves were generated for each of these regression models; the AUC, and the sensitivity and specificity at the "optimal" cutoff point for discrimination between groups were obtained. The optimal cut-off corresponded to the threshold that maximized the distance to the identity (diagonal) line. The optimality criterion was: max (sensitivities + specificities). AUCs 95% confidence intervals (CI) were computed with the Delong's method. The 95% Cls of the sensitivity and specificity values were computed with bootstrap resampling and the averaging methods described by Fawcett. All ROC analysis were performed using the R "pROC" package (Robin et al., "pROC: and open-source package for R and S+ to analyze and compare ROC curves", BMC Bioinformatics-2011, vol. no. 12:77). To assess the robustness of each protein panel, the "leave-one-out" cross-validation procedure was performed by applying to each sample in the dataset the logistic regression model adjusted to the remaining samples on the dataset, hence deriving a new ROC curve and afterwards performing the usual ROC analysis.

2. Results

EEC is the most common histology in EC and has a good prognosis when compared with non-endometrioid EC cases (NEEC). NEEC represents about 20% of all EC cases but accounts for more than 50% of recurrences and deaths from EC. Among NEEC, the serous EC (SEC) is the most common subtype. After investigating the abundance of the 51 proteins in the cohort of 49 EEC and 20 SEC cases, the levels of eleven proteins were significantly increased in uterine aspirate samples from EEC patients (adjusted p-value<0.05) , as depicted in Table 4. Among those, six proteins had fold change higher than 2 and presented the highest individual AUC values: PIGR with 0.85 (95% CI, 0.734-0.958), CAYP1 with 0.83 (95% CI, 0.725-0.942), CTNB1 with 0.78 (95% CI, 0.670-0.895), SG2A1 with 0.77 (95% CI, 0.661-0.880), VIME with 0.76 (95% CI, 0.645-0.881), and WFDC2 with 0.74 (95% CI, 0.624-0.855).

Table 4. Proteins differentially diagnosing EEC vs SEC (NEEC)

| Protein (ID Uniprot Accession Number) | Fold change; FC (EEC/SEC) | Adjusted p-value | AUC |
|---|---|---|---|
| PIGR (P01833) | 5.65 | 7.E-04 | 0.85 |
| CAYP1 (Q13938) | 4.48 | 7.E-04 | 0.83 |
| CTNB1 (P35222) | 2.70 | 5.E-03 | 0.78 |
| SG2A1 (075556) | 34.40 | 4.E-03 | 0.77 |
| VIME (P08670) | 2.40 | 8.E-03 | 0.76 |
| Protein (ID Uniprot Accession Number) | Fold change; FC (EEC/SEC) | Adjusted p-value | AUC |
| CADH1 (P12830) | 1.95 | 2.E-02 | 0.74 |
| WFDC2 (Q14508) | 4.55 | 2.E-02 | 0.74 |
| CD44 (P16070) | 1.76 | 5.E-02 | 0.71 |

(continued)

| Protein (ID Uniprot Accession Number) | Fold change; FC (EEC/SEC) | Adjusted p-value | AUC |
|---|---|---|---|
| LEG3 (P17931) | 1.61 | 5.E-02 | 0.71 |
| LEG1* (P09382) | 1.49 | 5.E-02 | 0.70 |
| CAPG* (P40121) | -1.67 | 2.E-01 | 0.67 |
| *p-value <0.05 | | | |

Inventors detected that robustness of classification was improved if besides determining the level of expression of one or more of the proteins in Table 4, the levels of other proteins were analyzed. In particular the levels of one or more of XPO2, PRDX1, CLIC1, PDIA1, KPYM, ENOA, GSTP1, GTR1, CH10, MIF, PEBP1, TPIS, NGAL, and LDHA.

Following the same procedure as described before, all possible combinations from two to twelve proteins and in particular of two and three proteins were evaluated among the diagnostic and predictive biomarkers to identify panels of proteins that will improve the outcome of individual biomarkers. A combination of three proteins, consisting of CTNB1, XPO2 and CAPG was the best-performing panel to discriminate between EEC and SEC in the fluid of uterine aspirate samples with an AUC of 0.99 (95% CI, 0.90-1). This panel achieved 95% (95% CI, 85%-100%) sensitivity and 95.9% (95% CI, 89.8%-100%) specificity (FIG. 1). After completion of the "leave-one-out" cross-validation the values were 95% sensitivity and 89.8% specificity.

As can be deduced from this FIG. 1, both markers CTNB1 and CAPG gave interesting values of sensitivity ans specificity for differentially diagnosing EEC from SEC, but surprisingly, the combination (panel) of CTNB1, CAPG and further XPO2 gave a highly sensitive and specific differentially diagnostic. Thus, the panel is to be understood as a diagnostic panel but also as a reliable prognostic panel, allowing classification of the EC subtype. Correct classification of the disease the earlier the better is translated to an increasing survival rate basically due to the correct medical regimen applied as soon as possible.

In this study it is disclosed a method of reliably distinguishing between EEC and SEC histologies by using a liquid biopsy obtained from the female genital tract (*i.e.,* the fluid of uterine aspirates). This supposes a real improvement, since current diagnostic procedure is based on the histological examination of the limited cellular content in this sample and it is associated to important drawbacks: an average of 22% of undiagnosed patients, and up to 50% of incorrect histotype and/or grade assignment of EC cases.

A clinical strength of this investigation is that women enrolled in the study covered the broad variability of women entering the EC diagnostic process. Regarding EC patients, the two most common histologies, EEC and SEC cases, were included. Non-EC patients covered all women with suspicion of EC mainly due to AUB or thickening of the endometrium. This included women suffering from benign pathological conditions (mainly polyps, myomas, and endometrial hyperplasia), and women with normal endometrium. Moreover, this study included both premenopausal women with functional endometrium and postmenopausal women mostly presenting an atrophic endometrium.

Regarding the role of the studied proteins to improve the risk group assignment of EC patients, current major risk stratification systems, such as the European Society for Medical Oncology (ESMO) classification, focus on the histology, grade, myometrial invasion and lymphovascular invasion of the tumors. As most information is not available preoperatively, histological subtype and grade become key factors for risk group assignment and for the determination of the extent of the surgical staging procedure. The combination of three proteins (CTNB1, XPO2 and CPG) derived from present invention allowed for the accurate discrimination between EEC and NEEC (SEC) histological EC types with a sensitivity of 95.0% and specificity of 95.9%.

These three proteins have been previously related to EC. Beta-catenin (CTNB1) has an important role in epithelial cell-cell adhesion, and in the transcription of essential genes responsible for cellular proliferation and differentiation in the Wnt signaling pathway. In concordance with the observations in current assay, CTNB1 has been described in EEC tissue specimens, but not in NEEC tumors. Macrophage-capping protein (CAPG) modulates cell motility by remodeling actin filaments. It is involved in cell migration and invasiveness in several type of cancers. Unlike CTNB1, higher levels of CAPG have been reported in more aggressive SEC tumors compared to EEC cases at tissue level, in agreement with the results in uterine aspirates reported herewith. Finally, exportin-2 (XPO2), also known as cellular apoptosis susceptibility protein, has a role in the mitotic spindle checkpoint. Depletion of XPO2 leads to cell-cycle arrest and, consequently, it has been associated with tumor proliferation; although it has also been related to tumor invasion and metastasis. Higher levels of XPO2 have been observed in many cancer types, including EC, and have been positively associated with a higher cancer grade and worse outcome of the patients. Although no significant differences in XPO2 levels were observed between EEC and SEC cases in this study, its inclusion in the panel formed by CTNB1 and CAPG significantly improved its performance.

Remarkably, this study covers an important clinical need in EC, since the uterine aspirate-based proteomic

approach here described paves the way for the identification of proteomic signatures that classify EC tumors into more clinically relevant risk groups to help on surgical treatment prediction. Therefore, a first step has been achieved with a signature to accurately classify the most common histologies. Altogether, the development of uterine aspirate-based biomarker signatures is expected to improve the management of EC patients and save great healthcare costs.

As indicated in the description, particular sets of proteins of Tables C and D are listed in the following:

Table C. Protein sets for prognosis of EC (EEC vs. NEEC) in uterine fluid samples

| PROTEIN 1 | PROTEIN 2 | PROTEIN 3 | AUC |
|---|---|---|---|
| CTNB1.1 * | XPO2 * | CAPG * | 0,991 |
| CTNB1.1 * | XPO2 * | PRDX1 * | 0,98 |
| CTNB1.1 ** | CLIC1 * | XPO2 ** | 0,98 |
| CTNB1.1 ** | XPO2 ** | PDIA1 * | 0,978 |
| CTNB1.1 ** | KPYM.1 * | XPO2 ** | 0,976 |
| CTNB1.1 ** | XPO2 ** | ENOA * | 0,973 |
| CTNB1 ** | GSTP1 ** | GTR1 ** | 0,973 |
| CTNB1.1 *** | XPO2 ** | GTR1 * | 0,972 |
| CTNB1 ** | XPO2 ** | CAPG * | 0,971 |
| CTNB1.1 *** | XPO2 ** | CH10 * | 0,971 |
| CTNB1 * | CAYP1 ** | PRDX1 * | 0,969 |
| CTNB1.1 ** | XPO2 ** | MIF * | 0,968 |
| CTNB1 * | CAYP1.1 ** | PRDX1 * | 0,967 |
| CTNB1.1 ** | XPO2 ** | PEBP1.1 * | 0,966 |
| CTNB1 *** | KPYM.1 ** | GTR1 ** | 0,965 |
| CTNB1.1 ** | KPYM * | XPO2 ** | 0,964 |
| CTNB1.1 ** | XPO2 ** | TPIS * | 0,963 |
| CTNB1.1 ** | XPO2 ** | GSTP1 | 0,963 |
| CTNB1 ** | XPO2 * | CH10 ** | 0,963 |
| CTNB1 ** | KPYM.1 * | XPO2 * | 0,962 |
| CTNB1.1 *** | KPYM.1 ** | GTR1 *** | 0,962 |
| CTNB1 ** | XPO2 ** | PRDX1 | 0,962 |
| CTNB1 ** | PRDX1 ** | GTR1 ** | 0,962 |
| CTNB1.1 *** | XPO2 ** | PIGR.1 | 0,961 |
| CTNB1.1 *** | XPO2 *** | NGAL | 0,961 |
| CTNB1.1 *** | XPO2 ** | VIME.1 | 0,96 |
| CTNB1.1 ** | XPO2 ** | LDHA | 0,96 |
| CTNB1.1 *** | XPO2 *** | HSPB1 | 0,959 |
| CTNB1.1 *** | XPO2 *** | SPIT1 | 0,959 |
| CTNB1.1 *** | XPO2 *** | K2C8 | 0,959 |
| CTNB1.1 *** | GSTP1 ** | GTR1 ** | 0,958 |
| CTNB1.1 ** | CAYP1.1 ** | PRDX1 ** | 0,958 |
| CTNB1.1 *** | MUC1 | XPO2 *** | 0,957 |
| CTNB1.1 ** | XPO2 ** | SG2A1 | 0,957 |
| CTNB1.1 *** | ANXA2 | XPO2 ** | 0,957 |

(continued)

| PROTEIN 1 | PROTEIN 2 | PROTEIN 3 | AUC |
|---|---|---|---|
| CTNB1.1 ** | XPO2 ** | WFDC2 | 0,957 |
| CTNB1.1 ** | XPO2 *** | LEG1.1 | 0,956 |
| CTNB1.1 *** | XPO2 *** | CADH1.1 | 0,956 |
| CTNB1.1 ** | CAYP1 ** | PRDX1 ** | 0,956 |
| CTNB1.1 *** | XPO2 ** | VIME | 0,956 |
| CTNB1.1 *** | XPO2 *** | CD44 | 0,956 |
| CTNB1.1 ** | XPO2 *** | LEG1 | 0,955 |
| CTNB1.1 *** | XPO2 *** | ANXA1 | 0,955 |
| CTNB1.1 ** | XPO2 ** | FABP5 | 0,955 |
| OSTP.1 | CTNB1.1 *** | XPO2 *** | 0,955 |
| CTNB1 | CTNB1.1 * | XPO2 *** | 0,955 |
| CTNB1.1 *** | XPO2 ** | WFDC2.1 | 0,955 |
| CTNB1.1 *** | XPO2 *** |  | 0,955 |
| CTNB1.1 ** | XPO2 *** | CASP3 | 0,954 |
| CTNB1.1 *** | XPO2 *** | CADH1 | 0,954 |
| CTNB1.1 *** | XPO2 ** | PIGR | 0,954 |
| CTNB1 ** | CLIC1 ** | GTR1 ** | 0,954 |
| CTNB1.1 *** | XPO2 *** | MMP9 | 0,953 |
| OSTP | CTNB1.1 *** | XPO2 ** | 0,953 |
| LEG3 | CTNB1.1 ** | XPO2 ** | 0,952 |
| LEG3.1 | CTNB1.1 ** | XPO2 ** | 0,952 |
| CTNB1.1 ** | XPO2 ** | CAYP1 | 0,952 |
| CTNB1.1 ** | XPO2 ** | CAYP1.1 | 0,952 |
| CTNB1 ** | XPO2 ** | GSTP1 * | 0,952 |
| CTNB1.1 *** | XPO2 *** | PERM | 0,951 |
| CTNB1 ** | XPO2 ** | PDIA1 * | 0,951 |
| CTNB1 *** | GTR1 ** | CAPG ** | 0,951 |
| CTNB1 *** | PEBP1.1 ** | GTR1 ** | 0,95 |
| SG2A1 | PIGR | PIGR.1 | 0,949 |
| CTNB1.1 *** | GTR1 ** | CAPG ** | 0,949 |
| CTNB1 *** | PDIA1 ** | GTR1 ** | 0,949 |
| CTNB1 ** | XPO2 ** | GTR1 * | 0,949 |
| CTNB1.1 *** | PEBP1.1 ** | GTR1 *** | 0,949 |
| CTNB1.1 *** | XPO2 *** | NAMPT | 0,948 |
| CTNB1 *** | GTR1 ** | ENOA ** | 0,947 |
| CTNB1 *** | CH10 * | GTR1 * | 0,947 |
| CTNB1 ** | XPO2 ** | PEBP1.1 * | 0,947 |
| CTNB1.1 *** | CLIC1 ** | GTR1 ** | 0,946 |
| CAYP1 ** | CASP3 * | PRDX1 * | 0,945 |

(continued)

| PROTEIN 1 | PROTEIN 2 | PROTEIN 3 | AUC |
|---|---|---|---|
| CAYP1.1 ** | CASP3 | PRDX1 * | 0,945 |
| CTNB1 ** | XPO2 ** | ENOA * | 0,945 |
| CTNB1 *** | XPO2 ** | VIME.1 | 0,944 |
| CTNB1 ** | XPO2 ** | SG2A1 | 0,944 |
| CTNB1 *** | XPO2 ** | VIME | 0,941 |
| CTNB1 ** | XPO2 ** | PIGR.1 | 0,941 |
| VIME.1 * | PIGR* | PIGR.1 * | 0,941 |
| OSTP ** | CADH1.1 ** | CH10 ** | 0,94 |
| CTNB1 *** | XPO2 ** | LDHA | 0,939 |
| CTNB1.1 *** | PRDX1 ** | GTR1 ** | 0,939 |
| CTNB1.1 *** | GTR1 ** | ENOA ** | 0,939 |
| CTNB1 * | PIGR | PIGR.1 * | 0,939 |
| CTNB1 ** | CLIC1 | XPO2 ** | 0,939 |
| CTNB1 ** | XPO2 ** | K2C8 | 0,939 |
| LEG3.1 ** | CTNB1 ** | CAPG ** | 0,939 |
| CTNB1 *** | CH10 * | CAPG * | 0,938 |
| LEG3 ** | CAYP1 ** | CAPG ** | 0,938 |
| CAYP1 ** | PRDX1 ** | CADH1.1 ** | 0,938 |
| CTNB1 ** | XPO2 ** | TPIS | 0,938 |
| LEG3.1 ** | CTNB1.1 ** | CAPG ** | 0,937 |
| SG2A1 * | TPIS * | CAPG ** | 0,937 |
| CTNB1 *** | XPO2 ** | PIGR | 0,937 |
| CTNB1 ** | GTR1 ** | PIGR.1 | 0,937 |
| LEG3.1 ** | CAYP1 ** | CAPG ** | 0,937 |
| CTNB1 ** | XPO2 ** | MIF | 0,937 |
| LEG3.1 ** | SG2A1 | CAPG ** | 0,936 |
| CTNB1 ** | SG2A1 | CH10 ** | 0,936 |
| CTNB1 ** | SG2A1 | CAPG ** | 0,936 |
| CTNB1 *** | XPO2 ** | PERM | 0,935 |
| CTNB1.1 * | SG2A1 | CAPG ** | 0,935 |
| OSTP | CTNB1 *** | XPO2 ** | 0,935 |
| OSTP * | PIGR | PIGR.1 * | 0,935 |
| CTNB1 ** | XPO2 ** | WFDC2 | 0,935 |
| CAYP1 ** | PRDX1 ** | CADH1 ** | 0,935 |
| CTNB1.1 * | PIGR | PIGR.1 * | 0,935 |
| LEG3 * | CTNB1 ** | CAPG ** | 0,935 |
| OSTP ** | CADH1 ** | CH10 ** | 0,934 |
| CTNB1 ** | XPO2 ** | CAYP1 | 0,934 |
| CTNB1 *** | VIME * | CAPG ** | 0,934 |

(continued)

| PROTEIN 1 | PROTEIN 2 | PROTEIN 3 | AUC |
|---|---|---|---|
| CTNB1 *** | XPO2 ** | NAMPT | 0,933 |
| LEG3 ** | SG2A1 | CAPG ** | 0,933 |
| CTNB1 ** | XPO2 ** | CAYP1.1 | 0,933 |
| CTNB1 *** | GTR1 ** | MIF * | 0,933 |
| LEG3.1 ** | CAYP1.1 ** | CAPG ** | 0,933 |
| CTNB1.1 *** | GTR1 ** | MIF ** | 0,932 |
| LEG3 ** | CAYP1.1 ** | CAPG ** | 0,932 |
| CTNB1 ** | XPO2 ** | CASP3 | 0,931 |
| CTNB1 *** | CASP3 ** | GTR1 ** | 0,931 |
| CTNB1.1 *** | PDIA1 ** | GTR1 ** | 0,931 |
| CAYP1 * | PIGR | PIGR.1 * | 0,93 |
| PIGR | PIGR.1 * | CAPG | 0,93 |
| CTNB1 *** | VIME.1 * | CAPG ** | 0,93 |
| CTNB1 *** | MUC1 | XPO2 ** | 0,93 |
| CTNB1 *** | XPO2 ** | | 0,93 |
| KPYM * | CAYP1 ** | PRDX1 *** | 0,929 |
| LEG3.1 | CTNB1 ** | XPO2 ** | 0,929 |
| CAYP1.1 * | PIGR | PIGR.1 * | 0,929 |
| LEG3 | CTNB1 ** | XPO2 ** | 0,929 |
| CTNB1 *** | ANXA2 | XPO2 ** | 0,929 |
| CTNB1 *** | XPO2 ** | MMP9 | 0,929 |
| CTNB1 ** | CAYP1.1 * | CAPG ** | 0,929 |
| CTNB1 *** | XPO2 ** | WFDC2.1 | 0,929 |
| CTNB1 ** | KPYM | XPO2 ** | 0,929 |
| CTNB1 *** | KPYM.1 * | CH10 ** | 0,929 |
| CASP3 * | SG2A1 * | CAPG ** | 0,928 |
| CTNB1 *** | XPO2 ** | CADH1 | 0,928 |
| CTNB1 ** | XPO2 ** | CADH1.1 | 0,928 |
| CAYP1 ** | PRDX1 ** | K2C8 * | 0,928 |
| CAYP1.1 ** | PRDX1 ** | CADH1 * | 0,928 |
| LEG3.1 * | CAYP1 ** | PRDX1 *** | 0,928 |
| CTNB1 ** | CH10 ** | WFDC2 | 0,928 |
| CAYP1 ** | PRDX1 ** | ENOA * | 0,928 |
| CTNB1 *** | XPO2 ** | CD44 | 0,928 |
| CTNB1 *** | XPO2 ** | FABP5 | 0,928 |
| CTNB1 ** | CAYP1 * | CAPG ** | 0,928 |
| CTNB1.1 *** | CH10 * | GTR1 * | 0,928 |
| LEG3 ** | CTNB1.1 * | CAPG ** | 0,927 |
| KPYM * | CAYP1.1 *** | PRDX1 *** | 0,927 |

(continued)

| PROTEIN 1 | PROTEIN 2 | PROTEIN 3 | AUC |
|---|---|---|---|
| OSTP.1 | CTNB1 *** | XPO2 ** | 0,927 |
| CTNB1 *** | VIME | CH10 ** | 0,927 |
| CTNB1 *** | XPO2 ** | ANXA1 | 0,927 |
| CAYP1.1 ** | PRDX1 ** | CADH1.1 ** | 0,927 |
| CTNB1 *** | VIME.1 | CH10 ** | 0,927 |
| CTNB1 ** | WFDC2 | CAPG ** | 0,927 |
| LEG1 * | PIGR | PIGR.1 | 0,927 |
| CLIC1 * | CAYP1 ** | PRDX1 ** | 0,926 |
| CTNB1 ** | XPO2 ** | LEG1 | 0,926 |
| CTNB1 *** | XPO2 ** | LEG1.1 | 0,926 |
| CTNB1 *** | FABP5 * | CH10 ** | 0,926 |
| CTNB1 *** | HSPB1 * | GTR1 ** | 0,926 |
| CAYP1.1 ** | PRDX1 ** | ENOA * | 0,926 |
| CTNB1 *** | CH10 ** | PIGR.1 | 0,924 |
| OSTP ** | CADH1.1 *** | CAPG ** | 0,924 |
| CTNB1 ** | SG2A1 * | GSTP1 * | 0,924 |
| CTNB1 *** | MUC1 * | CH10 *** | 0,924 |
| CTNB1 *** | XPO2 ** | NGAL | 0,924 |
| CTNB1 *** | XPO2 ** | HSPB1 | 0,924 |
| CTNB1 *** | CH10 ** | WFDC2.1 | 0,923 |
| VIME.1 * | CADH1 ** | CH10 ** | 0,923 |
| LEG3 | CAYP1 ** | PRDX1 *** | 0,923 |
| CTNB1.1 ** | ANXA1 * | CAPG ** | 0,923 |
| CTNB1 ** | WFDC2 | GTR1 ** | 0,923 |
| CTNB1 ** | PIGR.1 | CAPG ** | 0,923 |
| CAYP1 ** | VIME.1 | PRDX1 ** | 0,923 |
| CTNB1.1 *** | KPYM ** | GTR1 ** | 0,922 |
| KPYM * | SG2A1 ** | CAPG ** | 0,922 |
| LEG3.1 * | CAYP1.1 ** | PRDX1 *** | 0,922 |
| CAYP1 ** | PRDX1 ** | PIGR.1 | 0,922 |
| CTNB1 *** | XPO2 ** | SPIT1 | 0,922 |
| CTNB1 *** | ANXA1 * | CAPG ** | 0,922 |
| CTNB1 ** | KPYM * | GTR1 ** | 0,921 |
| CTNB1.1 ** | SG2A1 | CH10 * | 0,921 |
| LEG1.1 | PIGR | PIGR.1 | 0,921 |
| CTNB1.1 *** | CASP3 ** | GTR1 *** | 0,92 |
| CTNB1.1 ** | CAYP1 * | CAPG ** | 0,92 |
| OSTP * | CTNB1.1 *** | CAPG ** | 0,919 |
| LEG1 | SG2A1 * | CAPG ** | 0,919 |

(continued)

| PROTEIN 1 | PROTEIN 2 | PROTEIN 3 | AUC |
|---|---|---|---|
| VIME.1 * | SG2A1 * | CAPG | 0,919 |
| CAYP1.1 ** | VIME.1 | PRDX1 ** | 0,919 |
| CTNB1 *** | CH10 ** | MIF * | 0,919 |
| CTNB1.1 *** | HSPB1 ** | GTR1 ** | 0,919 |
| CAYP1.1 ** | PRDX1 ** | PIGR.1 | 0,919 |
| CTNB1 * | CADH1.1 | CH10 *** | 0,919 |
| CTNB1.1 ** | PIGR.1 | CAPG ** | 0,918 |
| VIME.1 * | CADH1.1 ** | CH10 ** | 0,918 |
| NGAL | PIGR * | PIGR.1 * | 0,918 |
| LEG3 | CAYP1.1 ** | PRDX1 *** | 0,918 |
| CTNB1 *** | GTR1 ** | TPIS * | 0,918 |
| CAYP1.1 ** | PRDX1 ** | K2C8 * | 0,918 |
| VIME * | PIGR | PIGR.1 | 0,918 |
| CTNB1.1 ** | GTR1 ** | PIGR.1 | 0,918 |
| CTNB1 *** | NGAL * | GTR1 ** | 0,918 |
| CAYP1 ** | CADH1.1 * | CAPG ** | 0,918 |
| CAYP1 * | SG2A1 | CAPG ** | 0,917 |
| CTNB1 *** | GSTP1 | CH10 ** | 0,917 |
| CTNB1.1 *** | CH10 | CAPG * | 0,917 |
| CLIC1 * | CAYP1.1 *** | PRDX1 ** | 0,917 |
| LEG3.1 *** | ENOA * | CAPG ** | 0,917 |
| CTNB1 ** | SG2A1 | GTR1 * | 0,917 |
| CTNB1.1 ** | CAYP1.1 * | CAPG ** | 0,917 |
| PERM | PIGR | PIGR.1 * | 0,917 |
| CLIC1 * | SG2A1 * | CAPG ** | 0,917 |
| GTR1 | PIGR | PIGR.1 * | 0,917 |
| CTNB1 ** | CAYP1 | CH10 ** | 0,917 |
| CTNB1 ** | CADH1 * | CH10 *** | 0,917 |
| MUC1 | CAYP1 ** | PRDX1 ** | 0,917 |
| CTNB1 ** | ANXA2 * | GTR1 ** | 0,917 |
| CTNB1 *** | CH10 ** | HSPB1 | 0,917 |
| OSTP | CTNB1 *** | CH10 ** | 0,917 |
| CTNB1.1 ** | VIME.1 * | CAPG ** | 0,916 |
| CAYP1.1 * | SG2A1 | CAPG ** | 0,916 |
| CTNB1 *** | CH10 ** | ENOA * | 0,916 |
| MMP9 | PIGR | PIGR.1 * | 0,916 |
| CTNB1 * | CTNB1.1 | CH10 *** | 0,916 |
| CTNB1 *** | CH10 ** | NGAL | 0,916 |
| CAYP1 ** | VIME | PRDX1 ** | 0,916 |

(continued)

| PROTEIN 1 | PROTEIN 2 | PROTEIN 3 | AUC |
|---|---|---|---|
| LEG3 *** | VIME | CAPG ** | 0,915 |
| VIME.1 * | CADH1 ** | CAPG ** | 0,915 |
| OSTP ** | CADH1 ** | CAPG ** | 0,915 |
| VIME.1 * | SG2A1 | PIGR.1 | 0,915 |
| CTNB1 ** | LEG1 | CAPG ** | 0,915 |
| CAYP1 ** | VIME * | CAPG ** | 0,915 |
| CAYP1 ** | PRDX1 ** | WFDC2.1 | 0,915 |
| CAYP1.1 ** | PRDX1 ** | MIF * | 0,915 |
| CTNB1.1 *** | GSTP1 | CAPG * | 0,915 |
| CAYP1.1 ** | PRDX1 ** | HSPB1 | 0,915 |
| CTNB1 ** | CAYP1.1 | CH10 ** | 0,915 |
| OSTP * | CTNB1.1 ** | PIGR.1 * | 0,915 |
| CTNB1.1 ** | WFDC2 | CAPG ** | 0,915 |
| CTNB1 ** | VIME * | GTR1 ** | 0,915 |
| CTNB1 *** | FABP5 * | GTR1 ** | 0,915 |
| CAYP1 ** | PRDX1 ** | HSPB1 | 0,914 |
| CTNB1 *** | PRDX1 | CH10 ** | 0,914 |
| CTNB1.1 *** | PDIA1 | CAPG ** | 0,914 |
| CTNB1.1 *** | NGAL * | GTR1 ** | 0,914 |
| CAYP1 ** | PRDX1 *** | TPIS * | 0,914 |
| VIME.1 *** | LDHA ** | CAPG ** | 0,914 |
| CAYP1 ** | PRDX1 *** | PDIA1 | 0,914 |
| CAYP1.1 ** | CADH1.1 * | CAPG ** | 0,914 |
| LEG3 | CTNB1 *** | CH10 ** | 0,914 |
| CTNB1 ** | PRDX1 * | SG2A1 * | 0,913 |
| SG2A1 * | CADH1.1 * | CAPG ** | 0,913 |
| LEG3 ** | CASP3 * | CAPG *** | 0,913 |
| OSTP.1 | CTNB1.1 *** | CAPG ** | 0,913 |
| SG2A1 * | PIGR.1 | CAPG | 0,913 |
| CTNB1 ** | CLIC1 * | SG2A1 | 0,913 |
| VIME.1 * | CADH1.1 ** | CAPG ** | 0,913 |
| CAYP1 ** | PRDX1 ** | SG2A1 | 0,913 |
| CAYP1 ** | K2C8 | CAPG ** | 0,913 |
| WFDC2.1 | PIGR | PIGR.1 * | 0,913 |
| OSTP.1 | CTNB1 *** | CH10 ** | 0,913 |
| CTNB1.1 ** | VIME * | CAPG ** | 0,913 |
| CAYP1.1 ** | PRDX1 *** | TPIS * | 0,913 |
| CTNB1.1 *** | ANXA2 * | GTR1 ** | 0,913 |
| CTNB1.1 *** | FABP5 | GTR1 ** | 0,913 |

(continued)

| PROTEIN 1 | PROTEIN 2 | PROTEIN 3 | AUC |
|---|---|---|---|
| CAYP1 ** | PRDX1 *** | LDHA | 0,913 |
| LEG3.1 ** | CASP3 * | CAPG *** | 0,913 |
| CAYP1 ** | PRDX1 ** | WFDC2 | 0,913 |
| LEG3.1 ** | VIME | CAPG ** | 0,912 |
| CTNB1 *** | CH10 ** | NAMPT | 0,912 |
| CAYP1.1 ** | CADH1 * | CAPG ** | 0,912 |
| OSTP.1 | PIGR | PIGR.1 * | 0,912 |
| CTNB1.1 ** | WFDC2 | GTR1 ** | 0,912 |
| CAYP1 ** | PRDX1 ** | MIF * | 0,912 |
| CAYP1 ** | CADH1 * | CAPG ** | 0,912 |
| CAYP1.1 ** | VIME | PRDX1 ** | 0,912 |
| HSPB1 | PIGR | PIGR.1 * | 0,912 |
| WFDC2 | PIGR | PIGR.1 * | 0,912 |
| MUC1 | CAYP1.1 ** | PRDX1 ** | 0,912 |
| CAYP1.1 ** | PRDX1 ** | PDIA1 | 0,912 |
| OSTP * | CTNB1 ** | PIGR.1 * | 0,912 |
| CTNB1 *** | CH10 ** | PIGR | 0,911 |
| CTNB1.1 ** | LEG1 | CAPG *** | 0,911 |
| SG2A1 ** | HSPB1 * | CAPG ** | 0,911 |
| SG2A1 * | PDIA1 | CAPG * | 0,911 |
| OSTP | CTNB1 *** | CAPG ** | 0,911 |
| CTNB1 *** | CH10 ** | TPIS | 0,911 |
| CTNB1 *** | GTR1 ** | PIGR | 0,911 |
| CAYP1.1 ** | PRDX1 ** | WFDC2.1 | 0,911 |
| CAYP1 ** | CD44 * | PRDX1 ** | 0,911 |
| WFDC2 * | TPIS ** | CAPG ** | 0,911 |
| PIGR | PIGR.1 * |  | 0,911 |
| LEG3 ** | VIME.1 | CAPG ** | 0,91 |
| SG2A1 ** | ANXA1 * | CAPG ** | 0,91 |
| CTNB1 *** | CH10 ** | LDHA | 0,91 |
| CTNB1.1 ** | SG2A1 * | GSTP1 * | 0,91 |
| LEG1 | CAYP1 ** | PRDX1 *** | 0,91 |
| OSTP | SG2A1 | PIGR.1 | 0,91 |
| CH10 | PIGR | PIGR.1 * | 0,91 |
| CTNB1 *** | CH10 ** | K2C8 | 0,91 |
| MUC1 | PIGR | PIGR.1 * | 0,91 |
| CTNB1 ** | WFDC2.1 | GTR1 ** | 0,91 |
| CAYP1.1 ** | K2C8 | CAPG ** | 0,91 |
| VIME.1 * | PIGR.1 * | CAPG | 0,91 |

(continued)

| PROTEIN 1 | PROTEIN 2 | PROTEIN 3 | AUC |
|---|---|---|---|
| LEG3.1 | PIGR | PIGR.1 * | 0,91 |
| VIME.1 * | CD44 * | SG2A1 | 0,91 |
| SG2A1 * | ENOA * | CAPG ** | 0,91 |
| CTNB1 *** | LEG1.1 | CH10 ** | 0,91 |
| CTNB1 *** | LEG1 | CH10 ** | 0,91 |
| CTNB1 *** | MMP9 | CH10 ** | 0,91 |
| CTNB1 *** | ANXA1 | CH10 ** | 0,91 |
| LEG3 ** | LDHA | CAPG ** | 0,909 |
| LEG3 ** | PIGR.1 | CAPG ** | 0,909 |
| LEG3.1 ** | LDHA | CAPG ** | 0,909 |
| CTNB1.1 ** | PRDX1 * | SG2A1 | 0,909 |
| VIME.1 * | SG2A1 | CADH1.1 | 0,909 |
| LEG3 | PIGR | PIGR.1 * | 0,909 |
| CASP3 * | SG2A1 ** | GSTP1 ** | 0,909 |
| CAYP1.1 ** | PRDX1 *** | LDHA * | 0,909 |
| OSTP | VIME.1 * | PIGR.1 * | 0,909 |
| CAYP1.1 ** | CD44 * | PRDX1 ** | 0,909 |
| CTNB1 ** | SG2A1 | ENOA * | 0,909 |
| LEG3.1 | CTNB1 *** | CH10 ** | 0,909 |
| CTNB1 *** | CH10 ** |  | 0,909 |
| CTNB1 ** | VIME.1 * | PRDX1 * | 0,908 |
| CTNB1.1 *** | LEG1.1 | CAPG ** | 0,908 |
| OSTP.1 ** | CADH1.1 *** | CAPG ** | 0,908 |
| CTNB1 *** | CD44 | CH10 *** | 0,908 |
| KPYM.1 | CAYP1 *** | PRDX1 ** | 0,908 |
| LEG3.1 ** | KPYM | CAPG *** | 0,908 |
| CTNB1.1 ** | VIME * | GTR1 ** | 0,908 |
| CTNB1.1 ** | CH10 * | PIGR.1 | 0,908 |
| XPO2 | PIGR | PIGR.1 * | 0,908 |
| SG2A1 ** | MIF * | CAPG ** | 0,908 |
| PIGR | PIGR.1 * | SPIT1 | 0,908 |
| CTNB1 *** | LEG1.1 | CAPG ** | 0,908 |
| CTNB1.1 ** | CLIC1 | CAPG * | 0,908 |
| ANXA2 | CAYP1 ** | PRDX1 ** | 0,908 |
| CAYP1.1 ** | PRDX1 ** | SG2A1 | 0,908 |
| CAYP1.1 ** | PRDX1 ** | WFDC2 | 0,908 |
| FABP5 | PIGR | PIGR.1 * | 0,908 |
| CTNB1 ** | VIME.1 | GTR1 * | 0,908 |
| TPIS | PIGR | PIGR.1 * | 0,908 |

(continued)

| PROTEIN 1 | PROTEIN 2 | PROTEIN 3 | AUC |
|---|---|---|---|
| LEG3.1 ** | WFDC2 | CAPG ** | 0,908 |
| CAYP1 ** | PRDX1 *** | PERM | 0,908 |
| SG2A1 * | K2C8 | CAPG * | 0,908 |
| CADH1.1 *** | CH10 * | CAPG | 0,908 |
| CTNB1 *** | ANXA2 | CH10 ** | 0,908 |
| CTNB1 *** | CH10 ** | PERM | 0,908 |
| CTNB1 *** | CH10 ** | SPIT1 | 0,908 |
| LEG3.1 ** | VIME.1 | CAPG ** | 0,907 |
| LEG1 | CAYP1.1 ** | PRDX1 ** | 0,907 |
| LEG3.1 ** | PIGR.1 | CAPG ** | 0,907 |
| CTNB1 *** | CLIC1 | CH10 * | 0,907 |
| CTNB1.1 ** | KPYM | CAPG * | 0,907 |
| VIME.1 * | SG2A1 | CADH1 | 0,907 |
| SG2A1 * | PEBP1.1 | CAPG ** | 0,907 |
| OSTP.1 | VIME.1 * | PIGR.1 * | 0,907 |
| CTNB1 *** | MMP9 | GTR1 ** | 0,907 |
| CTNB1.1 ** | SG2A1 | GTR1 * | 0,907 |
| KPYM.1 | PIGR | PIGR.1 * | 0,907 |
| CTNB1.1 ** | WFDC2.1 | CAPG ** | 0,907 |
| LEG1.1 | SG2A1 * | CAPG * | 0,907 |
| CTNB1 *** | GTR1 ** | NAMPT | 0,907 |
| CTNB1.1 *** | WFDC2.1 | GTR1 ** | 0,907 |
| LEG3 ** | WFDC2 | CAPG ** | 0,907 |
| SG2A1 | CADH1.1 * | CH10 ** | 0,906 |
| LEG3 *** | ENOA | CAPG ** | 0,906 |
| CTNB1.1 *** | GTR1 ** | TPIS * | 0,906 |
| CAYP1 ** | PRDX1 | CAPG | 0,906 |
| CTNB1.1 ** | LDHA | CAPG ** | 0,906 |
| MMP9 | SG2A1 * | PIGR.1 | 0,906 |
| VIME.1 * | SG2A1 * | CH10 | 0,906 |
| PRDX1 | SG2A1 * | CAPG * | 0,906 |
| CAYP1.1 ** | PRDX1 *** | PERM | 0,906 |
| CAYP1 ** | PRDX1 ** | SPIT1 | 0,906 |
| CTNB1 ** | PIGR.1 | ENOA * | 0,906 |
| KPYM.1 | SG2A1 ** | CAPG * | 0,905 |
| CASP3 ** | PIGR.1 * | CAPG ** | 0,905 |
| CTNB1 *** | KPYM | CH10 ** | 0,905 |
| CTNB1 ** | VIME.1 * | GSTP1 * | 0,905 |
| CTNB1.1 * | CADH1 * | CH10 *** | 0,905 |

(continued)

| PROTEIN 1 | PROTEIN 2 | PROTEIN 3 | AUC |
|---|---|---|---|
| VIME * | CADH1.1 ** | CAPG ** | 0,905 |
| LEG3 ** | KPYM | CAPG *** | 0,905 |
| CTNB1.1 ** | ANXA2 | CAPG ** | 0,905 |
| KPYM.1 | CAYP1.1 *** | PRDX1 *** | 0,905 |
| PIGR | PIGR.1 * | NAMPT | 0,905 |
| CTNB1 ** | SG2A1 * | FABP5 * | 0,905 |
| CLIC1 | PIGR | PIGR.1 * | 0,905 |
| CASP3 | PIGR | PIGR.1 * | 0,905 |
| CTNB1.1 ** | K2C8 | CAPG ** | 0,905 |
| ANXA1 | PIGR | PIGR.1 * | 0,905 |
| CAYP1 ** | PRDX1 ** | PIGR | 0,905 |
| ANXA1 * | CADH1.1 ** | CAPG *** | 0,905 |
| CTNB1 ** | TPIS | CAPG ** | 0,905 |
| CTNB1.1 *** | FABP5 | CAPG ** | 0,905 |
| CAYP1 ** | PRDX1 ** | GSTP1 | 0,905 |
| LEG3.1 ** | CADH1 * | CAPG ** | 0,904 |
| CAYP1 ** | VIME.1 | CAPG ** | 0,904 |
| CTNB1 *** | VIME.1 | ENOA * | 0,904 |
| CTNB1 *** | CASP3 | CH10 ** | 0,904 |
| CTNB1 *** | PIGR | CAPG ** | 0,904 |
| SG2A1 | CADH1 * | CH10 ** | 0,904 |
| LEG3 ** | TPIS | CAPG *** | 0,904 |
| LEG3.1 ** | CADH1.1 * | CAPG *** | 0,904 |
| CTNB1.1 ** | MUC1 | CAPG ** | 0,904 |
| CTNB1.1 ** | CASP3 | CAPG ** | 0,904 |
| CTNB1.1 ** | TPIS | CAPG ** | 0,904 |
| CTNB1.1 ** | PERM | CAPG ** | 0,904 |
| CTNB1 *** | GSTP1 | CAPG * | 0,904 |
| CTNB1.1 *** | NAMPT | CAPG * | 0,904 |
| CAYP1 ** | PRDX1 ** | PEBP1.1 | 0,904 |
| CAYP1.1 ** | PRDX1 ** | PEBP1.1 | 0,904 |
| PRDX1 | PIGR | PIGR.1 * | 0,904 |
| SG2A1 * | CH10 | PIGR.1 | 0,904 |
| SG2A1 * | WFDC2.1 | PIGR.1 | 0,904 |
| CTNB1.1 *** | PRDX1 | CAPG * | 0,904 |
| CTNB1.1 ** | SG2A1 | ENOA * | 0,904 |
| CAYP1 ** | PRDX1 ** | FABP5 | 0,904 |
| CAYP1.1 ** | VIME * | CAPG ** | 0,904 |
| PIGR | PIGR.1 * | K2C8 | 0,904 |

(continued)

| PROTEIN 1 | PROTEIN 2 | PROTEIN 3 | AUC |
|---|---|---|---|
| CAYP1 | CAYP1.1 | PRDX1 ** | 0,904 |
| CD44 | PIGR | PIGR.1 * | 0,904 |
| CTNB1 *** | PDIA1 | CAPG * | 0,904 |
| VIME.1 * | SG2A1 * | GTR1 | 0,904 |
| CTNB1 ** | WFDC2.1 | CAPG ** | 0,904 |
| CTNB1.1 ** | CH10 * | WFDC2 | 0,904 |
| CTNB1 *** | CH10 ** | PEBP1.1 | 0,904 |
| LEG3.1 ** | TPIS | CAPG *** | 0,903 |
| CTNB1.1 ** | PIGR | CAPG ** | 0,903 |
| CTNB1.1** | SG2A1 * | FABP5 * | 0,903 |
| LEG1 | CADH1 *** | CH10 ** | 0,903 |
| LEG1.1 | CAYP1 ** | PRDX1 ** | 0,903 |
| CAYP1 | CADH1.1 ** | CH10 ** | 0,903 |
| OSTP.1 | CTNB1.1 ** | PIGR.1 * | 0,903 |
| CTNB1.1 ** | VIME.1 | CH10 * | 0,903 |
| PDIA1 | PIGR | PIGR.1 * | 0,903 |
| ANXA2 | SG2A1 * | PIGR.1 | 0,903 |
| CAYP1 * | CASP3 | GSTP1 * | 0,903 |
| CAYP1 * | CADH1 ** | CH10 ** | 0,903 |
| OSTP.1 | CAYP1 ** | PRDX1 ** | 0,903 |
| CTNB1.1 *** | KPYM.1 | CAPG * | 0,903 |
| CTNB1.1 * | CADH1.1 | CAPG ** | 0,903 |
| CTNB1 ** | PEBP1.1 | CAPG ** | 0,903 |
| CTNB1.1 *** | MMP9 | CAPG ** | 0,903 |
| ANXA1 * | CADH1 ** | CAPG ** | 0,903 |
| SG2A1 * | PIGR.1 | | 0,903 |
| CTNB1.1 ** | CLIC1 * | SG2A1 | 0,902 |
| LEG3 ** | CADH1.1 | CAPG *** | 0,902 |
| CTNB1 ** | SG2A1 | PDIA1 | 0,902 |
| CTNB1.1 ** | MIF | CAPG * | 0,902 |
| ANXA2 | CAYP1.1 ** | PRDX1 ** | 0,902 |
| CAYP1 ** | PRDX1 ** | CH10 | 0,902 |
| CAYP1.1 | CADH1.1 ** | CH10 ** | 0,902 |
| CADH1 *** | CH10 * | CAPG | 0,902 |
| CTNB1 *** | ANXA2 | CAPG ** | 0,902 |
| SG2A1 * | HSPB1 | PIGR.1 | 0,902 |
| CTNB1 ** | KPYM.1 | CAPG * | 0,902 |
| CTNB1 ** | GSTP1 * | PIGR.1 | 0,902 |
| SG2A1 * | GSTP1 | PIGR.1 | 0,902 |

(continued)

| PROTEIN 1 | PROTEIN 2 | PROTEIN 3 | AUC |
|---|---|---|---|
| SG2A1 * | PIGR.1 | NAMPT | 0,902 |
| OSTP.1 ** | CADH1.1 *** | CH10 ** | 0,902 |
| CTNB1.1 ** | CD44 * | GTR1 ** | 0,902 |
| CTNB1.1 ** | VIME | CH10 * | 0,902 |
| CTNB1.1 * | CADH1 | CAPG ** | 0,902 |
| CTNB1 *** | VIME * | ENOA ** | 0,902 |
| CTNB1 | CTNB1.1 | CAPG ** | 0,902 |
| SG2A1 * | PIGR | CAPG * | 0,902 |
| VIME * | CADH1 ** | CH10 ** | 0,902 |
| CTNB1.1 *** | CAPG ** | | 0,902 |
| CAYP1 *** | PRDX1 ** | | 0,902 |
| LEG1 * | PIGR.1 * | CAPG * | 0,901 |
| CAYP1.1 * | CASP3 | CAPG ** | 0,901 |
| SG2A1 ** | PERM | CAPG * | 0,901 |
| OSTP.1 | CTNB1 ** | PIGR.1 * | 0,901 |
| CTNB1.1 * | CADH1.1 * | CH10 *** | 0,901 |
| LEG1.1 | CAYP1.1 ** | PRDX1 ** | 0,901 |
| CAYP1 * | CASP3 | CAPG ** | 0,901 |
| CASP3 ** | VIME ** | CAPG ** | 0,901 |
| CTNB1 *** | PDIA1 | CH10 ** | 0,901 |
| CTNB1.1 *** | GTR1 ** | NAMPT * | 0,901 |
| CTNB1.1 ** | ENOA | CAPG | 0,901 |
| SG2A1 * | CADH1 * | CAPG ** | 0,901 |
| SG2A1 * | NGAL | PIGR.1 | 0,901 |
| MIF | PIGR | PIGR.1 * | 0,901 |
| PIGR | PIGR.1 * | ENOA | 0,901 |
| CTNB1 ** | CASP3 | CAPG ** | 0,901 |
| CTNB1.1 *** | NGAL | CAPG ** | 0,901 |
| CAYP1 ** | PRDX1 ** | GTR1 | 0,901 |
| CAYP1.1 ** | PRDX1 ** | PIGR | 0,901 |
| OSTP | CAYP1 ** | PRDX1 ** | 0,901 |
| CAYP1 ** | PRDX1 ** | ANXA1 | 0,901 |
| CTNB1 ** | CAYP1 | GSTP1 * | 0,901 |
| CTNB1 * | CADH1.1 | CAPG ** | 0,901 |
| SG2A1 * | FABP5 | PIGR.1 | 0,901 |
| CAYP1 ** | ENOA | CAPG ** | 0,901 |
| CTNB1 ** | FABP5 | CAPG ** | 0,901 |
| CAYP1.1 ** | PRDX1 ** | GSTP1 | 0,901 |
| LEG1 | CADH1.1 ** | CH10 ** | 0,9 |

(continued)

| PROTEIN 1 | PROTEIN 2 | PROTEIN 3 | AUC |
|---|---|---|---|
| CTNB1 ** | TPIS * | PIGR.1 * | 0,9 |
| CADH1 *** | CH10 ** | PERM | 0,9 |
| XPO2 | CAYP1 ** | PRDX1 ** | 0,9 |
| CAYP1 * | PIGR.1 | CAPG ** | 0,9 |
| CTNB1 ** | CAYP1.1 | GSTP1 * | 0,9 |
| CTNB1 ** | VIME * | GSTP1 * | 0,9 |
| CTNB1.1 ** | SPIT1 | CAPG ** | 0,9 |
| ANXA2 | PIGR | PIGR.1 * | 0,9 |
| XPO2 | CAYP1.1 ** | PRDX1 ** | 0,9 |
| CAYP1.1 * | LDHA | CAPG ** | 0,9 |
| PEBP1.1 | PIGR | PIGR.1 * | 0,9 |
| CTNB1 ** | SG2A1 | MIF * | 0,9 |
| CTNB1 *** | NGAL | CAPG ** | 0,9 |
| CASP3 ** | WFDC2 * | CAPG ** | 0,9 |
| OSTP * | CTNB1.1 *** | GSTP1 ** | 0,9 |
| CTNB1.1 ** | CAYP1 | CH10 ** | 0,9 |
| CTNB1.1 ** | CAYP1.1 | CH10 ** | 0,9 |
| CADH1 | PIGR | PIGR.1 * | 0,9 |
| CADH1.1 | PIGR | PIGR.1 * | 0,9 |

**Table D.** Protein sets for diagnosis of EC in uterine fluid samples

| PROTEIN1 | PROTEIN2 | PROTEIN3 | AUC |
|---|---|---|---|
| KPYM *** | MMP9 *** | NAMPT * | 0,969 |
| KPYM *** | MUC1 | MMP9 ** | 0,969 |
| KPYM ** | MMP9 ** | LDHA | 0,968 |
| CLIC1 | KPYM ** | MMP9 ** | 0,967 |
| KPYM ** | CASP3 | MMP9 ** | 0,967 |
| KPYM *** | MMP9 ** | SG2A1 | 0,967 |
| KPYM *** | MMP9 * | PERM | 0,967 |
| KPYM ** | MMP9 ** | GSTP1 | 0,966 |
| KPYM *** | MMP9 ** | NGAL | 0,966 |
| KPYM ** | MMP9 ** | TPIS | 0,966 |
| CTNB1 | KPYM ** | MMP9 ** | 0,965 |
| KPYM *** | MMP9 ** | K2C8 | 0,965 |
| KPYM ** | MMP9 ** | CADH1 | 0,965 |
| KPYM ** | MMP9 ** | CH10 | 0,965 |
| KPYM *** | CAYP1 | MMP9 ** | 0,965 |
| KPYM ** | MMP9 ** | HSPB1 | 0,965 |
| KPYM * | MMP9 ** | PRDX1 | 0,965 |

(continued)

| PROTEIN1 | PROTEIN2 | PROTEIN3 | AUC |
|---|---|---|---|
| OSTP | KPYM *** | MMP9 ** | 0,965 |
| KPYM *** | MMP9 ** | | 0,9650 |
| KPYM * | KPYM.1 | MMP9 ** | 0,964 |
| KPYM ** | MMP9 ** | SPIT1 | 0,964 |
| KPYM ** | MMP9 ** | FABP5 | 0,964 |
| KPYM * | MMP9 ** | ENOA | 0,964 |
| OSTP.1 | KPYM *** | MMP9 ** | 0,964 |
| KPYM ** | ANXA2 | MMP9 ** | 0,964 |
| KPYM ** | XPO2 | MMP9 ** | 0,964 |
| KPYM ** | MMP9 ** | ANXA1 | 0,964 |
| KPYM *** | MMP9 ** | GTR1 | 0,964 |
| KPYM ** | MMP9 ** | CAPG | 0,964 |
| KPYM ** | MMP9 ** | MIF | 0,963 |
| KPYM ** | MMP9 ** | CD44 | 0,963 |
| KPYM *** | MMP9 ** | PIGR | 0,963 |
| KPYM.1 *** | MMP9 ** | PERM * | 0,963 |
| KPYM ** | MMP9 ** | PDIA1 | 0,962 |
| KPYM *** | TPIS | PERM * | 0,962 |
| KPYM.1 | MMP9 *** | PRDX1 | 0,961 |
| KPYM.1 * | MMP9 ** | CADH1 | 0,96 |
| KPYM.1 ** | MMP9 *** | ANXA1 | 0,96 |
| KPYM ** | CASP3 | PERM * | 0,959 |
| MMP9 ** | PRDX1 | ENOA | 0,959 |
| CAYP1 | MMP9 *** | PRDX1 *** | 0,959 |
| MMP9 ** | FABP5 | ENOA ** | 0,959 |
| KPYM *** | GSTP1 * | PERM * | 0,959 |
| KPYM.1 ** | MMP9 *** | CH10 | 0,959 |
| KPYM.1 ** | ANXA2 | MMP9 *** | 0,959 |
| MMP9 ** | SG2A1 | ENOA *** | 0,959 |
| KPYM.1 * | MMP9 *** | CAPG | 0,959 |
| MMP9 ** | PRDX1 * | CADH1 | 0,959 |
| KPYM.1 * | MMP9 *** | FABP5 | 0,959 |
| MMP9 ** | CADH1 | ENOA * | 0,958 |
| MMP9 ** | TPIS | ENOA * | 0,958 |
| KPYM.1 * | MMP9 *** | PDIA1 | 0,958 |
| KPYM.1 * | MMP9 *** | HSPB1 | 0,958 |
| MMP9 ** | GTR1 | ENOA *** | 0,958 |
| KPYM.1 *** | MUC1 | MMP9 *** | 0,958 |
| KPYM.1 ** | MMP9 ** | SPIT1 | 0,958 |

(continued)

| PROTEIN1 | PROTEIN2 | PROTEIN3 | AUC |
|---|---|---|---|
| MMP9 ** | PRDX1 * | LDHA | 0,958 |
| KPYM.1 ** | CAYP1 | MMP9 *** | 0,958 |
| KPYM ** | PERM * | MIF | 0,958 |
| MMP9 * | PERM | ENOA *** | 0,957 |
| KPYM.1 ** | MMP9 *** | GTR1 | 0,957 |
| MMP9 ** | ENOA * | CAPG | 0,957 |
| MMP9 ** | ANXA1 | ENOA ** | 0,957 |
| MMP9 *** | PRDX1 *** | K2C8 | 0,957 |
| ANXA2 | MMP9 ** | ENOA ** | 0,957 |
| MMP9 ** | CD44 | PRDX1 *** | 0,957 |
| MMP9 ** | ENOA ** | NAMPT | 0,957 |
| MMP9 ** | PRDX1 ** | SPIT1 | 0,957 |
| OSTP | MMP9 ** | ENOA *** | 0,957 |
| MMP9 ** | GSTP1 | ENOA * | 0,957 |
| XPO2 | MMP9 ** | ENOA ** | 0,957 |
| MMP9 ** | HSPB1 | ENOA ** | 0,957 |
| MMP9 ** | K2C8 | ENOA *** | 0,957 |
| KPYM.1 ** | MMP9 *** | SG2A1 | 0,957 |
| KPYM.1 ** | MMP9 *** | NGAL | 0,957 |
| KPYM.1 ** | MMP9 *** | NAMPT | 0,957 |
| OSTP | KPYM.1 ** | MMP9 *** | 0,957 |
| OSTP.1 | KPYM.1 ** | MMP9 *** | 0,957 |
| CLIC1 | KPYM.1 * | MMP9 *** | 0,957 |
| KPYM.1 ** | MMP9 *** | PIGR | 0,957 |
| KPYM.1 ** | MMP9 *** | K2C8 | 0,957 |
| KPYM.1 | MMP9 ** | ENOA | 0,957 |
| MUC1 | MMP9 ** | ENOA *** | 0,957 |
| KPYM.1 | MMP9 *** | MIF | 0,957 |
| MMP9 ** | ENOA *** | | 0,957 |
| KPYM.1 ** | MMP9 *** | | 0,957 |
| KPYM *** | SG2A1 | PERM * | 0,956 |
| CASP3 | MMP9 *** | PRDX1 * | 0,956 |
| MMP9 ** | PRDX1 ** | FABP5 | 0,956 |
| CLIC1 | MMP9 *** | PRDX1 ** | 0,956 |
| MMP9 *** | PRDX1 * | CAPG | 0,956 |
| KPYM.1 * | CASP3 | MMP9 *** | 0,956 |
| MMP9 *** | CADH1 | MIF * | 0,956 |
| MMP9 ** | CADH1 * | CAPG * | 0,956 |
| MMP9 ** | CH10 | ENOA ** | 0,956 |

(continued)

| PROTEIN1 | PROTEIN2 | PROTEIN3 | AUC |
|---|---|---|---|
| MMP9 ** | PIGR | ENOA *** | 0,956 |
| KPYM.1 * | XPO2 | MMP9 *** | 0,956 |
| CAYP1 | MMP9 ** | ENOA ** | 0,956 |
| MMP9 ** | CD44 | ENOA ** | 0,956 |
| CTNB1 | MMP9 *** | PRDX1 * | 0,956 |
| CLIC1 | MMP9 ** | ENOA * | 0,956 |
| MMP9 ** | SPIT1 | ENOA ** | 0,956 |
| CASP3 | MMP9 ** | ENOA * | 0,956 |
| MMP9 ** | PDIA1 | ENOA * | 0,956 |
| MMP9 ** | LDHA | ENOA * | 0,956 |
| MMP9 ** | MIF | ENOA | 0,956 |
| OSTP.1 | MMP9 ** | ENOA *** | 0,956 |
| CTNB1 | MMP9 ** | ENOA ** | 0,956 |
| CTNB1 | KPYM.1 * | MMP9 *** | 0,955 |
| KPYM.1 | MMP9 ** | LDHA | 0,955 |
| MMP9 ** | NGAL | ENOA *** | 0,955 |
| KPYM *** | CAYP1 | PERM * | 0,955 |
| OSTP | MMP9 *** | PRDX1 *** | 0,955 |
| MMP9 *** | PRDX1 ** | ANXA1 | 0,955 |
| MMP9 ** | PRDX1 *** | GTR1 | 0,955 |
| KPYM.1 | MMP9 *** | GSTP1 | 0,955 |
| KPYM.1 * | MMP9 *** | TPIS | 0,955 |
| MMP9 *** | PRDX1 ** | PDIA1 | 0,955 |
| MMP9 *** | PRDX1 *** | PIGR | 0,955 |
| MMP9 ** | PRDX1 *** | NGAL | 0,955 |
| KPYM.1 ** | MMP9 *** | CD44 | 0,955 |
| CLIC1 | KPYM ** | PERM * | 0,954 |
| ANXA2 | MMP9 *** | MIF** | 0,954 |
| KPYM ** | CD44 | PERM * | 0,954 |
| KPYM ** | PDIA1 | PERM * | 0,954 |
| KPYM ** | PERM ** | NAMPT | 0,954 |
| MMP9 *** | PRDX1 ** | TPIS | 0,954 |
| MMP9 *** | PRDX1 | MIF | 0,954 |
| MMP9 *** | PRDX1 ** | CH10 | 0,954 |
| XPO2 | MMP9 *** | PRDX1 ** | 0,954 |
| OSTP.1 | MMP9 *** | PRDX1 *** | 0,954 |
| ANXA2 | MMP9 *** | PRDX1 ** | 0,954 |
| MUC1 | MMP9 *** | PRDX1 *** | 0,954 |
| MMP9 *** | PRDX1 *** | SG2A1 | 0,954 |

(continued)

| PROTEIN1 | PROTEIN2 | PROTEIN3 | AUC |
|---|---|---|---|
| MMP9 *** | PRDX1 ** | HSPB1 | 0,954 |
| MMP9 ** | PDIA1 * | CADH1 * | 0,954 |
| MMP9 *** | PRDX1 *** | | 0,954 |
| MMP9 ** | PRDX1 *** | PERM | 0,953 |
| KPYM *** | MUC1 | PERM * | 0,953 |
| MMP9 ** | GTR1 | MIF *** | 0,953 |
| MMP9 ** | CADH1 * | LDHA * | 0,953 |
| KPYM * | KPYM.1 | PERM * | 0,953 |
| MMP9 ** | ANXA1 | CADH1 ** | 0,953 |
| MMP9 *** | PRDX1 ** | GSTP1 | 0,953 |
| MMP9 ** | PRDX1 ** | NAMPT | 0,953 |
| MMP9 ** | GSTP1 | CADH1 | 0,953 |
| MMP9 ** | CD44 | CAPG ** | 0,953 |
| MMP9 ** | CH10 * | LDHA ** | 0,953 |
| MMP9 ** | FABP5 * | CADH1 * | 0,953 |
| MMP9 *** | CADH1 * | HSPB1 * | 0,953 |
| XPO2 | MMP9 *** | MIF ** | 0,953 |
| KPYM *** | ANXA2 | PERM * | 0,952 |
| CLIC1 * | MMP9 ** | CADH1 * | 0,952 |
| CASP3 * | MMP9 *** | CADH1 | 0,952 |
| MMP9 *** | MIF | CAPG | 0,952 |
| MMP9 ** | SG2A1 * | LDHA *** | 0,952 |
| KPYM *** | NGAL | PERM * | 0,952 |
| KPYM *** | PERM * | PIGR | 0,952 |
| KPYM ** | PERM * | SPIT1 | 0,952 |
| KPYM *** | HSPB1 | PERM * | 0,952 |
| KPYM *** | PERM * | CAPG | 0,952 |
| KPYM *** | ANXA1 | PERM * | 0,952 |
| MMP9 *** | SG2A1 | MIF *** | 0,952 |
| MMP9 *** | FABP5 | MIF * | 0,952 |
| MMP9 *** | MIF *** | PIGR | 0,952 |
| MMP9 *** | ANXA1 | MIF ** | 0,952 |
| MMP9 *** | PDIA1 | MIF * | 0,952 |
| MMP9 ** | ANXA1 | LDHA ** | 0,952 |
| KPYM *** | PERM * | | 0,952 |
| KPYM ** | FABP5 | PERM * | 0,951 |
| CASP3 | MMP9 *** | CAPG | 0,951 |
| KPYM * | LDHA | PERM * | 0,951 |
| MMP9 ** | MIF ** | SPIT1 | 0,951 |

(continued)

| PROTEIN1 | PROTEIN2 | PROTEIN3 | AUC |
|---|---|---|---|
| KPYM ** | PRDX1 | PERM * | 0,951 |
| KPYM ** | XPO2 | PERM * | 0,951 |
| KPYM * | PERM * | ENOA | 0,951 |
| MMP9 ** | GSTP1 ** | SPIT1 | 0,951 |
| MMP9 ** | PDIA1 | LDHA * | 0,951 |
| MMP9 *** | PDIA1 | CAPG * | 0,951 |
| ANXA2 * | MMP9 ** | CADH1 ** | 0,951 |
| CTNB1 | KPYM ** | PERM * | 0,951 |
| CTNB1 * | MMP9 *** | CAPG * | 0,951 |
| CTNB1 | MMP9 *** | MIF* | 0,951 |
| MMP9 ** | LDHA | MIF | 0,951 |
| OSTP.1 | KPYM *** | PERM * | 0,95 |
| MMP9 *** | CH10 | CAPG * | 0,95 |
| MMP9 *** | GSTP1 ** | GTR1 | 0,95 |
| MMP9 *** | SG2A1 | GSTP1 *** | 0,95 |
| MMP9 *** | GSTP1 * | ANXA1 | 0,95 |
| MMP9 ** | PERM | MIF *** | 0,95 |
| OSTP.1 | MMP9 *** | MIF *** | 0,95 |
| MMP9 *** | GSTP1 * | FABP5 | 0,95 |
| KPYM *** | GTR1 | PERM * | 0,95 |
| KPYM *** | PERM * | K2C8 | 0,95 |
| MMP9 *** | GSTP1 | PDIA1 | 0,95 |
| CTNB1 | MMP9 ** | CADH1 * | 0,95 |
| MUC1 | MMP9 *** | MIF *** | 0,95 |
| MMP9 ** | MIF ** | NAMPT | 0,95 |
| MMP9 ** | LDHA | CAPG | 0,95 |
| MMP9 *** | ANXA1 | CAPG ** | 0,95 |
| MMP9 *** | HSPB1 | MIF * | 0,95 |
| ANXA2 | MMP9 ** | LDHA ** | 0,95 |
| MMP9 *** | MIF *** |  | 0,95 |
| OSTP | KPYM *** | PERM * | 0,949 |
| XPO2 | MMP9 *** | CAPG * | 0,949 |
| MMP9 *** | FABP5 | CAPG * | 0,949 |
| MMP9 *** | CH10 | MIF ** | 0,949 |
| OSTP | MMP9 *** | MIF *** | 0,949 |
| MMP9 ** | CADH1 ** | NAMPT * | 0,949 |
| ANXA2 | MMP9 *** | CAPG ** | 0,949 |
| XPO2 | MMP9 ** | CADH1 ** | 0,949 |
| MMP9 *** | TPIS | MIF * | 0,949 |

(continued)

| PROTEIN1 | PROTEIN2 | PROTEIN3 | AUC |
|---|---|---|---|
| MMP9 *** | TPIS | CAPG * | 0,949 |
| KPYM ** | CADH1 | PERM * | 0,949 |
| MMP9 *** | HSPB1 | CAPG * | 0,949 |
| MMP9 ** | GSTP1 | LDHA | 0,949 |
| MP9 * | LDHA*** | PERM | 0,949 |
| MMP9 ** | CADH1 *** | NGAL | 0,949 |
| MMP9 *** | GSTP1 | CAPG | 0,949 |
| CASP3 | MMP9 *** | MIF | 0,949 |
| MMP9 *** | FABP5 * | PDIA1 * | 0,949 |
| MMP9 *** | MIF *** | K2C8 | 0,949 |
| MMP9 * | LDHA ** | SPIT1 | 0,949 |
| MMP9 *** | GSTP1 | MIF | 0,949 |
| CLIC1 | MMP9 *** | MIF * | 0,949 |
| CLIC1 | MMP9 *** | CAPG | 0,949 |
| MMP9 ** | FABP5 | LDHA * | 0,949 |
| MUC1 | MMP9 *** | CAPG *** | 0,948 |
| MUC1 | MMP9 *** | GSTP1 *** | 0,948 |
| OSTP | MMP9 *** | CAPG *** | 0,948 |
| CLIC1 ** | MMP9 *** | CH10 * | 0,948 |
| CTNB1 * | MMP9 *** | FABP5 * | 0,948 |
| MMP9 ** | NGAL | MIF *** | 0,948 |
| MMP9 *** | SG2A1 | CAPG *** | 0,948 |
| MUC1 | MMP9 ** | CADH1 *** | 0,948 |
| CASP3 * | MMP9 *** | PDIA1 | 0,948 |
| MMP9 ** | HSPB1 ** | SPIT1 * | 0,948 |
| CASP3 ** | MMP9 ** | SPIT1 | 0,948 |
| MMP9 ** | LDHA * | TPIS | 0,948 |
| OSTP.1 | MMP9 *** | CADH1 *** | 0,948 |
| MMP9 *** | PDIA1 * | HSPB1 | 0,948 |
| CAYP1 | MMP9 *** | MIF *** | 0,948 |
| MMP9 ** | CD44 | MIF *** | 0,948 |
| MMP9 *** | GSTP1 *** | PIGR | 0,947 |
| OSTP.1 | MMP9 *** | GSTP1 *** | 0,947 |
| MMP9 *** | GSTP1 | TPIS | 0,947 |
| CAYP1 | MMP9 *** | GSTP1 ** | 0,947 |
| MMP9 *** | CADH1 * | TPIS | 0,947 |
| OSTP.1 | MMP9 *** | CAPG *** | 0,947 |
| MMP9 ** | CD44 | GSTP1 ** | 0,947 |
| CASP3 ** | MMP9 *** | CH10 * | 0,947 |

(continued)

| PROTEIN1 | PROTEIN2 | PROTEIN3 | AUC |
|---|---|---|---|
| MMP9 *** | GSTP1 *** | NGAL | 0,947 |
| KPYM *** | CH10 | PERM * | 0,947 |
| MMP9 *** | ANXA1 | PDIA1 * | 0,947 |
| MMP9 ** | GSTP1 *** | PERM | 0,947 |
| MMP9 ** | FABP5 ** | SPIT1 * | 0,947 |
| ANXA2 | CASP3 ** | MMP9 *** | 0,947 |
| MMP9 ** | HSPB1 | LDHA * | 0,947 |
| MMP9 *** | NGAL | CAPG *** | 0,946 |
| MMP9 ** | NAMPT | CAPG ** | 0,946 |
| OSTP | MMP9 *** | GSTP1 *** | 0,946 |
| MMP9 *** | GSTP1 ** | K2C8 | 0,946 |
| MMP9 *** | FABP5 | TPIS * | 0,946 |
| MMP9 *** | PIGR | CAPG *** | 0,946 |
| CASP3 * | MMP9 *** | FABP5 | 0,946 |
| MMP9 *** | GTR1 | CAPG ** | 0,946 |
| MMP9 *** | PERM | CAPG *** | 0,946 |
| MMP9 *** | K2C8 | CAPG *** | 0,946 |
| ANXA2 | MMP9 *** | GSTP1 ** | 0,946 |
| MMP9 *** | GSTP1 * | CH10 | 0,946 |
| MMP9 *** | ANXA1 | FABP5 * | 0,946 |
| CAYP1 | MMP9 *** | CAPG ** | 0,946 |
| MMP9 *** | PDIA1 ** | GTR1 | 0,946 |
| CTNB1 | MMP9 ** | LDHA * | 0,946 |
| CASP3 * | MMP9 *** | HSPB1 | 0,946 |
| MMP9 ** | GSTP1 * | NAMPT | 0,946 |
| MMP9 ** | CADH1 *** | K2C8 | 0,946 |
| CLIC1 | MMP9 *** | GSTP1 | 0,946 |
| MMP9 ** | PDIA1 *** | PERM | 0,946 |
| KPYM *** | CASP3 ** | CD44 * | 0,946 |
| SG2A1 * | LDHA *** | PERM ** | 0,946 |
| MMP9 *** | GSTP1 *** | | 0,946 |
| MMP9 *** | CAPG *** | | 0,946 |
| MMP9 ** | LDHA *** | PIGR | 0,945 |
| MMP9 ** | LDHA * | NAMPT | 0,945 |
| MMP9 ** | CD44 | PDIA1 ** | 0,945 |
| CASP3 *** | MMP9 *** | GTR1 | 0,945 |
| MMP9 ** | GTR1 | LDHA *** | 0,945 |
| ANXA2 | MMP9 *** | FABP5 * | 0,945 |
| OSTP | MMP9 *** | PDIA1 *** | 0,945 |

(continued)

| PROTEIN1 | PROTEIN2 | PROTEIN3 | AUC |
|---|---|---|---|
| CTNB1 ** | ANXA2 * | MMP9 *** | 0,945 |
| CASP3 ** | MMP9 *** | ANXA1 | 0,945 |
| ANXA2 | MMP9 *** | HSPB1 * | 0,945 |
| MMP9 *** | FABP5 | HSPB1 | 0,945 |
| ANXA2 | MMP9 *** | PDIA1 * | 0,945 |
| MMP9 *** | PDIA1 | TPIS | 0,945 |
| MMP9 *** | CH10 * | HSPB1 * | 0,945 |
| CLIC1 | MMP9 *** | PDIA1 | 0,945 |
| CASP3 | MMP9 *** | TPIS | 0,945 |
| MMP9 ** | SG2A1 | CADH1 *** | 0,945 |
| MMP9 ** | PDIA1 ** | SPIT1 | 0,945 |
| MMP9 *** | PDIA1 *** | PIGR | 0,945 |
| XPO2 | MMP9 *** | FABP5 * | 0,945 |
| CLIC1 ** | MMP9 *** | ANXA1 | 0,945 |
| XPO2 | MMP9 ** | LDHA * | 0,944 |
| CADH1 | LDHA ** | PERM * | 0,944 |
| CTNB1 | MMP9 *** | GSTP1 | 0,944 |
| CASP3 | MMP9 *** | GSTP1 | 0,944 |
| MUC1 | MMP9 ** | LDHA *** | 0,944 |
| MMP9 ** | CD44 | LDHA ** | 0,944 |
| MMP9 ** | SPIT1 | CAPG ** | 0,944 |
| KPYM *** | CH10 ** | TPIS ** | 0,944 |
| CASP3 | MMP9 ** | LDHA | 0,944 |
| MMP9 ** | ANXA1 * | SPIT1 ** | 0,944 |
| MMP9 *** | ANXA1 | HSPB1 * | 0,944 |
| MMP9 ** | PDIA1 * | NAMPT | 0,944 |
| MUC1 | MMP9 *** | HSPB1 *** | 0,944 |
| MMP9 *** | SG2A1 | PDIA1 *** | 0,944 |
| MMP9 *** | PDIA1 *** | NGAL | 0,944 |
| CASP3 *** | MMP9 *** | PIGR | 0,944 |
| MMP9 *** | GSTP1 * | HSPB1 | 0,944 |
| OSTP | MMP9 ** | CADH1 *** | 0,944 |
| MMP9 ** | CD44 | HSPB1 ** | 0,944 |
| OSTP.1 | MMP9 *** | PDIA1 *** | 0,944 |
| MMP9 ** | CADH1 * | CH10 | 0,944 |
| MMP9 * | CADH1 *** | PERM | 0,944 |
| MMP9 ** | CADH1 * | SPIT1 | 0,944 |
| MMP9 ** | CADH1 *** | GTR1 | 0,944 |
| MMP9 *** | PDIA1 * | CH10 | 0,944 |

(continued)

| PROTEIN1 | PROTEIN2 | PROTEIN3 | AUC |
|---|---|---|---|
| CLIC1 ** | MMP9 ** | SPIT1 | 0,944 |
| CLIC1 * | MMP9 *** | FABP5 | 0,944 |
| CLIC1 *** | MMP9 *** | SG2A1 | 0,944 |
| CAYP1 | MMP9 ** | LDHA *** | 0,944 |
| CLIC1 ** | ANXA2 | MMP9 *** | 0,944 |
| CLIC1 *** | MMP9 ** | GTR1 | 0,943 |
| CTNB1 | CASP3 * | MMP9 *** | 0,943 |
| KPYM ** | KPYM.1 * | CD44 * | 0,943 |
| CLIC1 | MMP9 *** | TPIS | 0,943 |
| CLIC1 | MMP9 ** | LDHA | 0,943 |
| MMP9 * | NGAL | LDHA *** | 0,943 |
| MMP9 *** | FABP5 * | CH10 * | 0,943 |
| MMP9 *** | GTR1 | TPIS *** | 0,943 |
| CLIC1 | CASP3 | MMP9 *** | 0,943 |
| CTNB1 * | MMP9 *** | CH10 * | 0,943 |
| MUC1 | MMP9 *** | PDIA1 *** | 0,943 |
| XPO2 | MMP9 *** | PDIA1 * | 0,943 |
| CAYP1 | MMP9 *** | PDIA1 ** | 0,943 |
| MMP9 *** | PDIA1 *** | K2C8 | 0,943 |
| CAYP1 | MMP9 *** | HSPB1 ** | 0,943 |
| OSTP.1 | MMP9 *** | HSPB1 *** | 0,943 |
| MMP9 ** | HSPB1 * | NAMPT | 0,943 |
| OSTP | MMP9 ** | LDHA *** | 0,943 |
| OSTP.1 | MMP9 ** | LDHA *** | 0,943 |
| KPYM.1 ** | CD44 * | PERM ** | 0,943 |
| MMP9 ** | LDHA *** | K2C8 | 0,943 |
| MMP9 ** | CD44 | CADH1 *** | 0,943 |
| MMP9 ** | CD44 | FABP5 *** | 0,943 |
| MMP9 *** | HSPB1 *** | PIGR | 0,943 |
| MMP9 ** | CADH1 *** | PIGR | 0,943 |
| MMP9 ** | LDHA *** |  | 0,943 |
| MMP9 *** | PDIA1 *** |  | 0,943 |
| MMP9 *** | HSPB1 *** |  | 0,943 |
| MMP9 ** | CADH1 *** |  | 0,943 |
| FABP5 | LDHA ** | PERM ** | 0,942 |
| KPYM ** | CD44 * | TPIS * | 0,942 |
| FABP5 * | CADH1 ** | PERM * | 0,942 |
| MMP9 ** | CD44 | CH10 *** | 0,942 |
| CTNB1 ** | MMP9 *** | ANXA1 | 0,942 |

(continued)

| PROTEIN1 | PROTEIN2 | PROTEIN3 | AUC |
|---|---|---|---|
| XPO2 | MMP9 *** | GSTP1 * | 0,942 |
| KPYM ** | CD44 * | GSTP1 * | 0,942 |
| XPO2 * | MMP9 *** | ANXA1 * | 0,942 |
| OSTP | MMP9 *** | HSPB1 *** | 0,942 |
| MMP9 *** | SG2A1 | HSPB1 *** | 0,942 |
| CTNB1 | MMP9 *** | PDIA1 | 0,942 |
| MMP9 *** | CH10 | TPIS * | 0,942 |
| CAYP1 | MMP9 ** | CADH1 *** | 0,942 |
| ANXA2 ** | MMP9 ** | CD44 * | 0,942 |
| ANXA2 * | XPO2 * | MMP9 *** | 0,942 |
| CASP3 *** | MMP9 ** | PERM | 0,942 |
| MMP9 *** | NGAL | HSPB1 *** | 0,942 |
| MMP9 *** | HSPB1 *** | PERM | 0,942 |
| MMP9 *** | HSPB1 | TPIS | 0,942 |
| CLIC1 * | MMP9 *** | HSPB1 | 0,942 |
| CTNB1 | MMP9 *** | TPIS * | 0,941 |
| KPYM.1 | LDHA * | PERM ** | 0,941 |
| CTNB1 | MMP9 *** | HSPB1 | 0,941 |
| MMP9 ** | TPIS ** | SPIT1 | 0,941 |
| CAYP1 | MMP9 *** | FABP5 ** | 0,941 |
| MMP9 *** | HSPB1 *** | K2C8 | 0,941 |
| KPYM ** | GSTP1 * | NGAL | 0,941 |
| ANXA2 | MMP9 *** | TPIS * | 0,941 |
| MMP9 *** | ANXA1 | TPIS * | 0,941 |
| MMP9 ** | FABP5 * | NAMPT | 0,941 |
| MMP9 * | SPIT1 * | NAMPT ** | 0,941 |
| MMP9 *** | FABP5 *** | PIGR | 0,941 |
| CAYP1 | CASP3 *** | MMP9 *** | 0,941 |
| MMP9 ** | ANXA1 | NAMPT * | 0,941 |
| XPO2 | CASP3 * | MMP9 *** | 0,941 |
| CASP3 *** | MMP9 *** | SG2A1 | 0,941 |
| MMP9 *** | HSPB1 ** | GTR1 | 0,941 |
| LDHA *** | PERM * | SPIT1 | 0,941 |
| CLIC1 *** | CAYP1 | MMP9 *** | 0,941 |
| CLIC1 *** | MMP9 ** | NGAL | 0,941 |
| CASP3 * | MMP9 ** | NAMPT | 0,941 |
| CLIC1 *** | MUC1 | MMP9 *** | 0,941 |
| CLIC1 *** | MMP9 ** | PERM | 0,941 |
| KPYM *** | NGAL | TPIS * | 0,94 |

(continued)

| PROTEIN1 | PROTEIN2 | PROTEIN3 | AUC |
|---|---|---|---|
| XPO2 * | MMP9 *** | CH10 * | 0,94 |
| MMP9 *** | ANXA1 | CH10 ** | 0,94 |
| KPYM ** | CASP3 * | SPIT1 | 0,94 |
| KPYM.1 * | CADH1 * | PERM ** | 0,94 |
| OSTP.1 | CASP3 *** | MMP9 *** | 0,94 |
| ANXA2 | MMP9 *** | CH10 * | 0,94 |
| MMP9 ** | CH10 ** | NAMPT * | 0,94 |
| MMP9 ** | FABP5 *** | GTR1 | 0,94 |
| KPYM.1 ** | PERM ** | SPIT1 * | 0,94 |
| MUC1 | CASP3 *** | MMP9 *** | 0,94 |
| KPYM ** | GSTP1 * | SPIT1 * | 0,94 |
| OSTP | CLIC1 *** | MMP9 *** | 0,94 |
| OSTP.1 | CLIC1 *** | MMP9 *** | 0,94 |
| CLIC1 * | MMP9 ** | NAMPT | 0,94 |
| CASP3 *** | MMP9 ** | NGAL | 0,94 |
| CLIC1 *** | MMP9 *** | | 0,94 |

## Citation List

- DeSouza LV, et al, "Endometrial cancer biomarker discovery and verification using differentially tagged clinical samples with multidimensional liquid chromatography and tandem mass spectrometry", Mol Cell Proteomics MCP-2007, vol. no.6, pp.:1170-8.
- Kemik P, et al. "Diagnostic and prognostic values of preoperative serum levels of YKL-40, HE-4 and DKK-3 in endometrial cancer", Gynecol Oncol- 2016; vol. no.140, pp.:64-9.
- Martinez-Garcia E, et al. "Development of a sequential workflow based on LC-PRM for the verification of endometrial cancer protein biomarkers in uterine aspirate samples", Oncotarget -2016, vol. no. 7(33), pp.:53102-53114.
- Robin et al., "pROC: and open-source package for R and S+ to analyze and compare ROC curves", BMC Bioinformatics-2011, vol. no. 12:77.


## Claims

**1.** A method of diagnosis and/or prognosis of endometrial cancer (EC), the method comprising determining the level of expression of protein BCAM in a uterine fluid sample from the female genital tract.

**2.** A method of deciding or recommending whether to initiate a medical regimen of a subject suffering endometrial cancer in the function of the diagnosis and/or prognosis, which method comprises the steps of:

a) determining, *in vitro,* the level of expression of protein BCAM in a uterine fluid sample from the subject's female genital tract; and
b) establishing the prognosis of said EC by distinguishing endometroid endometrial cancer (EEC) and non-endometrioid EC cases (NEEC), if the protein level in the test sample is higher than a reference control level for BCAM;

wherein:

i) if the subject is diagnosed of suffering from endometrial cancer, or of being suspicious of suffering from endometrial cancer, and the subject is differentially diagnosed of suffering EEC, then the initiation of the medical regimen for EEC is recommended;
ii) if the subject is diagnosed of suffering from endometrial cancer, or of being suspicious of suffering from endometrial cancer, and the subject is differentially diagnosed of suffering NEEC, then the initiation of the medical regimen for NEEC is recommended; and

by determining the marker level in a test sample, the skilled person can establish, additionally, which is the most suitable therapy that can be recommended, because the level detected in the sample may reflect the aggressiveness of the disease.

**3.** A method for identifying a subject suspicious of suffering from endometrial cancer and for further identifying EC subtype, by differentially diagnosing EEC of NEEC, the method comprising:

a) determining, *in vitro,* the level of expression of protein BCAM in an uterine fluid sample from the subject's female genital tract; and
b) comparing the level of step (a) with a reference control level, wherein if the level determined in step (a) is higher than the reference control level for BCAM, it is indicative that the subject is suspicious of suffering from NEEC and of suffering EEC.

**4.** The method according to any of the preceding claims, wherein the uterine fluid sample is uterine aspirate fluid sample from the female genital tract.

**5.** The method according to any of the preceding claims, which comprises determining the level of expression of one or more of the following sets: BCAM and RL29; BCAM and PODXL; AGRIN and BCAM; ANXA and BCAM; BCAM and RAB8A; BCAM and SYIC; AGR2, BCAM and PODXL; BCAM and PODXL; BCAM and PIGR; BCAM and MMP9.

**6.** The method according to any of the preceding claims, wherein the level of expression is determined at the protein level.

**7.** The method according to any of the preceding claims, wherein the protein level is determined by an assay or technology selected from the group consisting of an immunoassay, a bioluminescence assay, a fluorescence assay, a chemiluminescence assay, electrochemistry assay, mass spectrometry, and combinations thereof.

**8.** The method according to any of the claims 5 or 6, wherein the level of expression of protein is determined using an antibody or a fragment thereof able to bind to the protein.

**9.** The method according to claim 7, wherein said antibody or fragment thereof forms part of a kit.

**10.** In vitro use of BCAM for differentially diagnosing EEC and NEEC and/or for the prognosis of endometrial cancer.

**11.** Use of means for determining the level of expression of BCAM for differentially diagnosing EEC and NEEC and/or for the prognosis of endometrial cancer.

**12.** Use of a kit for the diagnosis and/or prognosis of EC, the kit comprising a solid support and means for detecting the level of expression of BCAM.

**13.** Use of the kit of claim 11, wherein the means for detecting the level of expression are antibodies or fragments thereof.

**14.** A method according to any of the claims 1 to 8, in which after the determination of the level of expression of the protein, said level(s) are given a value and/or a score, and optionally are computed in a mathematical formula to obtain a computed value; wherein in function of the said level(s), score(s) and or computed value(s), a decision is taken between the options of suffering or not from EC and/or between the options of suffering among different EC subtypes.

## Patentansprüche

**1.** Verfahren zur Diagnose und/oder Prognose von Endometriumkrebs (EC), wobei das Verfahren das Bestimmen des

Expressionsniveaus des Proteins BCAM in einer Uterusflüssigkeitsprobe aus dem weiblichen Genitaltrakt umfasst.

2. Verfahren zum Entscheiden oder zum Empfehlen, ob ein medizinisches Regime eines Individuums, welcher unter Endometriumkrebs leidet, in Abhängigkeit von der Diagnose und/oder Prognose gestartet werden soll, welches Verfahren die folgenden Schritte umfasst:

   a) das *In-vitro*-Bestimmen des Expressionsniveaus des Proteins BCAM in einer Uterusflüssigkeitsprobe aus dem weiblichen Genitaltrakt des Individuums; und
   b) das Feststellen der Prognose des genannten EC, indem Fälle von endometroidem Endometriumkrebs (EEC) und nicht endometroidem EC (NEEC) unterscheiden werden, wenn das Proteinniveau in der Testprobe höher als ein Referenz-Kontrollniveau für BCAM ist;

   wobei:

   i) wenn beim Individuum diagnostiziert wird, dass es unter Endometriumkrebs leidet, oder dass es unter Verdacht steht unter Endometriumkrebs zu leiden, und beim Individuum differenziell diagnostiziert wird, dass es unter EEC leidet, dann der Start des medizinischen Regimes für EEC empfohlen wird;
   ii) wenn beim Individuum diagnostiziert wird, dass es unter Endometriumkrebs leidet, oder dass es unter Verdacht steht unter Endometriumkrebs zu leiden, und beim Individuum differenziell diagnostiziert wird, dass es unter NEEC leidet, dann der Start des medizinischen Regimes für NEEC empfohlen wird; und

   wobei, indem das Markerniveau in einer Testprobe bestimmt wird, der Fachmann, zusätzlich, feststellen kann, welche die am besten geeignete Therapie ist, welche empfohlen werden kann, weil das in der Probe detektierte Niveau die Aggressivität der Krankheit widerspiegeln kann.

3. Verfahren zum Identifizieren eines Individuums, welches unter Verdacht steht unter Endometriumkrebs zu leiden und zum zusätzlichen Identifizieren des EC-Subtyps, indem EEC oder NEEC differenziell diagnostiziert werden, wobei das Verfahren Folgendes umfasst:

   a) das *In-vitro*-Bestimmen des Expressionsniveaus des Proteins BCAM in einer Uterusflüssigkeitsprobe aus dem weiblichen Genitaltrakt des Individuums; und
   b) das Vergleichen des Niveaus aus Schritt (a) mit einem Referenz-Kontrollniveau, wobei, wenn das in Schritt (a) bestimmte Niveau höher als das Referenz-Kontrollniveau für BCAM ist, dies darauf schließen lässt, dass das Individuum unter Verdacht steht unter NEEC zu leiden und unter EEC zu leiden.

4. Verfahren nach einem der vorhergehenden Ansprüche, wobei die Uterusflüssigkeitsprobe eine Aspirat-Uterusflüssigkeitsprobe aus dem weiblichen Genitaltrakt ist.

5. Verfahren nach einem der vorhergehenden Ansprüche, welches das Bestimmen des Expressionsniveaus eines oder mehrerer der folgenden Sätze umfasst: BCAM und RL29; BCAM und PODXL; AGRIN und BCAM; ANXA und BCAM; BCAM und RAB8A; BCAM und SYIC; AGR2, BCAM und PODXL; BCAM und PODXL; BCAM und PIGR; BCAM und MMP9.

6. Verfahren nach einem der vorhergehenden Ansprüche, wobei das Expressionsniveau auf Proteinebene bestimmt wird.

7. Verfahren nach einem der vorhergehenden Ansprüche, wobei das Proteinniveau mittels eines Assays oder einer Technologie bestimmt wird, welche aus der Gruppe bestehend aus einem Immuntest, einem Biolumineszenzassay, einem Fluoreszenzassay, einem Chemiluminisezenzassay, einem elektrochemischen Assay, Massenspektrometrie und Kombinationen derselben ausgewählt wird.

8. Verfahren nach einem der Ansprüche 5 oder 6, wobei das Expressionsniveau von Protein unter Verwendung eines Antikörpers oder eines Fragments desselben, welcher in der Lage ist, das Protein zu binden, bestimmt wird.

9. Verfahren nach Anspruch 7, wobei der genannte Antikörper oder Fragment desselben Bestandteil eines Kits ist.

10. *In-vitro*-Verwendung von BCAM für die differenzielle Diagnose von EEC und NEEC und/oder für die Prognose von Endometriumkrebs.

**11.** Verwendung von Mitteln zur Bestimmung des Expressionsniveaus von BCAM für die differenzielle Diagnose von EEC und NEEC und/oder für die Prognose von Endometriumkrebs.

**12.** Verwendung eines Kits für die Diagnose und/oder Prognose von EC, wobei das Kit eine feste Abstützung und Mittel zum Detektieren des Expressionsniveaus von BCAM umfasst.

**13.** Verwendung des Kits nach Anspruch 11, wobei die Mittel zum Detektieren des Expressionsniveaus Antikörper oder Fragmente derselben sind.

**14.** Verfahren nach einem der Ansprüche 1 bis 8, in welchem, nach der Bestimmung des Expressionsniveaus des Proteins, das genannte Niveau/die genannten Niveaus einen Wert und/oder eine Punktzahl bekommt/bekommen, und wahlweise in einer mathematischen Formel berechnet werden, um einen berechneten Wert zu erhalten; wobei, in Abhängigkeit vom genannten Niveau/von den genannten Niveaus, von der genannten Punktzahl/von den genannten Punktzahlen und/oder vom genannten berechneten Wert/von den genannten berechneten Werten, eine Entscheidung zwischen den Möglichkeiten des Leidens oder nicht Leidens unter EC und/oder zwischen den Möglichen des Leidens unter den unterschiedlichen EC-Subtypen getroffen wird.

## Revendications

**1.** Procédé de diagnostic et/ou pronostic de cancer de l'endomètre (EC), le procédé comprenant déterminer le niveau d'expression de protéine BCAM dans un échantillon de liquide utérin à partir de l'appareil génital féminin.

**2.** Procédé pour décider ou recommander si initier un régime médical d'un sujet atteint de cancer de l'endomètre dans la fonction du diagnostic et/ou pronostic, dont le procédé comprend les étapes de :

a) déterminer, *in vitro*, le niveau d'expression de protéine BCAM dans un échantillon de liquide utérin à partir de l'appareil génital féminin du sujet ; et
b) établir le pronostic dudit EC en distinguant des cas de cancer de l'endomètre endométrioïde (EEC) et d'EC non endométrioïde (NEEC), si le niveau de protéine dans l'échantillon d'essai est supérieur à un niveau de contrôle de référence pour BCAM ;

dans lequel :

i) si le sujet est diagnostiqué comme être atteint de cancer de l'endomètre, ou comme être suspecté d'être atteint de cancer de l'endomètre, et le sujet est diagnostiqué différentiellement comme être atteint d'EEC, alors l'initiation du régime médical pour l'EEC est recommandée ;
ii) si le sujet est diagnostiqué comme être atteint de cancer de l'endomètre, ou comme être suspecté d'être atteint de cancer de l'endomètre, et le sujet est diagnostiqué différentiellement comme être atteint de NEEC, alors l'initiation du régime médical pour le NEEC est recommandée ; et

en déterminant le niveau de marqueur dans un échantillon d'essai, l'homme du métier peut établir, en outre, quelle est la thérapie la plus appropriée qui peut être recommandée, car le niveau détecté dans l'échantillon peut refléter l'agressivité de la maladie.

**3.** Procédé pour identifier un sujet suspecté d'être atteint de cancer de l'endomètre et pour identifier en outre le sous-type d'EC, en diagnostiquant différentiellement l'EEC par rapport au NEEC, le procédé comprenant :

a) déterminer, *in vitro*, le niveau d'expression de protéine BCAM dans un échantillon de liquide utérin à partir de l'appareil génital féminin du sujet ; et
b) comparer le niveau de l'étape (a) à un niveau de contrôle de référence, dans lequel si le niveau déterminé dans l'étape (a) est supérieur au niveau de contrôle de référence pour BCAM, cela est indicatif que le sujet est suspecté d'être atteint de NEEC et d'être atteint d'EEC.

**4.** Procédé selon l'une quelconque des revendications précédentes, dans lequel l'échantillon de liquide utérin est un échantillon de liquide d'aspiration utérine à partir de l'appareil génital féminin.

**5.** Procédé selon l'une quelconque des revendications précédentes, qui comprises la détermination du niveau

d'expression d'un ou plusieurs des ensembles suivants : BCAM et RL29 ; BCAM et PODXL ; AGRIN et BCAM ; ANXA et BCAM ; BCAM et RAB8A ; BCAM et SYIC ; AGR2, BCAM et PODXL ; BCAM et PODXL ; BCAM et PIGR ; BCAM et MMP9.

**6.** Procédé selon l'une quelconque des revendications précédentes, dans lequel le niveau d'expression est déterminé au niveau de protéine.

**7.** Procédé selon l'une quelconque des revendications précédentes, dans lequel le niveau de protéine est déterminé par un essai ou une technologie choisi dans le groupe constitué d'un immunoessai, un essai de bioluminescence, un essai de fluorescence, un essai de chimioluminescence, un essai d'électrochimie, une spectrométrie de masse, et des combinations de ceux-ci.

**8.** Procédé selon l'une quelconque des revendications 5 ou 6, dans lequel le niveau d'expression de protéine est déterminé en utilisant un anticorps ou un fragment de celui-ci capable de se lier à la protéine.

**9.** Procédé selon la revendication 7, dans lequel ledit anticorps ou fragment de celui-ci fait partie d'un kit.

**10.** Utilisation *in vitro* de BCAM pour le diagnostic différentiel d'EEC et de NEEC et/ou pour le pronostic de cancer de l'endomètre.

**11.** Utilisation de moyens pour la détermination du niveau d'expression de BCAM pour le diagnostic différentiel d'EEC et de NEEC et/ou pour le pronostic de cancer de l'endomètre.

**12.** Utilisation d'un kit pour le diagnostic et/ou le pronostic d'EC, le kit comprenant un support solide et des moyens pour la détection du niveau d'expression de BCAM.

**13.** Utilisation du kit selon la revendication 11, dans lequel les moyens pour la détection du niveau d'expression sont des anticorps ou des fragments de ceux-ci.

**14.** Procédé selon l'une quelconque des revendications 1 à 8, dans lequel, après la détermination du niveau d'expression de la protéine, une valeur et/ou un score sont assignés audit/auxdits niveau(x), et optionnellement ils sont calculés par le biais d'une formule mathématique pour obtenir une valeur calculée ; dans lequel, en fonction dudit/desdits niveau(x), score(s) et/ou valeur(s) calculée(s), une décision est prise entre les options d'être atteint ou non d'EC et/ou entre les options d'être atteint de différents sous-types d'EC.

1.0
0.8
0.6
0.4
0.2
0.0
Sensitivity
CTNB1 + XPO2 + CAPG
CTNB1
XPO2
CAPG
1.0
0.8
0.6
0.4
0.2
0.0
1-Specificity

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 17382483
- US 4168146 A
- US 4366241 A
- US 4235601 A
- US 4442204 A
- US 5208535 A


### Non-patent literature cited in the description


- **DESOUZA LV et al.** Endometrial cancer biomarker discovery and verification using differentially tagged clinical samples with multidimensional liquid chromatography and tandem mass spectrometry. *Mol Cell Proteomics MCP*, 2007 (6), 1170-8
- **KEMIK P et al.** Diagnostic and prognostic values of preoperative serum levels of YKL-40, HE-4 and DKK-3 in endometrial cancer. *Gynecol Oncol*, 2016 (140), 64-9
- **MARTINEZ-GARCIA E et al.** Development of a sequential workflow based on LC-PRM for the verification of endometrial cancer protein biomarkers in uterine aspirate samples. *Oncotarget*, 2016, vol. 7 (33), 53102-53114
- **BURTIS C. A. et al.** *Statistical Treatment of Reference Values*, 2008
- **ROBIN et al.** pROC: and open-source package for R and S+ to analyze and compare ROC curves. *BMC Bioinformatics*, 2011, vol. 12, 77
- **DESOUZA LV et al.** Endometrial cancer biomarker discovery and verification using differentially tagged clinical samples with multidimensional liquid chromatography and tandem mass spectrometry. *Mol Cell Proteomics MCP*, 2007, vol. 6, 1170-8
- **KEMIK P et al.** Diagnostic and prognostic values of preoperative serum levels of YKL-40, HE-4 and DKK-3 in endometrial cancer. *Gynecol Oncol*, 2016, vol. 140, 64-9